(19) Europäisches Patentamt
European Patent Office
Office européen des brevets

(11) Publication number: **0 248 040 B1**

(12) # EUROPEAN PATENT SPECIFICATION

(45) Date of publication of patent specification: **24.03.93**

(51) Int. Cl.⁵: **C07K 15/12**, A61K 37/02, A61K 39/395

(21) Application number: **86906742.1**

(22) Date of filing: **01.12.86**

(86) International application number: **PCT/AU86/00370**

(87) International publication number: **WO 87/03286 (04.06.87 87/12)**

(54) **RICIN-ANTIBODY CONJUGATES.**

(30) Priority: **29.11.85 AU 3647/85**

(43) Date of publication of application: **09.12.87 Bulletin 87/50**

(45) Publication of the grant of the patent: **24.03.93 Bulletin 93/12**

(84) Designated Contracting States: **AT BE CH DE FR GB IT LI LU NL SE**

(56) References cited:
**EP-A- 0 044 167**
**FR-A- 2 466 252**
**FR-A- 2 591 895**
**US-A- 4 414 148**
**AU-A- 9 033 482**
**FR-A- 2 566 403**
**US-A- 4 350 626**

**BLOOD, vol. 63, no. 5, 1984; C.D.MYERS et al., pp. 1178-1185&NUM;**

**CANCER RESEARCH, vol. 44, August 1984; J.C.MERRIAM et al., pp. 3178-3183&NUM;**

(73) Proprietor: **CONSOLIDATED PHARMACEUTICALS LIMITED**
**10 Eagle Street**
**Brisbane Oueensland(AU)**

(72) Inventor: **McKENZIE, Ian, Farquhar, Campbell**
**359 Brunswick Road**
**Brunswick, VIC 3056(AU)**
Inventor: **PIETERSZ, Geoffrey, Allan**
**120 New Street**
**Ringwood, VIC 3134(AU)**
Inventor: **KANELLOS, Jerry**
**8 Arden Street**
**North Melbourne, VIC 3951(AU)**

(74) Representative: **Allam, Peter Clerk et al**
**LLOYD WISE, TREGEAR & CO. Norman House 105-109 Strand**
**London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 248 040 B1

CANCER RESEARCH, vol. 44, July 1984, R.C.STRONG et al., pp. 3000-3006&NUM;

BLOOD, vol. 65, no 5, 1985; J.E.LEONARD et al., "Preclinical Studies on the Use of Selective Anti-body-Ricin Conjugates in Autologous Bone Marrow Transplantation"&NUM;

## Description

This invention relates to a chemical compound.

In a particular aspect this invention relates to a ricin-antibody conjugate.

The plant toxin ricin has been involved in extensive research since the 1950's when it was suggested that it may be a useful anti-tumor agent because of its great potency. Ricin has a molecular weight of approximately 67,000 and is composed of two polypeptide chains, referred to as the A and B chains, which are disulphide linked. Ricin has a strong binding affinity for simple sugars and an even stronger affinity for oligosaccharides bearing D-galactose or N-acetyl galatosamine as terminal residues. It is through such molecules on the cell surface that ricin attaches to the membranes of the cells. Thus in the absence of an inhibitory mechanism whole ricin is toxic to all cells. Such binding and toxicity can therefore be competitively inhibited in vitro by the presence of lactose or D-galatose. The problem of specificity of ricin in anti-tumor therapy is currently being tackled by covalently binding it to immunoglobulins to form hybrid molecules known as immunatoxins. In this way it is hoped that the ricin may be directed only to those cells specifically reacting with the immunoglobulin of the immunotoxin. When the whole toxin has been conjugated to antibodies there has been a strong residual non-specific cytotoxicity due to the binding capacity of the B chain of the toxin. As a consequence research has been concentrated on coupling the A-chain of the whole ricin molecules, which is responsible for inhibition of protein synthesis or ribosomes. Unfortuntely, A-chain immunotoxins have been disappointing and it is quite clear that the B chain is necessary for ricin to be a potent poison.

The present invention provides a conjugate of ricin being whole ricin or a derivative of ricin, which derivative per se being inhibitory of protein synthesis in cell free systems, with an antibody which is preferentially absorbed by a tumour cell as compared to a non tumour cell and wherein the galactose binding site of said ricin is blocked and pharmacologically acceptable salts thereof.

Preferably the conjugation is via a disulphide linkage.

The present invention also provides a compound of formula

$Ab\text{-}Y\text{-}X_1\text{-}NHR_1$

where Ab and the Y group attached thereto represent an antibody radical and wherein Y is S or NH, $R_1$ and the amino group attached thereto represent a whole ricin radical, and $X_1$, or Y-X (when Y is S), represent a conjugating chain including a disulphide linkage.

In a preferred instance, the present invention provides compounds of formula

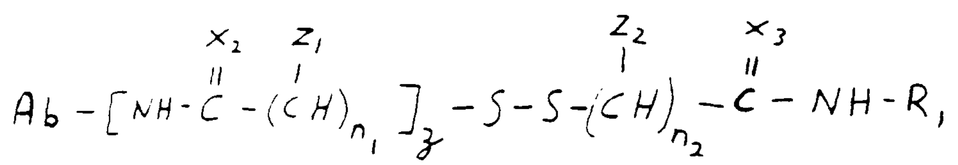 I

wherein Ab and the amino or sulphur to which it is attached represents antibody radical, $R_1$ and the amino group to which it is attached represents a whole ricin radical, $Z_1$ and $Z_2$ which may be the same or different are H, alkyl, aryl, carboxyl, halide, cyano, ester, carboxamide, sulphonate, hydroxy or amino, $n_1$ and $n_2$ which may be the same or different are 1-10 $X_2$ and $X_3$ which may be the same or different are NH, O or S, and z is 0 or 1 and salts thereof.

In a preferred instance, the present invention provides compounds of formula

$Ab\text{-}S\text{-}S\text{-}R_2\text{-}NH\text{-}R_1$ II

$Ab\text{-}NH\text{-}R_3\text{-}S\text{-}S\text{-}R_2\text{-}NHR_1$ III

wherein Ab-S-and Ab-NH- represents an antibody radical,

$R_1$-NH-represents a whole ricin radical and -$R_2$ NH and - NH-$R_3$ which may be the same or different are of formula

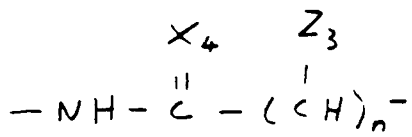

IV

wherein $X_4$ is O,S or NH, n is from 1 to 10 and $Z_3$ is H, alkyl, aryl, carboxyl, halide, cyamo, ester, carboxamide, sulphonate, hydroxy or amino.

Particularly preferred compounds are those of formula V

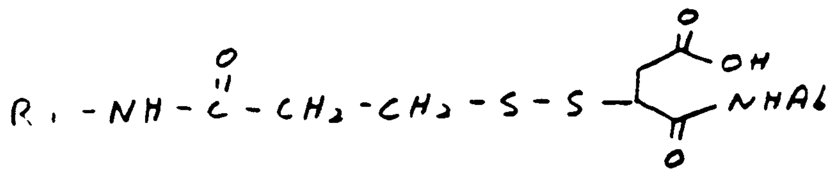

V

with galactose blocking as aforesaid.

The present invention also provides the intermediate compounds

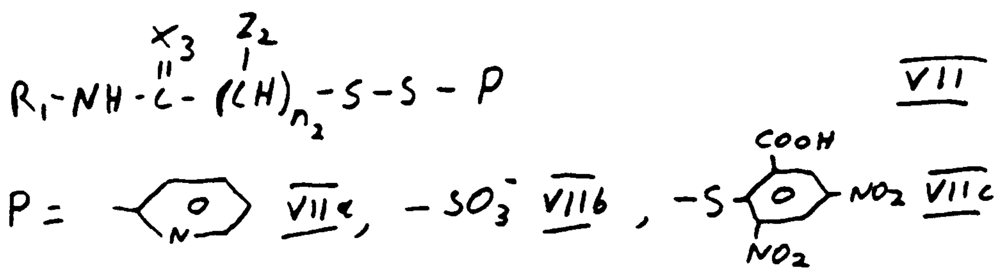

VII, VIIa, VIIb, VIIc

wherein $R_1$, $X_3$, $Z_2$ $n_2$ have the values given above.

Compounds in accordance with this invention can be prepared by reacting whole ricin with succinimidyl pyridyldithio-propionate (SPDP) or an analogue thereto appropriate to the compound desired to obtain a whole ricin conjugate containing a -S-S- group, reacting an antibody with SPDP or an analogue thereto appropriate to the compound desired to obtain an antibody conjugate containing a -S-S- group, reducing one of the conjugates to form a -SH group and reacting the reduced conjugate with the other of the conjugates to obtain the compound desired.

Other methods of obtaining compounds in accordance with this invention include using S-acetylmercaptosuccinic anhydride (SAMSA) or sulphydryl introducing compounds to produce a side chain on an antibody or whole ricin containing a -S- linkage, reducing and then reacting to form an antibody whole ricin conjugate containing a -S-S- linkage.

Intermediate VII can be prepared when ricin is reacted with SPDP.

Antibodies useful in the present invention included those showing specificity for breast, brain, melanoma, lung, pancreas and colon tumours.

The compounds of this invention may be combined with pharmaceutically acceptable carriers.

The mode of administration of the compounds of this invention will be as selected. In particular, the compounds of this invention may be administered intravenously, intraperitonealy, intrapluraly, intrapericardialy, intracerebo spinal fluid and directly intratumour.

Drawings and Tables forming part of this specification are:-

Formula Drawings - 1 Sheet

Part A:-
Figures: A1a, A1b, A2, A3a and A3b
Part B:-
Figures: B1, B2a, B2b, B3, B4, B5, B6, B7a, B7b, B8, B9a, B9b, B10a, B10b, B10c and B11
Table: B1, B2, B3 and B4

Part C:-
Figures: C1a, C1b, C3, C4, C6a, C6b, C6c, C7, C8a, C8b, and C9
Table: C1 and C2
Part D:-
Figures: D1a, D1b, D2a, D2b, D2c, D5, D7, D8 and D9
Table: D1

Part A

Conjugates in accordance with this invention appear not to bind "non-specifically" to target cells and do not require presence of lactose to inhibit any non specific binding (Fig. A1a and A1b). While these studies are of interest for in vitro studies in that lactose does not have to be used (of no great consequence) the major advantage of the non-specific binding sites is that whole ricin antibody complexes can now be used in vivo where such high concentrations of lactose cannot be achieved. Thus, mice bearing tumours were treated with three different regimes of whole ricin antibody conjugates. In the first, the ITT(1) 75 NS E3 thymoma was growing in the peritoneum of mice in the ascites form and whole ricin antibody conjugates were delivered into the ascites fluid (Fig. A2). when $1x10^5$ tumour cells were given and one injection of ricin-antibody (1 day after tumour inoculation) the tumours were erradicated and mice survived indefinitely. By contrast those receiving a mixture of antibody and ricin alone died rapidly from ricin poisoning, and those receiving no treatment died shortly thereafter from the tumour. Thus, the whole ricin-antibody conjugates were capable of erradicating tumours from mice in vivo. However, it should be noted that the therapeutic range of the ricin-antibody complex was narrower to greater doses (>0.1mu g) of ricin-antibody complexes led to the death of the mice due to toxicity and lower doses (<0.05mu g) led to death from the tumour. However, the dose range is not very different to that used for most cytotoxic drugs in the treatment of cancer.

In the second model, the same tumour grown subcutaneously and measuring 0.6cm in diameter, received two injections of antibody-ricin conjugate intravenously (0.1mu g ricin, 0.45mu g MoAb, on days 8 and 11 after tumour inoculation). A significant inhibition of tumour growth was observed in the group which received the specific ricin-antibody when compared to other controls which included an irrelevant ricin-antibody complex (Fig. A3). In the third model - perhaps the most dramatic - tumours 0.6cm in diameter growing in the subcutaneous tissue of mice received two injections of ricin-antibody conjugate directly into the tumour (approx 0.2mu g ricin, 0.9mu g MoAb on days 7 and 9 after tumour inoculation). Within 24 hrs there was a notable difference in size of tumours (Fig.A3b) compared to that of controls and by 48 hrs the treated group had virtually disappeared.

Thus, in these three different models whole ricin-antibody conjugates led to either erradication of the tumour or a marked dimunition in tumour size. Ricin would appear to have a lot to offer as a cytotoxic agent because of its great degree of potency - it is estimated that only one toxin molecule is apparently sufficient to kill one cell. Prior to these studies, use of ricin in vivo was impossible because of its high toxicity. However, with careful attention to dose and coupling procedures, it now appears that ricin may be suitable for consideration as a therapeutic agent for cancer in man.

EXAMPLE I

Specific cytotoxic activity of whole ricin immunotoxins. 5mg of affinity purified anti-Ly-2.1 (5.7mg/ml was thiolated with a 6 fold excess of SPDP (2mg/ml in dioxane). 3mg of ricin (2mg/ml) had a trace amount of $^{125}$I-ricin added and was thiolated with a 10 fold excess of SPDP. Both were reacted for 30 mins at room temperature and dialysed against 0.01M borats buffer containing 0.15M NaCl. The thiolated anti-Ly-2.1 was dialysed for 6 hrs against 0.2M Na acetate buffer pH 4.6 with 0.15M NaCl and 2mM EDTA when it was reduced with DTT (3.4mM final concentration). The reduced MoAb was dialysed against deoxygenated borate buffer for 6 hrs. The thiolated ricin was then added in a 1:1 molar ratio and the mixture allowed to react for 2 hrs when the reaction was stopped with N-ethylmaleimide. The mixture was purified on a sephacryl-S-300 column. The anti-Ly-2.1-ricin immunotoxin was collected and passed down a lactose sepharose column where 96% of the recovered immunotoxins did not bind thereby separating out poduct with unblocked galactose binding site.

Test I

The cytotoxicity of the purified immunotoxin was examined in the presence (Fig. A1a) or absence (Fig. A1b) of 100mM lactose. $8x10^5$ ITT(1) 75NS E3 cells were incubated with different concentrations of toxin or immunotoxin for 1 hr. The cells were washed three times to remove any unbound material and resuspended with leucine free RPMI and then pulsed with $^3$H-leucine (luCi) for 16-24 hrs. Results are expressed as the % inhibition of $^3$H-leucine incorporation in controls (cells treated with PBS).

Test II

Survival experiment: groups of 10 $(B6xBALB/c)F_1$ mice were injected i.p. with $1x10^5$ ITT(1) 75NS E3 cells. Mice received one of the following treatments i.p., one day after tumour inoculation: PBS; ricin + anti-Ly-2.1; anti-Ly-1.1-ricin; or anti-Ly-2.1-ricin. Each mouse received 0.05mu g ricin and 0.22mu g anti-Ly-2.1. Survival was followed for 130 days. Results are shown in Fig. A2.

Test III

Tumour growth experiment: Groups of 10 $(B6xBALB/c)F_1$ mice were injected s.c. with $4x10^6$ ITT(1) 75NS E3 cells. Mice received the following treatments: PBS; anti-Ly-2.1: anti-Ly-1.1-ricin; and anti-Ly-2.1-ricin. Each mouse received 0.1mu g ricin and 0.45 mu g anti-Ly-2.1 intravenously (Fig. A3a) on days 6 and 8 after tumour innoculation or directly into the tumour(Fig A3b) on days 7 and 9. Tumour growth was calculated as the product of two perpendicular diameters. Results are shown in Fig. A3.

Part B

This part reports on the chemical linkage of ricin to MoAb via a disulfide bridge with the use of two bifunctional crosslinkers. The coupling procedure led to a steric blocking of the galactose binding ability of the B-chain. A number of different MoAb, with three different immunoglobulin isotypes have been conjugated and all have retained cytotoxic activity but were devoid of galactose binding.

MATERIALS AND METHODS

Tumor cells BW5147 (10), EL4 (11) and several clonal variants of the murine thymoma ITT(1)75NS (12) were grown in 10% heat-inactivated newborn calf serum in Dulbecco's Modified Eagle medium (DMEM). The human T-cell leukemia line CEM (13) was maintained in RPMI-1640 with the same additives.

MoAb MoAb used were: anti-Ly-2.1 (IgG1, IgG2a) (14), anti-Ly-1.1 (IgG2a) (15), which recognize the murine lymphocyte alloantigens Ly-2.1 and Ly-1.1 respectively; HuLy-m9 (IgG1) (16), an antibody which reacts with the human transferrin receptor; 250-30.6 (IgG2b) (17), an antibody directed against an antigen present on human colon carcinoma and secretory epithelium; 17.1/2 (IgG2a) (18) MoAb directed against an antigen present on human breast tissue; anti-HLA (IgG2a) (19) a monomorphic HLA MoAb. The anti-HLA and 17.1/2 were purified by affinity chromatography or by Protein-A-Sepharose (Pharmacia).

Carbohydrate Modification of Ricin Modification of the carbohydrate in ricin was achieved using a mixture of sodium metaperiodate ($NaIO_4$) and sodium cyanoborohydride ($NaCNBH_3$) according to the procedure described by Thorpe et al (21). Briefly, the method was as follows, 10 mg ricin (1.8 mg/ml, Sigma Chemical Company St. Louis, MO, USA) in 0.2 M sodium acetate buffer pH 3.5 was mixed at 4°C in the dark with an equal volume of a mixture of 20 mM $NaIO_4$ and 40 mM $NaCNBH_3$. After 1 hour incubation 20% (v/v) solution of glycerol (100 mM final concentration) was added to the reaction mixture to destroy unreacted $IO_4^-$. The sample was kept on ice for 16 to 24 hours and was dialized against 0.01 M phosphate buffer containing 0.15 M NaCl pH 7.5. Since HCN is produced during the reaction it was carried out inside a fume cupboard and the dialysates were disposed of with appropriate precautions. The modified ricin was concentrated with an Amicon Concentrator (Amicon corporation, Massachusetts, USA) and stored in the phosphate buffer at -20°C. The significance of carbohydrate modification will be discussed in detail in Part C.

Radiolabeling of Ricin and Antibody Ricin (100 mug, 1 mg/ml) or ricin ($IO_4^-/CNBH_3^-$ modified) was labeled using chloramine T (22) in the presence of 100 mM lactose and MoAb (100 mug, 1 mg/ml) using the same procedure.

The Preparation of Ricin-Antibody Conjugates Buffer solutions: Three buffers were used during the preparation of the conjugates: (a) 0.01 M sodium phosphate, pH 7.5, containing 0.15 M NaC1; (b) 0.01 M

sodium borate, pH 8.0, containing 0.15 M NaC1; (c) 0.2 M sodium acetate, pH 4.6, containing 0.15 M NaCl and 2 mM EDTA.

Whole Ricin Conjugates: MoAb were coupled to ricin by 2 different procedures that involved the use of heterobifunctional protein crosslinking reagents - a novel method described in this part using the heterobifunctional reagent SAMSA, and a method using SPDP.

Conjugation Using SAMSA: Disulfide linked ricin-antibody conjugates were prepared as follows: (a) Thiolation of MoAb: MoAb (10 mg, 5 mg/ml) in phosphate buffer was treated with a 5-fold excess of SAMSA (Sigma) for 30 minutes and excess reagents were removed by dialysis into phosphate buffer containing 2 mM EDTA; (b) Thiolation of ricin/modified ricin ($IO_4^-/BH_3^-$ modified): 5 mg ricin (1.8 mg/ml) or ricin $IO_4^-/BH_3^-$ modified) in the phosphate buffer was treated with a 10-fold excess of SPDP (Pierce Chemical Company, Illinois, USA) for 30 minutes and dialysed into phosphate buffer containing 2 mM EDTA for 16 to 24 hours; (c) coupling of ricing to MoAb: The modified MoAb was then treated with 200 mul hydroxylamine (1 M in PBS pH 7.0) and after 30 minutes at room temperature it was mixed with the SPDP modified ricin (in a 1 to 1 molar ratio) and allowed to react for 1 to 6 hours. The reaction was terminated by the addition of N-ethylmaliimide (6.6 mM final concentration), allowed to stand a further 1 hour and than subjectd to gel permeation chromatography on Sephacryl S-300 equilibrated with phosphate buffer containing 20 mM lactose and eluted with the same buffer. To identify the conjugate fractions, trace amounts of $125_I$-labeled ricin (specific activity = 4000 cpm/mug) were added; the profile of the absorbance measured at 280 nm of the elute is shown in Figure B1 together with the radioactivity. Fractions 97-105 contained material of Mr 190,000 - 240,000 (calculated from the elution volume), and comprised IgG and ricin in the molar ratio 2:1, (calculated from measurements of absorbance at 280 nm and radioactivity) and were shown by sodium dodecyl sulfate gel analysis to contain as the major species the 210,000 Mr conjugate (consisting of one molecule of antibody linked to one molecule of ricin) and unconjugated MoAb. Fractions 97-105 and 125-133 (unconjugated ricin Mr65,000) were analysed further by affinity chromatography (see below). The chemistry of the thiolation and coupling is illustrated in Figure B2a.

Conjugation Using SPDP: Ricin was coupled to MoAb using the heterobifunctional crosslinkinbg agent SPDP. This method couples the proteins by the introduction of thiol groups which can be reacted to form stable disulfide linkages between proteins. SPDP was dissolved in dioxane to give a 2 mg/ml solution. Given amounts of SPDP were reacted with ricin or MoAb in phosphate buffer pH 7.0 or borate buffer pH 8.0 at room temperature for 30 minutes in the quantities shown in Table B1. The number of SPDP residues per molecule was previously determined by calculating the liberation of pyridine-2-thione residues from the SPDP molecule under reducing conditions. Release of pyridine-2-thione was measured by monitoring increasing absorbance at 343 nm on a Pye-Unicam SP8-400 spectrophotometer. Following 30 minutes incubation, thiolated MoAb and ricin were dialysed against the borate buffer for 16 to 20 hours at 4° C. The thiolated MoAb was dialysed against the acetate buffer for 2 hours, and then the SPDP thiol group on the MoAb molecule was reduced with DTT (3.4 mM final concentration) for 30 minutes at room temperature, and further dialysed against deoxygenated acetate buffer for 8 to 10 hours to remove unreacted DTT. Deoxygenation of the acetate buffer prevents oxidation of the sulfhydryl residues. The immunotoxin was then found by reacting thiolated ricin with the thiolated MoAb for 2 hours at room temperature. The reaction was terminated with the addition of N-ethylmaleimide (6.6 mM final concentration). The mixture was allowed to stand at room temperature for 1 hour, and the subjected to gel permeation chromatography on Sephacryl S-300 as described previously. The chemistry of the SPDP thiolation and coupling is illustrated in Figure B2b.

The concentration of ricin in the conjugate fractions was determined from the radioactivity, whereas the concentration of MoAb was ascertained by measuring the absorbance at 280 nm and subtracting the calculated absorbance due to the ricin (using absorption coefficients $E^{1\%}{}_{1cm}$ = 11.8 for ricin and 14.0 for IgG).

Galactose Binding Affinity: Two different matrices were employed to further purify the immunotoxins based on the affinity of ricin for Sepharose and were used to determine whether the coupling procedure altered the binding specificity of the bound ricin (conjugated) and free ricin (unconjugated). Conjugate fractions 97 to 105 (see Figure B1) and unconjugated ricin, fractions 125 to 133 (Mr65,000), were pooled and extensively dialysed against the phophate buffer. Lactose-Sepharose and Sepharose - 4B were used to determine the galactose binding ability of the immunotoxin and free (unconjugated) ricin. The immunotoxin and ricin fractions were absorbed onto columns at 4° C and also at room temperature. The solutions were passed through the column 3 times, the columns washed with phosphate buffer to remove the material that did not bind and the bound protein was then eluted with phosphate buffer contain 100 mM lactose.

Competitive Binding Assay: The Ly-2.1 MoAb was tested on the Ly-2.1$^+$ cell line E2 after each labeling for alteration in binding ability. Binding was assessed by comparison of modified (SAMSA treated) and

untreated anti-ly-2.1 in their ability to compete with a nonsaturating amount of $125_I$-anti-Ly-2.1 for binding to $2\text{-}_4x10^5$ cells; the CEM cell line was used as a nonreactive control. In this assay, 25 mul of the untreated or thiolated MoAb (1 mg/ml in 0.5% BSA) was serially diluted and iodinated MoAb ($2x10^5$ cpm/well) was added to each well. The cells were then added, incubated for 30 minutes on ice and nonbound material was removed by washing. The cell pellets were counted and the results expressed as the percentage inhibition of the maximum counts bound.

Protein Synthesis Assays: The inactivation of cellular protein synthesis was used as a measure of the in vitro effect of immunotoxins. Briefly, cells ($8x10^5$) were incubated for 30 to 60 minutes with varying dilutions of the immunotoxin or ricin in phosphate buffer with 200 mM lactose and then washed three times to remove any unbound material. The cell pellet was resuspended in leucine free RPMI-1640 medium and plated out onto a 96 well flat bottom microtitre plate in triplicate. The cells were then pulsed with [$3_H$]leucine (1muCi/well) and incubated at 37°C for 16 to 24 hours. The cells were harvested onto glass fiber filters and the incorporated radioactivity was counted with a scintillation counter. The results were expressed as the percentage inhibition of [$3_H$]leucine incorporation relative to control cells which received PBS as treatment.

Inhibition of Cytotoxicity: In this assay the cytotoxicity of the immunotoxin was inhibited by free MoAb. Cells ($8x10^5$) were treated with a constant amount of immunotoxin (220 ng/ml) and 2 different MoAb were than added in serial dilutions (anti-Ly-2.1 reactive and anti-Ly-1.1 nonrreactive). The cells were incubated for 1 hour and a cytotoxicity assay performed; the results were expressed as the percentage inhibition of cytotoxicity.

RESULTS

The study was conducted in 3 phases: (a) establishing optimum conditions for the coupling of ricin to MoAb; (b) characterization of the immunotoxin, including its binding to affinity matrices and SDS gel analysis; and (c) testing the potency of the immunotoxin on cell lines in vitro.

Coupling of Ricin to Antibody Ricin was chemically linked to MoAb via a disulfide bridge with the use of two bifunctional crosslinkers as described - the reaction of ricin with a 10-fold excess of SPDP introduced 2 to 3 pyridyldithio groups based on the release of 2-pyridinethione (24), as shown in Table B1 . Similarly, treatment of MoAb with a 5-fold excess of SAMSA introduced 2 to 3 protected sulfhydryl groups determined by reactions with dithiobis-2-nitrobenzoic acid (Table B2) after deprotection with hydroxylamine (25). Subsequent reaction of the thiol groups of ricin yielded a ricin-antibody conjugate. The coupling method using SAMSA has several advantages; it precludes the use of thiol reagents for deprotection and therefore prevents unnecessary dilution or removal of excess reagents, and it reduced the oxidation of free sulfhydryl groups and does not require low pH buffers. Of the ricin initially used, 70% was recovered in solution, 60% of this was bound to MoAb, 35% in the 210 KD complex (1 ricin molecule bound to 1 MoAb molecule), 25% in larger aggregates (2:1, 3:1, ..) and 40% remained unconjugated (Table B3), These yields were superior to the second method used in which MoAb was thiolated with SPDP. As shown in Table B3, the final yield of conjugated ricin was relatively low, being 10-15% of the ricin used initially.

Conjugates were examined by SDS gel electrophoresis. As found therefrom a 5% nonreduced gel indicated the immunotoxin consisted of unconjugated MoAb (150 KD) and a 210 KD complex (in a 1:1 molar ratio, determined by optical density measurements and radioactivity). The autoradiograph illustrated the 210 KD band to be radioactive and therefore indicated a complex that had one ricin molecule bound to one MoAb molecule. Additionally, under reducing conditions the immunotoxin was observed to consist of the heavy and light chains of immunoglobulin (50 KD and 25 KD respectively) and the expected 34, 32, and 30 KD subunits of ricin.

Galactose Binding Affinity of Immunotoxins and Ricin The galactose binding property of a whole ricin-anti-Ly-2.1 conjugate and SPDP thiolated ricin were first assessed on a Lactose-Sepharose column (2 ml) by affinity chromatography based on the affinity of ricin for sepharose. As shown in Figure B4, when thiolated unconjugated ricin was passed down a Lactose-Sepharose column, ricin bound strongly to the column through its galactose receptors. Less than 5% was eluted from the column with PBS and lactose (100 mM) was required to recover 90% of the material. However, when the rici-anti-Ly-2.1 conjugate was applied to the Lactose-Sepharose column 86-90% of the conjugate, as detected by the radioactivity on the ricin moiety, failed to bind or be significantly retarded by the affinity matrics, when the column was washed with 2 column volumes of phosphate buffer. An additional8% of bound protein was eluted with the same buffer containing 100 mM lactose. Similar results were obtained with 6 different MoAb conjugated to ricin and the results are summarized in Table B4.

Antibody Activity of Thiolated Antibody The antibody activity of thiolated MoAb was tested. Thiolation of the MoAb with SAMSA did not significantly effect its binding to target cells. Anti-Ly-2.1 thiolated with a 12

fold excess of SAMSA per MoAb molecule. This did not significantly alter the binding capability of the MoAb to E2 reactive target cells in a competitive binding assay using radioiodinated anti-Ly-2.1. As shown in Figure B5, the thiolated MoAb had similar activity to unmodified MoAb and increasing the concentration of unmodified anti-Ly-2.1 or thiolated anti-Ly-2.1 had equal inhibitory effects on the binding of $125_I$-labeled anti-Ly-2.1 to E2 target cells.

The Ly-2.1 MoAb was reacted with various amounts of SPDP and the effect on antibody activity measured with the use of rosetting assay (26). SPDP thiolation had no affect on antibody binding activity unless a vary high substitution level (<9.2 molecules SPDP/molecule anti-Ly-2.1) was used. It should be noted that only a 6 fold excess of SPDP was used for the conjugation procedure which led to the incorporation of 2 to 3 molecules of SPDP per MoAb molecule.

Binding of Conjugates to Cell Lines The antibody activity of the conjugated proteins was measured by the rosetting assay. The titers of antibody before and after conjugation were determined as the dilution at which 50% of the target cells demonstrated rosettes. As shown in Figure B6 the activites were comparable for both the unmodified anti-Ly-2.1 and ricin-anti-Ly-2.1 conjugate on E2 target cells, indicating that the coupling did not effect the antibody reactivity. However, it should be noted that noncoupled MoAb in the ricin-anti-Ly-2.1 conjugate could partially account for the conjugate binding. The reactivity was also tested by using a second cell line, D1, the E2 and D1 cell lines being high and low $Ly-2^+$ variants respectively of the ITT(1)75NS cell line . Both these cell lines were identical with the exception of their Ly-2.1 antigen density. As shown in Figure B6, there is a difference in the reactivity of conjugates with the two cell lines and clearly the binding of the conjugate was dependent on the concentration of reactive antigen binding sites on the target cells. No binding was found with a nonreactive ricin-anti-Ly1.1 conjugate on the E2 cell line ($Ly-1.1^-$). These results indicated that whole ricin was successfully conjugated with the MoAb and that the conjugated ricin did not play a role in the binding activity of the conjugates.

Cytotoxicity of the Immunotoxins The specificity of the ricin toxin, which normally binds, enters, and kills cells through receptors containing galactose, has been altered by covalently binding it to a MoAb. Hybrid conjugates (immunotoxins) of ricin and MoAb were synthesized with the bifunctional crosslinking agent SAMSA and SPDP and were shown by affinity chromatography not to bind to the galactose residues of Lactose-Sepharose or Sepharose-4B. Furthermore, the reactivity of the immunotoxins was dictated by the specificity of the covalently bound MoAb. It was, therefore, necessary to show that the inhibition effects produced were specific. Three procedures were performed.

Procedure 1 The immunotoxins, free ricin and MoAb, were assayed for inhibition of protein synthesis in reactive target cells. The MoAb alone at concentrations as high as 10 mug/ml did not inhibit protein synthesis in the target cells (not shown).

Figure B7 shows the dose-response of ricin and ricin-anti-TFR in CEM ($TFR^+$) cells. The anti-TFR immunotoxin on a molar basis, in the absence of lactose was as potent as free ricin (Figure B7a) and reduced the capacity of cells to incorporate ([$3_H$] leucine by 50% at 30 ng/ml. Lactose, which competitively blocks ricin binding to cells, inhibited the toxicity of ricin 40-fold in CEM cells. In contrast, the anti-TFR immunotoxin was inhibited less than 2-fold by lactose (Figure B7b). Thus, in the presence of 100 mM lactose, CEM cells were 40-fold more sensitive to the immunotoxin than ricin. These results were confimed in repeated experiments with other immunotoxins. For example, the cytotoxic activity of a ricin ($IO_4^-/BH_3^-$ treated) - anti-Ly-2.1 conjugate was tested on reactive target cells. The conjugate inhibited protein synthesis in vitro as shown in Figure B8. Lactose inhibited the observed toxicity of ricin, at least 40-fold in E3 cells, however, the observed inhibition of the immunotoxin was not altered by the addition of lactose and 50% inhibition of [$3_H$] leucine incorporation occurred at 65 ng/ml.

Procedure 2 To determine whether the observed inhibition was due to specific binding of the MoAb, the cytotoxic activity of two immunotoxins, an antigen reactive ricin-anti-Ly-2.1 immunotoxin and a nonreactive ricin-anti-Ly-1.1 immunotoxin was tested on E3 cells (Ly1-,2+). As shown in Figure B9a, there was no significant loss of cytoxicity by linkage or ricin to the Ly-2.1 MoAb, in the absence of lactose. This immunotoxin reduced the capacity of cells to incorporate [$3_H$] leucine by 50% at 45 ng/ml, whereas, the nonreactive ricin-anti-Ly-1.1 immunotoxin was ineffective and showed a low toxic effect on the E3 cells, where a concentration as high as 1000 ng/ml was required to inhibit protein synthesis. Thus, in the absence of lactose the reactive immunotoxin was 20 times more effective at inhibiting protein synthesis in E3 cells than a nonreactive immunotoxin. When 100 mM lactose was introduced into the assay (Figure B9b) the cytotoxicity of nonconjugated ricin was inhibited (50% inhibition of [$3_H$] leucine incorporation occurred at greater than 1000 ng/ml) whereas the cytotoxicity of the anti-Ly-2.1 immunotoxin was not significantly altered. The nonreactive immunotoxin however, was not toxic to E3 cells at concentrations as high as 104 ng/ml. Thus, in the presence of lactose, E3 cells were more than 200-fold more sensitive to the reactive immunotoxin than the nonreactive immunotoxin.

Procedure 3 Additional experiments were performed in which the cytotoxic activity of three immunotoxins was tested on reactive and nonreactive target cells. The immunotoxins were able to inhibit protein synthesis in cultured cells bearing the appropriate target antigen. The MoAb after conjugation were found to maintain their specificity.

The effect of an anti-TFR immunotoxin on $[3_H]$ leucine incorporation into CEM cells ($TFR^+$) and E3 cells ($TFR^-$) is shown in Figure B10a. Both cell lines were equally inhibited by ricin in the absence of lactose (50% inhibition of protein synthesis occurred at 20 ng/ml). The anti-TFR immunotoxin resulted in greater than 50% inhibition of $[3_H]$ leucine incorporation by CEM (reactive) cells at 6 ng/ml. Whereas 80 times more immunotoxin was required to inhibit protein synthesis in the nonreactive E3 cells.

Two different preparations of ricin-anti-Ly-2.1 demonstrated marked inhibition of protein synthesis in E3 ($Ly\text{-}2.1^+$) cells. EL4 cells were more sensitive to ricin ($IO_4^-/BH_3^-$) treated than E3 cells (Figure B10c), whereas BW5147 cells were 3 times less sensitive to ricin (data not shown). EL4 and BW5147 target cells that lacked the Ly-2.1 specificity were assayed for sensitivity to ricin-anti-Ly-2.1. In Figure B10b and B10c the sensitivity of these cell lines to the anti-Ly-2.1 immunotoxin in the absence of lactose is shown. No inhibition of protein synthesis was seen at the highest concentration of the immunotoxin (>1000 ng/ml) tested, whereas the anti-Ly-2.1 immunotoxin showed marked inhibition protein synthesis in E3 ($Ly\text{-}2.1^+$) cells and 50% inhibition of $[3_H]$ leucine incorporation of E3 cells occurred at 100 ng/ml (Figure B10b) and 60 ng/ml (Figure 10c) for the ricin-anti-Ly-2.1 and ricin ($NaIO_4^-/BH_4^-$ treated) -anti-Ly-2.1 preparations respectively. Thus, no antibody-mediated toxicity of the anti-Ly-2.1 immunotoxin was not detected in $Ly\text{-}2.1^-$ cells.

Inhibition of Cytotoxicity The mechanism of the specific cytotoxic action of the immunotoxin relied in part on the MoAb moiety binding to the target cells via a cell surface receptor. The specific cytotoxicity of a ricin ($NaIO_4^-/BH_4^-$ treated) - anti-Ly-2.1 conjugate on E3 cells was blocked by the presence of excess anti-Ly-2.1 as shown in Figure B11. The simultaneous addition of a 20 fold excess of anti-Ly-2.1 (10 ng/ml) with the immunotoxin caused a 50% reduction in the cytotoxicity of the anti-Ly-2.1 immunotoxin to E3 target cells. In contrast, excess anti-Ly-1.1 (an isotype control MoAb directed to an irrelevant antigen) did not alter the cytotoxicity of the anti-Ly-2.1 results indicate that the cytotoxicity of the immunotoxin is mediated via their antibody binding ability.

## DISCUSSION

In this part the coupling of ricin to antibody is reported, the most important result obtained was the immunotoxins produced were devoid of galactose binding. The study concentrated on several different aspects: (a) the design of an efficient coupling method which resulted in high yields of immunotoxin; (b) characterisation of the immunotoxin; (c) determining the binding properties of the immunotoxin; and (d) measuring the biological properties of the covalently coupled ricin and antibody. The use of whole ricin as an antitumor agent has been quite restricted and for obvious reasons, as this toxin is capable of binding to all cells. Nevertheless, a phase I clinical trial has been conducted with the use of native ricin (27) and a therapeutically safe dose was found to have only marginal antitumor effects. The concept of covalently coupling the whole toxin to tumor specific antibodies has been extensively researched (28,29) and successful studies have shown that whole ricin immunotoxins are powerful reagents in vitro in the presence of lactose.

Coupling procedures previously used for the covalent attachment of ricin to MoAb resulted in very poor yields of immunotoxin, presumably due to different reasons including the use of reducing agents to introduce free sulfhydryl groups into MoAb, low pH buffers which resulted in the precipitation of concentrated protein solutions and rapid oxidation of free sulfhydryl groups during coupling reaction. Thiolation of MoAb with SAMSA overcame a number of these problems, i.e. deprotection of the sulfhydryl groups of the MoAb and coupling to thiolated ricin is carried out in the same buffer as the coupling reaction. In this part, a number of parameters were carefully monitored, particular attention was paid to the number of active thiol groups introduced, the molar ratio of ricin and MoAb in the reaction mixture and the reaction time. The covalent attachement of ricin to MoAb resulted in up to 60% of the recovered ricin being conjugated, 35% of the ricin was present in a 21 KD species where one ricin molecule (Mr 65,000) was attached to one MoAb molecule (Mr 150,000) (Table B3). Thiolation of the MoAb with SAMSA with ricin with SPDP did not significantly change the binding ability of these proteins. Coupling reactions specific for a particular amino acid residue could theoretically denature the antigen binding site of certain antibodies during coupling or could result in extensive precipitation of protein due to the alteration of its charge. Neither occurred to any extent with SAMSA, and up to 4 molecules of SAMSA could be incorporated per MoAb molecule with the retention of 100% of the antibody activity (Table B2). SPDP thiolated ricin (2-3 SPDP residues/ricin

molecule) maintained an active galactose binding site (Table B4). The blockage of the galactose binding site was a specific event and a direct consequence of the close proximity of the Fc portion of the MoAb sterically blocking the galactose binding sites on the ricin molecule. When whole ricin was covalently attached to MoAb and the purified immunotoxin (210 KD) passed down Lactose-Sepharose affinity column, 90% of the immunotoxin failed to bind the galactose residues. Immunotoxins produced with whole IgG anti-Ly-2.1 (IgG2a) and anti-TFR (IgG1) were equally cytotoxic in the presence or absence of lactose (Figure B7-B9).

The rationale for constructing an immunotoxin is that a cytotoxic agent coupled to a MoAb is both cytotoxic and selective in action. The success of such an approach depends in part on whether the toxin and antibody retain their biological activities after conjugation. A number of studies were then performed to demonstrate that the immunotoxin reacted specifially with target cells carrying the antigen to which the antibody was directed and did not react with tumor cell lines which lacked this antigen. The anti-Ly-2.1 immunotoxin was shown to be effective and specific for reactive cells in vitro. A dose of 200 ng/ml efficiently reduced the protein synthesis of E3 target cells by >90%, whereas a nonreactive immunotoxin was not cytotoxic to these cells at the same molar concentration. Specific cytotoxicity was shown to be dependant on the anti-Ly-2.1 immunotoxin binding to the cell surface receptors since cytotoxicity was only inhibited upon addition of excess anti-Ly-2.1 but not by the addition of an unreactive MoAb (Figure B11). In addition, an anti-TFR immunotoxin was shown to be 4 times more toxic to CEM, TFR positive target cells than free ricin, >90% inhibition of protein synthesis was achieved with 50 ng/ml of the immunotoxin without affecting receptor negative E3 cells. Furthermore, 90 times more immunotoxin was needed to inhibit the protein synthesis of the nonreactive E3 cells (Figure B10a).

The selection of whole ricin for in vivo immunotherapy was based on the biological importance the B chain plays in the potentiation of the A chains patent cytotoxicity (5-7). The advantage of ricin conjugated to MoAb is that the cytotoxic nature of the toxin can be focussed to the surface of a particular target cell resulting in the specific killing of the cell. The blockage of the galactose binding site was a fortuitous, but reproducible finding as more than eight immunotoxins have been produced with different MoAb which are similarly specifically toxic. There have been few studies performed with whole ricin immunotoxins, although a thioether linked immunotoxin was prepared with a blocked galactose binding site (30) and in vitro studies were reported.

In this Part ricin was covalently attached to MoAb with the use of a disulfide bridge. The cytotoxicity of the immunotoxins was restricted to MoAb reactive cells and on the basis of the encouraging in vitro results the in vivo efficacy of the blocked immunotoxins was examined and reported in Parts C and D.

REFERENCES part B


10. HYMAN R, STALLINGS V. Complementation patterns of Thy-1 variants and evidence that antigen loss variants "preexist" in the parental population. JNCI 1974; 54:429-436.

11. GHOSE T, GUCLU A, TAI J, et al. Immunoprophylaxis and immunotherapy of EL4 lymphoma. Eur J Cancer 1977; 13:925-935.

12. SMYTH MJ, PIETERSZ GA, CLASSON BJ, et al. The specific targetting of chlorambucil to tumors, JNCI 1985; 76:503-510.

13. FOLEY GE, LAZARUS H, FARBER S, et al. Continuous culture of human lymphoblasts from peripheral blood of a child with acute leukemia. Cancer 1965; 18:522-529.

14. HOGARTH PM, HENNING MM, McKENZIE IFC. Monoclonal antibodies to murine Ly-2.1 cell surface antigen. Immunology 1982; 46:135-144.

15. HOGARTH PM, POTTER TA, CORNELL FN, et al. Monoclonal antibodies to murine cell surface antigens. J Immunol 1980; 125:1618-1624.

16. PANACCIO M. Monoclonal antibodies to the human transferrin receptor. BSc (Hons) report. Parkville, Victoria, Australia: Department of Pathology, University of Melbourne, 1983.

17. THOMPSON CH, JONES SL, PIHL E, et al. Monoclonal antibodies to human colon and colorectal carcinoma. Br J Caner 1983; 47:595-605.

18. THOMPSON CH, JONES SL, WHITEHEAD RH, et al. A human breast tissue-associated antigen detected by a monoclonal antibody. JNCI 1983; 70:409:419.

19. MICHAELIDES M. Studies of the Ia and Ly alloantigens in the mouse. PhD Thesis, Parkville, Victoria, Australia: Department of Pathology, University of Melbourne, 1981.

21. THORPE PE, DETRE SI, FOXWELL BMJ, et al. Modification of the carbohydrate in ricin with metaperiodatecyanoborohydride mixtures. Effects an toxicity and in vivo distribution. Eur J. Biochem 1985; 147:197-206.

22. GREENWOOD FC, HUNTER WM, GLOVER JS. The preparation of $^{131}$I-labelled human growth hormone of high specific radioactivity. Biochem J 1963: 89:114:123.
24. CARLSSON J, OREVIN J, AXEN R. Protein thiolation and reversible protein-protein conjugation. Biochem J 1978; 173:723-737.
25. ELLMAN GL. Tissue sulfhydryl groups. Arch Biochem Biophys 1959; 82:70.
26. PARISH CR, McKENZIE IFC. A sensitive rosetting method for detecting sub populations of lymphocytes which react with alloantisera. J. Immunol Methods 1978; 20:173-183.
27. FODSTAD O, KVALHEIN G. GODAL A, et al. Phase I study of the plant protein ricin. Cancer Res 1984; 44:862-865.
28. JANSEN FK, BLYTHMAN HE, CARRIERE D, et al, Immunotoxins: Hybrid molecules combining high specificity and patent cytotoxicity. Immunol Rev 1982; 62:185-216.
29. VITETTA ES, KROLICK KA, MIYAMA-INABA MM, et al. Immunotoxins: a new approach to cancer therapy. Science 1983; 219:644-650.
30. THORPE PE, ROSS WCJ, BROWN ANF, et al. Blcokagte of the galactose binding sites of ricin by its linkage to antibody. Specific cytotoxic effects of the conjugates. Eur J. Biochem 1984; 140:63-71.

Table 8l

(a) SPDP modification of MoAb[1]

| Molar excess SPDP | No. molecules SPDP/molecule MoAb | |
|---|---|---|
| | anti-Ly-2.1 | anti-Ly-1.1 |
| 2 | - | 0.2 |
| 5 | - | 1.8 |
| 6 | 2.8 | - |
| 10 | - | 2.5 |
| 12 | 4.1 | - |
| 18 | 7.2 | - |
| 24 | 9.2 | - |
| 50 | - | 11.1 |

(b) SPDP modification of Ricin[1]

| Molar excess SPDP | No. molecules SPDP/molecule Ricin | |
|---|---|---|
| | pH 7.0 | pH 8.0 |
| 4 | 0.5 | - |
| 5 | - | 2.0 |
| 6 | 0.9 | - |
| 7.5 | 1.0 | - |
| 10 | - | 3.2 |
| 15 | - | 3.9 |
| 20 | - | 5.5 |

1 The number of SPDP molecules introduced per protein molecule was determined by calculating the liberation of pyridine-2-thione.

Table B2

| SAMSA modification of MoAb[1] | |
|---|---|
| Molar excess SAMSA | No. molecules SAMSA/molecule anti-Ly-2.1 |
| 5 | 2.9 |
| 10 | 4.3 |

[1] The number of SPDP molecules introduced per protein molecule was determined by reactions with dithiobis-2-nitrobenzoic acid.

Table B3

| Comparison of the coupling efficiency of two different procedures used to covalently link ricin to MoAb. | | | |
|---|---|---|---|
| | % INITIAL RICIN[1] | | |
| | Procedure 1[2,3] (SAMSA/SPDP) | | Procedure 2[2] (SPDP/SPDP) |
| ricin-MoAb (1:1) | 29 | 25 | 13 |
| ricin-MoAb (2:1,3:1,...) | 12 | 17 | 8 |
| unconjugated ricin | 29 | 27 | 48 |
| Recovery | 70 | 69 | 69 |

[1] The results are expressed as the % initial ricin used.
[2] See materials and methods for details.
[3] Two different conjugations are reported with procedure one to illustrate the reproducibility of this method.

Table B4

(a) Binding affinity of SPDP modified ricin.

| % Initial ricin | Lactose-Sepharose | | Sepharose-4B |
|---|---|---|---|
| | Ricin | Modified Ricin[1] | Ricin |
| Bound | 78 | 62 | 61 |
| Not bound | 5 | 2 | 9 |
| Recovered | 83 | 64 | 70 |

(b) Binding affinity of ricin-anti-Ly-2.1 on Lactose-Sepharose

| % Initial ricin | Lactose-Sepharose | |
|---|---|---|
| | 22°C | 4°C |
| Bound | 7 | 8 |
| Not bound | 77 | 86 |
| Recovered | 84 | 94 |

[1] Ricin chemically treated with sodium metaperiodate and sodium cyanoborohydride.

(c) Binding affinity of ricin-MoAb conjugates on Lactose-Sepharose

| Immunotoxin | % Initial ricin | | |
|---|---|---|---|
| | Bound | Not Bound | Recovered |
| anti-Ly-1.1 | 0.5 | 89 | 90 |
| anti-TFR | 23 | 77 | 100 |
| anti-Ly-2.1 (modified ricin) | 7 | 87 | 94 |
| 250-30.6 | 16 | 83 | 99 |
| anti-HLA | 7 | 75 | 82 |
| 17.1/2 | 6 | 80 | 86 |

Part C

This part investigates the in vivo stability of ricin and ricin ($10_4^-$/$BH_3^-$ treated) and evaluates the use of disulfide linked antibody conjugates for the treatment of tumors in vivo. The ability to successfully use

whole ricin was due to the loss of the galactose binding sites on binding ricin to antibody to produce conjugates with limited nonspecific binding.

MATERIALS AND METHODS

Mice (56BL/6 x BALB/c)F1 (B6CF1) mice were produced in the Department of Pathology, University of Melbourne.

Tumor Cells A radiation induced murine thymoma ITT(1)75NS E3 (8) was grown in 10% newborn calf serum in DMEM. The human T-cell leukaemia line CEM (9) was maintained in RPMI-1640 with the same additives. The E3 cell line was maintained by serial passage into the peritoneum of B6CF1 mice. For in vivo experiments the cells from the ascitic fluid were washed and centrifuged (1,500 rpm for 5 minutes) twice in DMEM and PBS, resuspended in PBS, and injected subcutaneously or intraperitoneally into B6CF1 mice.

Carbohydrate Modification of Ricin with $IO_4^-/CNBH_3^-$ mixtures The method of carbohydrate modification has been described in detail in Part B. Extensive details of the characterization of ricin modified by $IO_4^-/CNBH_3^-$ treatment have been published elsewhere (7) but to briefly summarize, the properties were as follows. A slight (<500 D) reduction in molecular mass of both A and B chains was seen when ricin treated with $IO_4^-/CNBH_3^-$ was analyzed on SDS polyacrylamide gel electrophoresis in the presence of 2-mercaptoethanol. This may have been due to the elimination of CH.OH from carbohydrate residues. There was no significant change in the amino acid composition of ricin after treatment. No splitting of the disulfide bond between the A and B chains was detectable when modified ricin was analysed on SDS gels in the absence of 2-mercaptoethanol. There was no significant loss in the ribosome-damaging effect of the A-chain moiety of the modified toxin. Similarly, the carbohydrate-binding property of the B-chain was fully preserved, as also shown in Part B by its Sepharose-binding ability. The extent of the carbohydrate modification depended on the duration of the treatment, and resulted in the maximum destruction of 70% of the mannose residues and all xylose and fucose residues.

In vivo Distribution of Native and Modified Ricin ($IO_4^-/CNBH_3^-$ treated) This study compared the biodistribution of ricin and $IO_4^-/CNBH_3^-$ treated ricin in B6CF1 mice. Ricin (50 mug, 1 mg/ml) or modified ricin ($IO_4^-/CNBH_3^-$ treated) was labeled in the presence of 100 mM lactose with $^{125}I$ using chloramine-T. Groups of 3 mice were sacrificed 30 minutes after the injection of labeled toxin. Each mouse was given an injection in the tail vein of 0.12 mug of the toxin (Specific activity = 4.6-5.8x106 cpm/mug). The biodistribution of $^{125}I$-label was determined by counting the radioactivity of blood, liver, spleen, kidney and heart from these mice. Organs were weighed and the distribution of isotope measured in a gamma counter. Blood activity was calculated on the assumption that the overall blood volume represents 7% of the total body weight. Organ distributions were then calculated as a percentage of the original injected dose found in the organ.

Preparation of ricin-antibody conjugates Ricin or modified ricin ($IO_4^-/CNBH_3^-$ treated) was conjugated to MoAb by a disulfide bridge using two bifunctional crosslinking reagents, SAMSA and SPDP, according to the method previousl described in Part B. The resultant ricin-antibody conjugates had a diminished galactose binding capacity.

The native ricin-antibody conjugates were further purified from unconjugatad MoAb by affinity chromatography on a column of Concanavalin-A immobilized to Sepharose (10). According to this method unconjugated MoAb, which did not bind the lectin, was discarded in the filtrate while ricin because of its mannose containing oligosaccharide, binds to Con-A. Thus only MoAb bound to ricin is retained on the affinity column. The immunotoxin was eluted in a solution of 0.5 M methyl-alpha-D-mannoside.

Radiolabeling of ricin, modified ricin and ricin- antibody conjugates Ricin, modified ricin ($IO_4^-/CNBH_3^-$ treated) and ricin-MoAb (purified on Con-A-Sepharose to remove unconjugated MoAb) were labeled using the iodobead method: one iodobead (Pierce Chemical Company, Rockford, Illinois, USA) and 200 mul of protein (approx. 10 mug) were allowed to stand at room temperature for 5 minutes and 2.5 mCi of carrier-free $Na^{125}I$ (Amersham International Ltd, Amersham, England) then added and the reaction monitored for a further 5-15 minutes. The iodinated protein was separated from free iodine by gel filtration using a PD-10 Sephadex column.

Antibody activity and specificity of $^{125}I$-labelled ricin-antibody conjugates The immunoreactivity of the radiolabeled immunotoxin preparations was measured by a direct in vitro cell binding assay. The binding ability of $^{125}I$-labeled ricin-anti-Ly-2.1 was tested on two different target cell lines, one being reactive with the immunotoxin, the other nonreactive. Cells used for the assay were E3 (Ly-2.1$^+$) and CEM (Ly-2.1$^-$). Cells ($1x10^6$) were added to tubes pre-blocked with 1% BSA in PBS and the $^{125}I$-labeled immunotoxin was added at doubling dilutions (in the presence or absence of 200 mM lactose) and the tubes were incubated on ice for one hours. The cells were then washed with PBS or PBS containing 100 mM lactose (4x5 mls),

and the cell pellets counted for one minute in a gamma counter.

Competition assay In this assay the binding of $^{125}$I-labeled ricin-anti-Ly-2.1 to E3 reactive target cells was inhibited by free MoAb. The labeled immunotoxin had a specific activity of 3x$10^7$ cpm/mug. The association of iodinated immunotoxin with cells was examined. E3 cells (3x$10^6$) were added to tubes pre-blocked with 1% BSA in PBS and $^{125}$I-labeled ricin-anti-Ly-2.1 (13 ng/ml) and various dilutions of unlabeled anti-Ly-2.1 or anti-Ly-1.1 were added. The cells were incubated on ice for 30 minutes and then washed 4 times with PBS to remove any unbound material. The cell pellets were counted for one minute in a gamma counter and the results were expressed as the percent inhibition of the maximum counts bound.

Plasma clearance of ricin, modified ricin ($IO_4^-$ /$CNBH_3^-$ treated) and ricin-antibody conjugates The following preparations of ricin were labeled with $^{125}$I using an iodobead to a specific activity of 1.2 - 1.7 x $10^7$ cpm/mu g: (a) untreated ricin; (b) ricin treated with $IO_4^-$/$CNBH_3^-$; (c) ricin-anti-Ly-2.1 purified by affinity chromatography on a Con-A-Sepharose column. Groups of 4 female B6CF1 mice were injected with the iodinated protein solutions via the tail vein. Each mouse received either 0.24 - 0.3 mug of the toxins or 0.81 mug of the immunotoxin in 100 - 150 mul PBS. Blood samples were collected at timed intervals in heparin-treated tubes and plasma was separated by centrifugation. The time-course plasma level of the radiolabeled protein was then evaluated and the results expressed as a percentage of the initial dose injected. Blood activity was calculated on the assumption that the overall blood volume represents 7% of the total body weight.

Serum samples (5 ml) were further analyzed by SDS-gel electrophoresis using 5% and 12.5% acrylamide gels in order to determine the stability of the radiolabeled proteins in vivo.

In vivo treatment of murine tumors In a survival study, tumor cells were grown in the peritoneum of mice in the ascites form and treatment was delivered into the ascites fluid. The percentage survival of mice in each group was plotted as a function of time. In a tumor growth study, tumor cells were injected subcutaneously into the abdominal wall of the mouse and allowed to develop into palpable tumors measuring 0.5 - 0.8 cm in diameter before treatment was commenced. Mice were then subjected to a series of intravenous treatments, and the size of the tumors measured daily with a caliper square. Measurements were made along the perpendicular axes of the tumors, and the data was recorded as mean tumo size (product of two diameters ± standard error of the mean). Experimental groups of 10 - 40 mice of the same sex were used in each experiment.

Toxicity to mice Ricin and ricin-antibody conjugate solutions in PBS were injected intraperitoneally or intravenously into groups of three to five male B6CF1 mice. The LD50 was calculated according to Well (11).

Histopathology B6CF1 mice weighing 25 g and 30 g were injected intraperitoneally with ricin (3.3 mug/kg body weight) or ricin that had been treated with sodium metaperiodate and sodium cyanoborohydride or of ricin-antibody conjugates (3.3 mug ricin/kg). The animals were sacrificed 1 to 2 days later and the following organs were removed and fixed in 10% formalin: liver, spleen, pancreas, kidney, heart and lung. The organs were sectioned and stained with haematoxylin and eosin for light microscopy.

RESULTS

The objectives of this study were firstly to examine the plasma clearance of ricin after intravenous injection and secondly to assess the in vivo efficacy of the ricin-antibody conjugates sythesized with reduced capacity to bind to galactose.

In vivo biodistribution of ricin and modified ricin ($IO_4^-$/$CNBH_3^-$ treated) The biodistribution of $^{125}$I-labeled ricin and modified ricin was examined by injecting mice intravenously with the toxins and determining the relative amounts of radiolabel accumulated in the tissues. As shown in Table C1, ricin was rapidly cleared from the bloodstream and only 9% of the toxin remained in the blood 30 minutes after injection. The liver was the major site of clearance and 36% of the injected ricin was trapped by the liver. The radioactivity per gram of liver was 6 times greater than in the blood. Ricin also accumulated in the spleen as shown by the greater levels of radioactivity per gram of tissue (24.4% injected dose/g). The kidneys and the heart contained small amounts of radioactivity. The kidneys had twice the amount food in the blood per gram, whereas the heart had a similar level.

Ricin that had been treated with metaperiodate and cyanoborahydride was removed much less efficiently than the native ricin by the liver and only 8% of the modified toxin was found in the liver 30 minutes after injection, 4 to 5 times less than the observed level of native ricin (per gram of tissue). 22% of the modified toxin was still present in the blood (Table C1), twice the level of native ricin. The spleen, kidney and heart all took up the modified ricin to the same extent as native ricin. Thus the modified ricin

($IO_4^-$/$CNBH_3^-$ treated) had a greater in vivo blood circulation life than native ricin. The carbohydrate portion of native ricin is recognized by the liver end spleen and is thereby cleared from the bloodstream by the hepatic reticuloendothelial system.

Specificity of $^{125}$I-labeled ricin-anti-Ly-2.1 The $^{125}$I-labeled ricin-anti-Ly-2.1 conjugate was tested in different serological assays to determine whether the labeling procedure damaged or altered the binding or specificity of the MoAb.

Binding of $^{125}$I-labeled-anti-Ly-2.1 to antigen-positive E3 cells and antigen-negative CEM cells in the presence or absence of lactose is shown in Figure C1a. Clearly, there was a major difference in the binding of the labeled immunotoxin to the reactive cells (E3) when compared to that of the nonreactive cells (CEM). The reactive cells bound up to 10 times more immunotoxin then did the nonreactive cells, also the binding of the immunotoxin to reactive cells was slightly inhibitied with the introduction of lactose (Figure C1b). The significance of this reactivity was demonstrated when the labeled conjugates were analyzed by SDS-PAGE using 5% acrylamide gels. It was shown in two different $^{125}$I-labeled ricin-anti-Ly-2.1 preparations, one labeled using Chloramine-T and the other using an iodobead, there is free antibody (or heavy chain dimer) and unconjugated ricin present. Interestingly, both labeling procedures resulted in some breakdown of the immunotoxin. Thus the free ricin in the preparation could account for the nonspecific uptake observed.

To ensure that the binding of the immunotoxin to target cells was specific, after labeling had occurred at the antibody binding site, studies ware performed to inhibit the binding of conjugate by free MoAb. As shown in Figure C3, the binding of $^{125}$I-labeled ricin-anti-Ly-2.1 could be reduced by 50% upon the addition of 60 mug/m of anti-Ly-2.1, whereas a noreactive isotype control MoAb Ly-1.1 did not inhibit the binding even at MoAb concentrations as high as 200 mug/ml. Clearly indicating that the binding of the immunotoxin is directly related t its antibody-binding ability.

Plasma clearance of ricin, modified ricin, and ricin-antibody conjugates in mice Plasma concentrations of $^{125}$I-labeled ricin, modified ricin and ricin-antibody conjugates following intravenous injection in mice were determined at various times. Results were expressed as a percentage of the initial dose injected and plotted against time. As shown in Figure C4, native ricin displayed an extremely short plasma "survival" time. Within 30 minutes, more than 90% of the initial dose was removed. Contrary to what was observed with native ricin, modified ricin treated with metaperiodate and cyanoborohydrde and ricin-antibody conjugates remained in circulation for a much longer period of time. After an initial decline, 30 to 40% of the initial dose was present in the blood 30 minutes after injection and up to 20% of the initial injected dose was in circulation 4 hours after injection (Figure C4).

The same plasma samples taken at the various time intervals were further analyzed by SDS-PAGE gel electrophoresis using 5% and 12.5% acrylamide gels. Results were that ricin was cleared quickly from the plasma, and there was only trace amounts of the toxin detected at 2 hours and there was no detectable radioactivity corresponding to ricin by 8 hours. In contrast, modified ricin was cleared much slower and could be detected intact in the serum 8 hours after injection. Ricin-antibody conjugates were also detected in the serum samples. T he serum samples showed two major bands with estimated molecular weights of 150,000 and 200,000. Two other bands could also be seen on the gels of estimated Mr 65,000 and 110,000. The molecular weights of these bands estimated by their electrophoretic mobility in the gels, were close to values calculated for unconjugated MoAb (Mr = 150,000), ricin-antibody conjugate (one MoAb linked to one ricin molecule, Mr = 210,000) and free ricin (Mr = 65,000). The fourth band detected was shown to be the heavy chain dimer of the MoAb (Mr = 110,000) which was a byproduct of the labeling reaction. There was progressive loss of the band corresponding to ricin-antibody conjugate (Mr = 210,000), however, trace amounts could still be detected in the serum at 26 hours after injection. The relative amounts of protein in the bands of the autoradiograph culd be estimated by gel scanning.

It has been often hypothesized that the disulfide bridge binding ricin to MoAb could be unstable in vivo due to thio-disulfide exchange. This could explain why plasma ricin-antibody conjugates disappeared quickly. If such a process was occurring, deconjugated MoAb would be generated progressively in the circulation. Since MoAb molecules remain in the circulation a very long time, their presence should be easily measurable. SDS-gel electrophoresis revealed unconjugated MoAb and ricin-antibody conjugate at 30 minutes after injection, and progressive loss of the immunotoxin was observed. Contrary to what was predicted, the experimental results showed that plasma immunotoxin elimination was not associated with a increase of free MoAb, suggesting that it is very likely that splitting of the disulfide linkage does not occur in the blood.

In vivo efficacy of the Ricin-antibody conjugates The unique specificity of the ricin-antibody conjugate, without the need to have lactose present, led to the possibility of using the conjugate in vivo. All experiments were performed with the E3 variant of the murine thymoma ITT(1)75NS which in vivo is a rapidly proliferating tumor with a doubling time of less than 24 hours. Conjugates were tested in two types

of tumor model.

Survival Study In the first model B6CF1 mice were injected intraperitaneally with 1-2 x $10^5$ E3 cells and 6 - 24 hours later given one of the following treatments intraperitonealy: (a) PBS; (b) ricin-anti-Ly-2.1 (reactive conjugate); (c) ricin-anti-Ly-1.1 (nonreactive conjugate); or (d) a mixture of ricin and antibody. All treatments contained equivalent amounts of ricin and antibody which were 0.05 mug and 0.22 mug respectively. Three independent studies were performed, the difference being the initial tumor cell number inoculated, and the total amount of treatment received. The survival of these mice were monitored.

Experiment 1: Groups of 10 female B6CF1 mice (5 to 6 weeks old) were given intraperitoneal injections of 1 x $10^5$ tumor cells and 24 hours later given one of the treatments described above. Each reagent was administered intraperitoneally in 100 mul PBS. As shown in Figure C6a, mice that received a mixture of ricin and antibody died due to ricin toxicity by day 52 where 50% of the group had died by day 28, while groups receiving the nonreactive conjugate died by day 55 due to tumor similar to the PBS controls. The mice receiving the specific conjugate had a 90% survival and remained tumor free for greater than 200 days.

Experiment 2: Groups of 10 female B6CF1 mice were inoculated with 2 x $10^5$ tumor cells and 6 hours later give one of the following treatments: (a) PBS; (b) anti-Ly-2.1; (c) ricin-anti-Ly-2.1; (d) ricin-anti-ly-1.l; or (e) native ricin. The PBS treated mice had a survival time of 28 days, whereas the group that received either ricin-anti-Ly-1.1, ricin or MoAb alone died within 36 days (Figure C6b). By contrast, 50% of the mice treated with ricin-anti-Ly-2.1 survived tumor free for more than 200 days.

Experiment 3: Groups of 20 to 40 B6CF1 mice were inoculated with 2 x $10^5$ tumor cells and 6 hours later the mice received one of the treatments mentioned above. Each reagent was administered intraperitoneally in 100 mul of PBS on days 0, 1 and 2 after tumor inoculation. The total amount or ricin and antibody given was 0.15 mug and 0.66 mug respectively. Figure C8c shows a highly significant antitumor effect of the ricin-anti-Ly-2.1 treated mice, and 25 out of the 40 mice survived on day 70, whereas ricin-anti-Ly-1.1 and PBS control mice all died by day 38. Mice treated with unconjugated ricin were all dead by day 25 whereas mice treated with MoAb alone had some therapeutic effect and mice were all dead by 52 days.

Tumor Growth Study In the second model conjugates were tested for their ability to inhibit the growth of subcutaneous tumors. Mice injected subcutaneously with 4 x $10^6$ cells and on day 8 when tumors were 0.6 cm in diameter, groups of 10 female B6CF1 mice ware treated intravenously as above. A further treatment was given on day 10, the total dose being 0.2 mug and 0.9 mug for ricin and antibody respectively. On measuring the mean tumor size, on day 16, the specific anti-Ly-2.1 conjugate treated mice had tumor only 30% the size of the control group tumors (Figure C7). The other groups had a similar tumor size to the control. These mice were sacrificed and tissues were sectioned and stained for histological examination. Mice in both intravenous and conjugate treated groups showed extensive damage to the liver, spleen and kidney, demonstrating their nonspecific uptake by the reticuloendothelial system in these organs.

In vivo effect of modified ricin-antibody conjugates With the aim of specifically preventing loss of conjugate to liver and other organs, ricin was treated with a sodium emtaperiodate and sodium cyanobarohydride mixture in order to chemically cleave the carbohydrate residues responsible for hepatic uptake. The in vivo efficacy of the modified ricin conjugates was performed using the tumor growth model. Groups of 10 B6CF1 mice injected subcutaneously with 1 - 3 x $10^6$ tumor cells developed a solid tumor 0.5 $cm^2$ in size 5 days after inoculation. Mice with established tumors received an intravenous injection in 100 mul of PBS of: (a) PBS; (b) modified ricin ($IO_4^-$/$CNBH_3^-$ treated); (c) anti-Ly-2.1; or (d) modified ricin-anti-Ly-2.1 conjugate. Two independent studies were performed the major difference being the initial size of the tumors treated and the doses given.

Experiment 1: In this study mice were treated on days 5 and 8 with one of the treatments described above. The total dose of modified ricin and MoAb received per mouse was 0.2 mug and 0.90 mug respectively. The tumor growth was inhibited in mice that received the modified-ricin-antibody conjugate in their treatment compared to the tumor growth seen in mice that had been given PBS, modified ricin or antibody alone. As shown in Figure C8a, by day 13 the mean tumor size was only 70 to 75% that of the control mice, the conjugate group having significantly smaller tumors than the control groups.

Experiment 2: Mice bearing smaller thymomas (0.3 $cm^2$ in size) were treated on days 4 and 7 with one of the treatments described previously. The total amount of modified ricin and antibody given was increased to 0.25 mug and 1.1 mug respectively. The results shown in Figure C8b established significant ihibition of tumour proliferation in the group treted with modified ricin-anti-Ly-2.1 and by day 17 the mean tumor size was approximately 60% that of the controls.

Thus the modified ricin-antibody conjugate had some effect on tumor growth in the group with solid tumors.

The mice were sacrificed and tissues were sectioned and stained for histological examination. In sharp contrast to mice treated with native ricin, mice treated with the modified ricin had only minor histological changes observed in the organs. In particular the liver was normal and there was little or no damage to the spleen. Interestingly, however, the mice treated with the modified ricin-conjugate showed extensive damage to the liver and spleen.

Comparison of antitumor effects of Ricin and modified ricin-antibody conjugates The antitumor effects of 3 different anti-Ly-2.1 immunotoxins were compared in a tumor growth experiment. Groups of 20 B6CF1 mice bearing established tumor grafts received an intravenous injection in 100 mul of PBS of: (a) PBS; (b) anti-Ly-2.1; (c) ricin-anti-Ly-2.1; (d) ricin-anti-Ly-2.1 F(ab)2; (e) modified ricin-anti-Ly-2.1; or (f) ricin-17.1/2. Mice were treated intravenously on days 5 and 7. The total dose of ricin and antibody injected was 0.3 mug and 1.3 mug respectively.

The antitumor effects of the anti-Ly-2.1 conjugates were specific since antibody alone, ricin alone, antibody plus ricin and nonreactive ricin-17.1/2 control, did not significantly inhibit tumor growth. As shown in Figure C9 ricin-anti-Ly-2.1 F(ab')2 was a less effective antitumor agent than the two conjugates made with intact anti-Ly-2.1. The average tumor size of the animals treated with the F(ab')2 conjugate was 65 to 75% that of the untreated controls. Ricin-anti-Ly-2.1 and modified ricin-anti-Ly-2.1 were both effective antitumor agents. The protective effect given by the two anti-Ly-2.1 conjugates was similar and the mean tumor size of mice treated with ricin or modified ricin-anti-Ly-2.1 was significantly reduced to 50 to 55% the size of the untreated controls.

Thus as antitumor agents whole ricin anti-Ly-2.1 MoAb conjugates with either ricin or modified ricin were 20 to 30 times more effective at inhibiting tumor growth than the F(ab')2 conjugate and about 60 to 80 times more effective than the nonreactive ricin-17.1/2 control.

Toxicity to Mice Table C2 summarizes the results of the biological tests on ricin, modified ricin and antibody conjugates. Each set of results is representative of those obtained in two or more experiments.

Ricin had an acute $LD_{50}$ by either the intraperitoneal or intravenous route of 1.7 mug ricin per kg body weight (mug/kg) an 0.8 mug/kg respectively. By contrast, ricin coupled to immunoglobulin was 4-fold to 5-fold less toxic to mice. The $LD_{50}$ for ricin-antibody conjugates by the intraperitoneal and intravenous route was 7.0 mug/kg and 4.5 mug/kg respectively.

The toxicity of ricin to mice was enhanced by treatment with the ($IO_4^-$/$CNBH_3^-$ mixture 2-fold by the intravenous route and the $LD_{50}$ for the modified toxin was 0.4 mug/kg. However, modified-ricin-antibody conjugates were 2-fold to 3-fold less toxic to mice compared to the ricin-antibody conjugates and the $LD_{50}$ for the modified ricin immunotoxins was 8.3 mug/kg.

Histopathology Major histological changes in the organs of mice poisoned with native ricin and ricin-antibody conjugates were seen in the liver and the lymphoid tissue. They were essentially the same as observed by Derenzini et al (12). The liver, spleen and kidneys of mice that had received near-lethal doses of ricin were pale in color. The liver was congested with blood. Extensive damage was seen to the cells lining the sinusoides: apart from the occasional intact sinusoidal cell, there remained only pyknotic nuclei and nuclei debris. No damage to hepatic parenchymal cells was apparent. The spleen was also heavily congested with blood. The follicles and germinal centres appeared to be intact but the red pulp was largely necrotic with non-pyknotic cells visible. All other organs appeared normal.

In sharp contrast, only minor histological changes were seen in the organs of mice poisoned with ricin that had been modified by treatment with metaperiodate and cyanoborohydride. In particular, the liver was normal and there was little or no damage evident to the spleen. However, mice injected intravenously with modified ricin-antibody conjugates showed extensive damage to both the liver and spleen.

## DISCUSSION

In this part the use of whole ricin-antibody conjugates in vivo was reported and the date presented indicated that, under certain circumstances, whole ricin-antibody conjugates can lead to complete eradication of tumors without the usual side effects associated with whole ricin. The previous use of whole ricin has been quite restricted and for obvious reasons this toxin is capable of binding to all cells. Nonetheless, a phase I study has bee conducted in patients using ricin alone, i.e. in the absence of antiody, and some effects noted (13).

The covalent linkage of ricin to MoAb yielded an immunotoxin devoid of galactose binding, that was specificaly cytotoxic to MoAb reactive cells. On the basis of the encouraging in vitro results reported in Part B, in vivo experiments were performed and lead to some promising results; however, the success depended on the route of administration of the whole ricin-antibody conjugate. Firstly, using the intraperitoneal route with tumors growing in the ascites form there was complete eradication of the tumor (Figure C6). While

these results are encouraging, ascites tumors growing in mice were not considered to be equivalent to ascites found in patients where there are frequently large deposits of solid tumor scattered throughout the peritoneum rather than just tumor cells in the fluid. Nonetheless, in pleural, pericardial or ascites fluid one would expect, on the basis of results shown herein, whole ricin-antibody conjugates to eradicate free tumor cells in suspension. Secondly, intravenous injection of whole ricin-antibody conjugate was used with some success (Figure C7). However, two problems are noted: firstly the conjugates given intravenously do have some nonspecific toxicity on liver and spleen - presumably as they era taken up by reticuloendothelial cells, possibly due to the high mannose content of the ricin. The second problem of intravenous use was that the efficacy of the treatment was reduced. This could well have been due to the clearance of the conjugate by the reticuloendothelial system, but may also have been due to the difficulty of large complexes entering tumors. To pursue this strategy the carbohydrate residues on the ricin molecule was chemically modified and the biological survival time of the modified ricin was compared to ricin and ricin-antibody conjugates.

Immunotoxin localization on the tumor is an obvious critical step in order to achieve any in vivo therapy. It is therefore necessary that the immunotoxin remains in circulation at a sufficiently high level for a long enough period of time to allow binding to the tumor cells to occur. Thus the examination of the in vivo circulation life of the toxin and immunotoxin may elucidate the reasons for the poor in vivo tumoricidal effect of the immunotoxin. In this study the biological life time of ricin and modified ricin were evaluated. $^{125}$I-labeled ricin injected into mice was found to be removed rapidly from the blood stream by the liver and spleen. The clearance of the toxin by the reticuloendothelial system is likely to be mediated through recognition of the high mannose oligosaccharides of the toxin (7). Destruction of the carbohydrate portion of ricin using a mixture of sodium metaperiodate and sodium cyanoborohydride reduced the clearance by the reticuloendothelial system. The modified toxin caused none of the histopathological changes caused by the native toxin to the liver and spleen. Carbohydrate modification reduced the rate at which the toxin is cleared from the bloodstream so allowing it to persist for longer periods and at higher concentrations (Figure C4), therefore it was argued that immunotoxins prepared from ricin that have been modified in this way should have a better chance of gaining access to and killing their target cells.

Part B established the specificity and efficacy of the modified ricin-antibody conjugates in vitro, and this study evaluated the in vivo activity of the modified ricin-antibody conjugates. In tumor growth experiments, therapy did not commence until tumors were well established (0.5 $cm^2$ in size). Of the treatments administered, the modified ricin-anti-Ly-2.1 conjugate was the most effective tumor inhibitor (Figure C8). The Ly-2.1 MoAb alone and the modified ricin alone displayed no significant antitumor activity towards the murine thymoma E3.

The ricin and modified ricin immunotoxins prepared with intact Ly-2.1 MoAb were significantly better as antitumor agents in vivo than an immunotoxin made with the F(ab')2 fragment of the Ly-2.1 MoAb (Figure C9). This difference could be because the Fc portion of the intact MoAb contributes to the effective blockage of the glactose-binding sites on the B-chain of the toxin. The blockage of the galactose-binding sites was not uniform using the F(ab')2 fragment. As shown in Part B, most of the ricin-F(ab')2 conjugate molecules that did not bind the Lactose-Sepharose column were retarded in their motion down the column. Also the F(ab')2 immunotoxin that passed through the Lactose-Sepharose column was not entirely free from nonspecific toxicity. Another possible explanation for the weaker antitumor activity of the F(ab')2 immunotoxin is that it is more rapidly cleared from the mice.

Interestingly this study showed that immunotoxins of ricin and modified ricin-anti-Ly-2.1 had an equal antitumor effect on the E3 thymoma in vivo, and tumor growth was inhibited by 50-55% (Figure C9). However, it should be noted that although the modified ricin had a 2-fold increased plasma circulation life, the covalent attachment of the native ricin to MoAb also increased the plasma survival time of the ricin molecule (Figure C4). furthermore, one must take into account that the E3 tumor divides extremeley rapdily and perhaps a significant difference in antitumor activity between the two preparations could be observed with a slower growing tumor.

An unexpected finding was that the modified ricin-antibody conjugate caused damage to the liver of mice treated intravenously. The toxicity could be due to binding to cells that have receptors for the Fc portion of the immunoglobulin, however, liver and spleen damage was also inflicted by the F(ab')2 immunotoxin.

From the data it is apparent that although there was no significant MoAb released after immunotoxin administration, i.e. the disulfide linkage connecting MoAb and the toxin was stable in the blood as demonstrated by the absence of increasing amounts of unconjugated MoAb in the circulation following immunotoxin injection. The contamination of the immunotoxin preparation with free MoAb, even at a low level became a dominant feature in vivo after only a few hours due to the difference in elimination rates between immunotoxin and MoAb. For example only trace amounts of the immunotoxin were observed in the

serum of mice injected intravenously with $^{125}$I-labeled ricin-anti-Ly-2.1, 26 hours after injection whereas unconjugated MoAb could be readily detected, up to 90 hours after injection. Accordingly free MoAb could act as a potential inhibitor of the binding of residual immunotoxin to the target cells in vivo. Recently Bourrie et al (10) reported that yeast mannan could enhance the level of a ricin-A-chain immunotoxin in circulation by inhibition of liver uptake and thus increased the anticancer efficacy of immunotoxin in vivo. It is likely that such blocking agents can also be used with ricin-antibody conjugates.

REFERENCES PART C


8. SMYTH MJ, PIETERSZ GA, CLASSON B, et al. Specific targeting of chlorambucil tumors with the use of monoclonal antibodies. JNCI 1985; 76:503-510.

9. FOLEY GE, LAZARUS H, FARBER S, et al. Continuous culture of human lymphoblasts from peripheral blood of a child with acute leukemia. Cancer 1965; 18:522-529.

10. BOURRIE B, CASELLAS P, BLYTHMAN H, et al. Study of the plasma clearance of antibody-ricin-A-chain immunotoxins. Evidence for specific recognition sites on the A chain that mediate rapid clearance of the immunotoxin. Eur J. Biochem 1986; 155:1-10.

11. WEIL C. Tables for convenient calculation of median-effective dose (LD50) or ED50) and instruction in their use. Biometrics 1952; 8:249-254.

12. KNAW B, COONEY J, EDINGTON T, et al. Differences in experimental tumor localization of dual-labeled monoclonal antibody. J Nucl Med 1986: 27:1293-1299.

13. FODSTAD O, KVALHEIN G. GODAL A, et al. Phase I study of the plant protein ricin. Cancer Res 1984; 44:862-865.

14. ANDERSON M, MEISTER A. Dynamic state of glutathione in blood plasma. J Biol Chem 1980; 255:9530-9533.

Table C1

| Biodistribution of $^{125}$I-labeled ricin and modified ricin 30 minutes after intravenous injection into $B6CF_1$ mice.[1] | | | | |
|---|---|---|---|---|
| Tissue | % Initial dose per organ | | % Initial dose per gram tissue | |
| | Ricin | Modified Ricin | Ricin | Modified Ricin |
| Blood | 9.0 | 21.5 | 4.3 | 10.3 |
| Liver | 35.7 | 8.1 | 23.1 | 4.9 |
| Spleen | 3.4 | 3.1 | 24.4 | 25.9 |
| Kidney | 2.9 | 4.7 | 7.2 | 10.2 |
| Heart | 0.7 | 0.8 | 4.5 | 4.6 |

[1] Mice received an injection of 0.12 μg toxin (4.6-5.8x$10^6$ cpm/μg)

Table C2

Biological activity of native ricin, modified ricin, ricin-antibody and modified ricin-antibody conjugates. The results show the dose of ricin in μg/kg body weight calculated to kill 50% of the mice.

| Treatment | Toxicity to Mice $LD_{50}$ | |
|---|---|---|
| | i.p. | i.v. |
| Ricin | 1.7 | 0.8 |
| Ricin-MoAb | 7.0 | 4.5 |
| Modified Ricin | - | 0.4 |
| Modified Ricin-MoAb | - | 8.3 |

blood activity was calculated on the assumption that the overall blood volume represent 7% of total body weight. Mice were sacrificed, tumor removed, weighed and the radoactivity measured and calculated as a percent of the initial injected dose.

Tumor Treatment with Ricin-antibody Conjugates

In tumor growth studies, mice bearing solid tumors (0.2 to 0.6 $cm^2$ in size) were treated by IT injection and the size of the tumors measured daily along the perpendicular axes of the tumors; the data were recorded as mean tumor size (the product of two diameters ± standard error of the mean). Experimental groups of up to 40 mice all of the same sex and age were used in each experiment and mice were observed for up to 200 days.

In vitro Binding Assay

A binding assay previously described in Part B was used to determine the reactivity of $^{125}$I-anti-Ly-2.1 to E3 tumor cell lines re-establihsed in culture after IT treatment with ricin- anti-Ly-2.1.

Histology

Sections from the tumors, spleens, livers and kidneys were fixed in 10% formalin and stained with haematoxylin and eosin for examination under light microscopy.

RESULTS

Clearance of $^{125}$I- labeled MoAb and Ricin from Tumor

B6CF1 mice bearing E3 (Ly-1$^{-}$, 2$^{+}$) thymoma grafts received an IT injection of $^{125}$I-labeled anti-Ly-2.1, anti-Ly-1.1, or ricin and the clearance of iodinated material was determined from the relative amount of radiolabel in the tumor and in the blood. The clearance from tumors of two MoAb and ricin in B6CF1 mice was examined (Figure D1), 3 groups of 30 mice injected; 6 mice from each group were sacrificed at different time intervals (1,8,21,29,51 hours) and the distribution of the injected radioactivity measured. As shown in Figure D1a, 45-53% of the initial injected MoAb was retained by the tumor 1 hour after injection. The reactive (anti-Ly-2.1$^{+}$) MoAb was retained by the tumor at a higher concentration for a longer time, and 53% of the initial

Part D

This Part evaluated the use of whole ricin-antibody conjugates as therapeutic agents for cancer. With the use of IT injection, therapeutic doses of whole ricin could be localized to tumors. The ability to successfully use whole ricin was due to the loss of the galactose binding site on coupling of ricin to antibody to produce conjugates with little nonspecific binding (described previously in Parts B and C).

MATERIALS AND METHODS

Mice

Athymic, BALB/c mice (nu/nu) were obtained from the Royal Dental Hospital (Melbourne) and were maintained in specific pathogen free conditions. The inbred strain (C57BL/6xBALB/c(F1 (B6CF1) mice were maintained in the animal colony, Department of Pathology, The University of Melbourne.

Tumor cells

The human T-cell leukaemia CEM (14) and the colon carcinoma HT-29 (15) were maintained in culture. CEM cells (2x10p$^{7}$) or HT-29 cells (5x10$^{6}$) were injected subcutaneously into the abdominal wall of nude mice. The murine thymoma E3 (16) was maintained by serial passage of ascites fluid in B6CF1 mice. For in vivo experiments, E3 cells (2- 3x10$^{6}$) were injected subcutaneously into B6CF1 mice.

Preparation of Ricin-antibody Conjugates

Ricin (Sigma Chemical Company, St. Louis, MD, USA) was conjugated to MoAb by a disulfide bridge using the heterobifunctional crosslinkers SPDP an SAMSA (Part B).

Clearance of $^{125}$I-labeled MoAb and ricin Injected into Tumors

Ricin (30 mug, 1 mg/ml) in PBS containing 100 mM lactose and MoAb (30 mug, 1mg/ml) were labeled with $^{125}$I using chloramine T (17) , to a specific activity of 2-3x10$^{6}$ cpm/ mug and 4.6x10$^{6}$ cpm/ mug respectively. Groups of 6 B6CF1 mice all bearing established E3 thymomas (0.6-0.8 cm$^{2}$ in size) received IT injections of MoAb (2.0 mug) or ricin (0.5 mug); blood samples were taken at different times, and the injected dose was found in the tumor 8 hours after injection; 31% of the initial dose remained 21 hours after injection. By comparison the clearance of the nonreactive MoAb was faster and only 15% of the initial dose

was found in the tumor 8 hours after injection and only 6% of the initial dose remained 21 hours after injection. The decrease in tumor radioactivity observed for the nonreactive MoAb coincided with a proportional increase in blood activity and 21 hours after injection 49% of the initial injected activity was found in the blood. by contrast the blood activity observed for the reactive MoAb was less than 4% at all time intervals measured.

Whole ricin alone, by virtue of its galactose binding property was cleared more slowly from the tumor than the nonreactive MoAb but faster than the reactive MoAb. As shown in Figure D1b, 29% of the initial injected dose was retained by the tumor 1 hour after injection. The amount retained by the tumor decreased with time and only 10% of the initial dose remained in the tumor 21 hours after injection and only 4% of the initial dose remained 51 hours after injection. Blood activity increased with time and 19% of the initial injected dose was found in the blood 21 hours after injection.

Intratumor Treatment of the Murine Thymoma with a Ricin-antibody Conjugate

To determine whether intact ricin-antibody conjugates could be used for IT treatment, palpable tumors of 0.3-0.4cm$^2$ in size were treated. In this study groups of at least 10 mice received an IT injection of one of the following treatments in 100 mul of PBS: (i) PBS; (ii) anti-Ly-2.1; (iii) ricin-anti-Ly-2.1; (iv) ricin-anti-Ly-1.1.

In a representative experiment mice bearing tumors received two injections directly into the tumor on days 7 and 9 with preparations containing 0.2 mug ricin and 0.9 mug antibody. As shown in Figure D2a, the ricin-anti-Ly-2.1 treated tumors regressed and within 48 hours all tumors had virtually disappeared, while untreated E3 tumors grew to a large size (4.5-5.0 cm$^2$). The antitumor effect of the ricin-anti-Ly-2.1 conjugate was specific since there was no significant inhibition of tumor growth in the groups which received MoAb alone or ricin-anti-Ly-1.1 (Figure D2a), which did not bind E3 cells (the anti-Ly-1.1 conjugate treatment gave some inhibition of tumor growth but the tumors grew on relentlessly and the mice dies). Figure D2b shows the individual growth curves of the mice treated with ricin-anti-Ly-2.1 and complete regressions were observed in 4/10 mice which remained tumor free for greater than 200 days. In 6/10 mice the tumors regressed for 2-4 days and then grew again, usually at the original site. When tracing the individual tumor growth curves of the mice treated with ricin-anti-Ly-1.1, no complete regressions were obtained (Figure D2c). Autopsy of the animals in the ricin-anti-Ly-2.1 treated group and histological examination of organs (liver, kidneys, spleen) did not reveal any tissue damage. Animals in the group treated with the nonreactive conjugate showed damage to the liver, spleen and kidneys, presumably as these conjugates were not retained by the tumor.

Morphology and Histology of Treated and Untreated E3 Tumors

Regression was characterized morphologically and histologically in mice bearing E3 tumors which were treated IT with the ricin-antibody conjugates. Mice were examined 17 days following treatment and a comparison of the size of a ricin-anti-Ly-2.1 treated tumor to a tumor treated with antibody alone was made. The ricin-anti-Ly-2.1 treated tumors shrank to a dry black scar that eventually separated leaving a clean shallow ulcer at the injection site, whereas tumors treated with MoAb alone grew to 5.0cm$^2$ in size. Several (2/4) cured mice were sacrificied 30 days after treatment and were autopised to ensure that there was no tumor regrowth and showed no pathological evidence of tumor. Of 2/6 mice treated with ricin-anti-Ly-2.1 where the tumors recurred, one should note that the tumor reappeared around the initial injection site. The treated and untreated tumors were analyzed histologically. Untreated E3 tumors were well vascularized and were populated with viable cells, and also showed numerous mitoses. By contrast, the ricin-anti-Ly-2.1 treated tumors contained large areas of necrotic tissue showing much cellular debris with only small patches of viabl cells. Adjacent normal connective tissue and vascularized fat tissue were damaged. Some blood vessels contained thrombi. Together these observations suggested destruction of the E3 tumor cells by ricin-anti-Ly-2.1 conjugate and some minor damage in the immediate vicinity.

Treatment of Tumors with Modified ($IO_4^-$/$CNBH_3^-$ treated) Ricin-antibody conjugates

The specificity and potency of a "modified" ($IO_4^-$/$CNBH_3^-$ treated), ricin-anti-Ly-2.1 conjugate was undertaken in a tumor growth study to evaluate the antitumor effects when injected directly into established tumors. Groups of 10 B6CF1 mice bearing established E3 tumor 0.6 cm$^2$ in size received an IT injection in 100 mul of PBS either: (i) PBS; (ii) anti-Ly-2.1; (iii) modified ricin; (iv) modified ricin-anti-Ly-2.1. In this study the mice received a single IT injection of 0.88 mug of modified ricin and 4.0 g MoAb 5 days after tumor

innoculation. As shown in Figure D5, there was significant inhibition of tumor growth in mice treated with conjugate and all of the tumors regressed within 2 days after treatment but then regrew in 7/10 mice 11 days after the initial treatment. These recurring tumors were established in tissue culture and a binding assay showed the tumor calls to have the same distribution of Ly-2.1 antigen as the parent E3 cell line. The binding curve obtained when $^{125}$I-anti-Ly-2.1 was incubated with the cells clearly showed that there was no noticeable difference in the uptake of radioactivity by the 2 cell lines. Thus the tumor cells that escaped being killed in the conjugate treated mice ware not a mutant subclone deficient in Ly-2.1 antigen. Furthermore, the serum of the treated mice was analyzed for an anti-ricin response using a radioimmunoassay, but no ricin antibodies could be detected in the serum (not shown). Therefore these recurring tumors could then be retreated with a course of modified ricin-anti-Ly-2.1.

As a control, E3 tumors were treated either with antibody alone or with modified ricin. Antibody when injected directly into the tumor had no effect on the development of the tumors (Figure D5), whereas mice treated with modified ricin (>0.3 mug) displayed symptoms of poisoning (weight loss, hemorrhage, respiratory difficulties, loss of balance) followed by death 2 days later. Lower doses of modified ricin (<0.2 g) did not kill the mice, nor did they cause tumors to regress. Thus a dose of modified ricin (not attached to antibody) which was safe to mice and destructive to tumors could not be found.

Comparison of the antitumor effects of Ricin and Modified ($IO_4^-$/$CNBH_3^-$) Ricin

The antitumor effects of the two different anti-Ly-2.1 conjugates were compared on established tumor. B6CF1 mice bearing thymoma grafts 0.6 cm in diameter were treated with a single IT injection 5 days after tumor innoculation. All treatments contained equivalent amount of ricin and antibody which were 1.0 mug and 4.6 mug respectively. The results obtained are summarized in Table D1.

Both anti-Ly-2.1 conjugates were able to cause regression of solid tumors. However the modified ricin-anti-Ly-2.1 conjugate was a less effective antitumor agent than the native ricin conjugate: modified ricin-anti-Ly-2.1 treatment cured 13/23 mice, which remained tumor free for greater than 200 days. Solid tumors recurred in 7/23 mice, which died due to tumor and 3/23 mice died due to ricin toxicity. By contrast 24/30 mice treated with the native ricin conjugate survived tumor free (>200 days). One of the mice developed a subcutaneous tumor at the site of injection whereas 6/30 mice developed ascites tumors in the peritoneium. The mean survival time of these mice was extended to 40 days compared to the 20 days mean survival time of the PBS treated controls (i.e. ricin-anti-Ly-2.1 treatment extended the survival time of the PBS treated controls (i.e. ricin-anti-Ly-2.1 treatment extended the survival time of tumor bearing mice).

The antitumor effect of the two anti-Ly-2.1 conjugates was specific since antibody alone, ricin alone, and modified ricin alone were all without protective effect, and mice either died due to ricin toxicity or due to tumor.

The conjugate treated mice that showed no signs of tumor growth after 200 days were either given an intraperitoneal or subcutaneous injection of $3x10^6$ E3 cells. The mice all developed tumors and died over the same period (18-20 days) as an age matched group of control animals. The conjugate treated mice had therefore not developed detectable resistance to the tumor cells.

Treatment of Human Tumor Xenografts in Nude Mice

To study the potential use of immunotoxins for therapeutic treatment of human tumors an experimental model of human neoplasia was used where intact ricin-antibody conjugates were used for IT treatment of human tumor xenografts in nude mice. Three studies were performed.

Experiment 1: Groups of 4 nude mice with palpable CEM tumors 0.2 $cm^2$ in size received a single IT injection in 100 mul PBS of either: (i) PBS; (ii) anti-TFR; (iii) ricin-anti-TFR. Mice were treated 7 days after tumor inoculation with one of the preparations containg 1 mug ricin and 4.6 mug antibody. As shown in Figure D7 all CEM tumors treated with ricin-anti-TFR regressed within 2 days following treatment and there was no relapse even after 120 days. Injection of anti-TFR alone had no effect on CEM tumor growth.

Experiment 2: Nude mice bearing human colon carcinoma cell line (HT-29) xenografts were used. Three groups of 5 mice were treated as follows: (i) The first group received a reactive ricin-17.1/2 conjugate; (ii) the second group a nonreactive ricin-anti-HLA conjugate, our HT-29 cells are nonreactive with our anti-HLA; (iii) the third, the control group, received PBS alone. Each reagent was administered directly into established tumor 0.3 $cm^2$ in size, 10 days after tumor inoculation. The results clearly demonstrate complete tumor regression in the group that received the ricin-17.1/2, and this was apparent after only 2 days (Figure D8). To determine whether regression of HT-29 tumors was due to the specific binding of

the immunotoxin through the antibody moiety, HT-29 tumors were also treated with an irrelevant nonreactive control immunotoxin, ricin-anti-HLA which did not bind HT-29 cells. There was no significant inhibition of tumor growth (Figure D8). Thus the specificity of ricin-antibody conjugate IT therapy is proven. In a comparison of a ricin-17.1/2 treated tumor at 120 days after IT injection to an untreated mouse bearing a HT-29 tumor 40 days after tumor inoculation it was noted that only a fibrotic scar remained at the previous location of the ricin-17.1/2 treated tumor indicated by the arrow, whereas the untreated tumor measured 1.4 $cm^2$ in size.

Experiment 3: To determine whether intact ricin-antibody conjugates could be effective on large tumors, mice bearing HT-29 tumors 1.5-2.0 $cm^2$ in size (1.2-1.4 cm in diameter) were treated with one of two conjugates: (i) reactive ricin-17.1/2; (ii) nonreactive ricin-anti-HLA. Mice received an intratumoral injection containing 1 mug ricin in 100 mul PBS 40 days after tumor inoculation and this was repeated 3 days later, as indicated by the arrows in Figure 9. All three HT-29 tumors treated with ricin-17.1/2 regressed, and there was no relapse. When ricin-anti-HLA was administered HT-29 tumors did not regress.

DISCUSSION

Selected MoAb should be suitable vehicles for carrying toxins such as ricin to tumor cells because of their specificity (by motive of their monoclonality) and ability to be produced in virtually unlimited amounts. Part B demonstrated that such conjugates are indeed powerful reagents in in vitro systems but obviously, the most attractive use of the conjugates are as a therapeutic agent for cancer in patients. However the in vivo application of these conjugates was not as dramatic as expected, and a number of limitations were noted such as toxicity towards nontarget tissue, instability, and rapid plasma clearance.

The localization of the conjugate to the tumor is an obvious critical step to achieve in any in vivo therapy and the relatively short circulation life of whole ricin-antibody conjugate demonstrated in Part C and also reported by a number of other laboratories (18-20) may explain the reasons for the failure of intravenously injected ricin-antibody conjugates to show greater antitumor activity to solid tumors. To improve the action of these conjugates on tumors the amount of conjugate localized to a tumor was increased by the direct injection of the conjugate into established tumors. The studies in this part showed that a therapeutic dose of whole ricin-antibody conjugate could be localized to solid tumors and the cytotoxic efficacy of the conjugate could be dramatically improved when the treatment was administered directly into the tumor. There were three unique features of this study that will be separately discussed. The first is the mode of conjugate administration where conjugates were injected directly into and around solid tumors in mice. Secondly studies showed that MoAb could be specifically localized to tumor following regional injection; and finally therapeutic experiments were carried out which demonstrated that regionally injected ricin-antibody conjugate was a potent antitumor agent which was effective in causing the regression of established tumors.

The direct injection of whole ricin-antibody conjugate into tumors gave some remarkable results. Tumors disappeared rapidly although a proportion reappeared (Figures D2 and D5). Modification of the injection protocol, which included injecting around the edge of the tumor and using multiple treatments led to a high proportion of tumors being completely eradicated ( Table D1). The most important finding in these studies was that established E3 thymomas ($Ly\text{-}1^-$, $2^+$) treated with a reactive ricin-anti-Ly-2.1 conjugate regressed, while similar sized tumors treated with a nonreactive ricin-anti-Ly-1.1 conjugate did not and thus selectivity was observed without lactose being present.

Similar results were observed in the nude mouse model. Established CEM (Figure D7) and HT-29 (Figure D8) tumors 0.2-0.4 $cm^2$ in size treated with ricin-anti-TFR and ricin-17.1/2 respectively regressed. HT-29 tumors 1.5-2.0 $cm^2$ in size were given two injections of ricin-17.1/2 conjugate three days apart, and treatment was administered in this manner to saturate the tumor with immunotoxin and prevent recurrence due to tumor cells which may have escaped the first treatment. Under these conditions, HT-29 tumors treated with ricin-17.1/2 conjugate regressed wherease tumor treated with a nonreactive conjugate did not (Figure D9).

In all studies, the regression of tumors was marked by a rapid decrease in the mass of tumor which remained a flat, fibrotic scar. Light microscopy showed that this scar resulted from organization of the necrotic tissue. Furthermore examination of spleen and liver did not reveal any evidence of damage.

As a control, ricin injected alone in doses equivalent to conjugate dose were lethal to mice two days after treatment (Figure D5). Death was preceded by weight loss, skin hemorrages, respiratory and nervous system disfunctions. The spleen and liver of these mice were damaged, findings in agreement with earlier light microscopic descriptions of damage caused due to ricin poisoning. A therapeutic effective dose of whole ricin alone could not be found and a dose of 0.20 mug or lower of ricin did not kill tumor bearing

mice but also did not cause tumor regression. Thus in contrast to reactive ricin-antibody conjugates, nonreactive conjugate and ricin alone were not effective antitumor reagents.

This study has shown that the antibody not only served to target ricin to the tumor but antibody holds the ricin in the tumor and very little escapes. For example, 53% of the initial injected dose of a reactive MoAb could be localized to established tumor 8 hours after injection (Figure D1a). Thus the high affinity of the MoAb contributed to the localization of the reactive ricin-antibody conjugate to the tumour. By contrast conjugates using antibody which did not bind or ricin alone which has a lower binding coefficient are able to leak out of the tumor (Figure D1) and cause some nonspecific toxicity to the reticuloendothelial system.

In conclusion, this form of therapy may have some practical value for treating superficial tumors and recurrences of tumors in sites which are difficult to remove surgically yet are amenable to injection. (Surgical colleagues have suggested fixed regional lymphadenopathy causing pressure symptoms, hepatic metastases causing pain or obstructive jaundice and locally recurrent breast cancer or melanoma nodules. It should be noted that the clinical management of locally recurrent tumors is a problem which is currently treated by radiation where damage to surrounding normal tissue occurs concomitantly. It is clearly important to fully explore the development of whole ricin-antibody conjugate for intratumor treatment which should be effective against only the tumor tissue.

REFERENCES PART D

14. Foley, G.E., Lazarus, H., Farber, S. et al. Continous culture of human lymphocytes from peripheral blood of a child with acute leukaemia. Cancer 18:522-529 (1965).

15. Fogh, J. and Trempe, G. New human tumor cell lines. In: Fogh, J., ed. Human tumour cells in vitro. New York : Plenum Press, pp 115-120 (1975).

16. Smyth, M.J., Pietersz, G.A., Classon, B.J. et al. Specific targeting of chlorambucil to tumor with the use of monoclonal antibodies. Journal Natl. Cancer Inst. 76:503-510 (1986).

17. Greenwood, F.C., Hunter, W.M. and Glover, J.S. The preparation of 131I-labeled human growth hormone of high specific radioactivity. Biochem. J. 89:114-123 (1963).

18. Blythman, H.E., Casellas, P., Gros, O. et al. Immunotoxins: hybrid molecules of monoclonal antibodies and a toxin subunit specifically kill tumor cells. Nature 20:145-146 (1981).

19. Trowbridge, I.S. and Domingo, D.L. Anti-transferrin receptor monoclonal antibody and toxin antibody conjugates affect growth of human tumor cells. Nature 294:171-173 (1981).

20. Bourke, B.J.P., Casellas, P., Blythman, H.E. et al. Study of the plasma clearance of antibody-ricin-A-chain immunotoxins. Eur. J. Biochem. 155:1-10 (1986).

TABLE D1

TREATMENT OF E3 TUMOURS WITH MoAb NATIVE RICIN, MODIFIED RICIN OR CONJUGATES

| Treatment | Mice No. | Tumour | | Death | | % Cure |
|---|---|---|---|---|---|---|
| | | Solid | Ascites | Tumour | Toxicity | |
| PBS | 10 | 10 (100%) | - | 10 | 0 | 0 |
| anti-2.1-R (1:1) | 30 | 1 (3%) | - | 6 | | 80 |
| anti-2.1-R (1:2) | 20 | 10 (50%) | - | 10 | | 50 |
| anti-2.1-R ($Na1O_4$) | 23 | 7 (30%) | - | 7 | 3 (13%) | 56 |
| Ricin | 10 | | | | 10 | 0 |
| MoAb | 10 | 10 | | | | 0 |
| Native Ricin | 10 | | | | 10 | 0 |

Mice bearing E3 tumors (0.6 cm in diameter) were given injections intratumorly with preparations containing 1ug of ricin and 4.6 ug of anti-Ly-2.1 in 100 ul of PBS.

## Claims

1. A conjugate of ricin being whole ricin or a derivative of ricin, which derivative per se being inhibitory of protein synthesis in cell free systems, with an antibody which is preferentially absorbed by a tumour cell as compared to a non tumour cell and wherein the galactose binding site of said ricin is blocked and pharmacologically acceptable salts thereof.

2. A conjugate as claimed in claim 1, wherein the conjugation is via a disulphide linkage.

3. A compound of formula $Ab\text{-}Y\text{-}X_1\text{-}NHR_1$ wherein Ab and the Y group attached thereto represent an antibody radical and wherein Y is S or NH, $R_1$ and the amino group to which it is attached represents a ricin radical being a whole ricin radical or a radical of a derivative of ricin which derivative per se being inhibitory of protein synthesis in cell free systems and $X_1$, or $Y\text{-}X_1$ (when Y is S), represents a conjugating chain including a disulphide linkage and wherein the galactose binding site of said ricin radical is blocked and pharmacologically acceptable salts thereof.

4. A compound of formula I

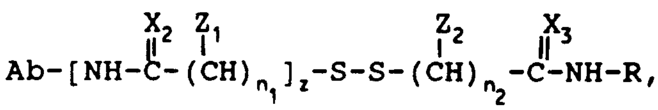

I

wherein Ab and the amino or sulphur to which it is attached represents an antibody radical, $R_1$ and the amino group to which it is attached represents a ricin radical being a whole ricin radical or a radical of a derivative of ricin which derivative per se being inhibitory of protein synthesis in cell free systems, $Z_1$ and $Z_2$ which may be the same or different are H, alkyl, aryl, carboxyl, halide, cyano, ester, carboxamide, sulphonate, hydroxy or amino, $n_1$ and $n_2$ which may be the same or different are 1-10, $X_2$ and $X_3$ which may be the same or different are NH, O or S and Z is O or 1 and wherein the galactose binding site of ricin radical is blocked and pharmacologically acceptable salts thereof.

5. A compound as claimed in claim 4, and of formula II

$Ab\text{-}S\text{-}S\text{-}R_2\text{-}NH\text{-}R_1$ II

wherein Ab-S- represents an antibody radical, $R_1\text{-}NH\text{-}$ represents a ricin radical being a whole ricin radical or a radical of a derivative of ricin which derivative per se being inhibitory of protein synthesis in cell free systems, and $\text{-}R_2\text{-}NH$ and $NH\text{-}R_3\text{-}$, which may be the same or different, are of formula IV

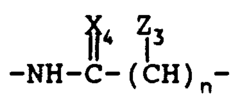

IV

wherein $X_4$ is O, S or NH, n is from 1 to 10 and $Z_3$ is hydrogen, alkyl, aryl, carboxyl, halide, cyano, ester, carboxamide, sulphonate, hydroxy or amino.

6. A compound as claimed in claim 4, and of formula III

$Ab\text{-}NH\text{-}R_3\text{-}S\text{-}S\text{-}R_2\text{-}NHR_1$ III

wherein Ab-N represents an antibody radical, $R_1\text{-}NH\text{-}$ represents a ricin radical being a whole ricin radical or a radical of a derivative of ricin which derivative per se being inhibitory of protein synthesis in cell free systems, and $\text{-}R_2\text{-}NH$ and $NH\text{-}R_3$, which may be the same or different, are of formula IV

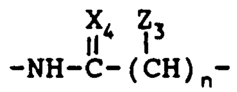

IV

wherein $X_4$ is O, S or NH, n is from 1 to 10 and $Z_3$ is hydrogen, alkyl, aryl, carboxyl, halide, cyano, ester, carboxamide, sulphonate, hydroxy or amino.

**7.** A compound as claimed in claim 4 and of formula V

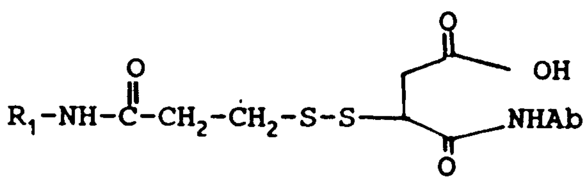

V

wherein Ab-NH- represents an antibody radical and $R_1$-NH represents a ricin radical being a whole ricin radical or a radical of a derivative of ricin which derivative per se being inhibitory of protein synthesis in cell free systems.

**8.** A compound as claimed in any one of claims 3-7 wherein said ricin radical is a whole ricin radical.

**9.** A compound as claimed in any one of claims 3-7 wherein said antibody radical is a radical of an antibody showing specificity for a selected one of breast, brain, melanoma, lung, pancreas and colon tumours.

**10.** A pharmaceutical composition comprising a compound in accordance with any preceding claim together with a pharmaceutically acceptable diluent.

**11.** The use of a conjugate as claimed in claim 1 or claim 2, or of a compound as claimed in any one of claims 3-9, in the manufacture of an injectable medicament for the treatment of cancer.

**12.** Compounds of formula VII

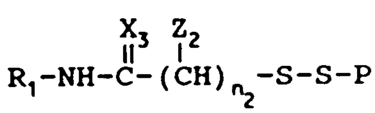

VII

wherein P is of formula VIIa, VIIb or VIIc

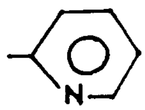

VIIa

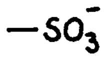

VIIb

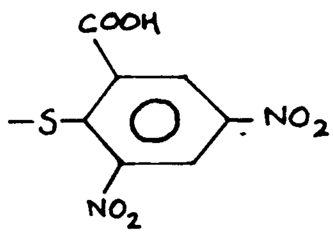

VIIc

and $R_1$, $X_3$, $Z_2$ and $n_2$ have the meaning given in claim 4.

**13.** A method of making a compound in accordance with any one of claims 1-9 comprising reacting whole ricin or a derivative or ricin which derivative per se is inhibitory of protein synthesis in cell free systems to obtain a ricin containing a -S-S- group, reacting an antibody to obtain an antibody containing a -S-S- group, reducing one of the conjugates to form a -SH group and reacting the reduced conjugate with the other of the conjugates to obtain the compound desired.

**14.** A method of making a compound in accordance with any one of claims 1-9 comprising utilizing a sulphydryl introducing compound to produce a side chain on an antibody or whole ricin or a derivative of ricin which derivative per se is inhibitory of protein synthesis in cell free systems containing a -S- linkage, reducing that linkage and then reacting to form an antibody ricin conjugate containing a -S-S- linkage.

**Patentansprüche**

**1.** Ein Verbindungsstoff des Rizins, sei es ganzes Rizin oder ein Derivat des Rizins, welches Derivat per se Eiweisssynthese in zellenfreien Systemen durch einen Antikörper behindert, der von einer Tumorzelle im Vergleich zu einer Zelle ohne Tumor bevorzugt absorbiert und worin die Galaktosebindungsstelle von dem genannten Rizin blockiert wird und pharmakologisch annehmbare Salze davon.

**2.** Ein Verbindungsstoff, wie in Anspruch 1 beansprucht, worin die Konjugation durch eine Disulfidbindung ist.

**3.** Eine Verbindung der Formel Ab-Y-$X_1$-$NHR_1$, worin Ab und die daran befestigte Y Gruppe ein Antikörperradikal darstellen und worin Y S oder NH ist, $R_1$ und die an $R_1$ befestigte Aminogruppe ein Rizinradikal als ein ganzes Rizinradikal oder ein Radikal eines Rizinderivats darstellt, welches Derivat per se Eiweisssynthese in zellenfreien Systemen behindert und $X_1$ oder Y-$X_1$ (wenn Y S ist) eine konjugierende Kette mit einer Disulfidbindung inbegriffen darstellt und worin die Galaktosebindungsstelle von dem genannten Rizinradikal blockiert wird und pharmakologisch annehmbare Salze davon.

**4.** Eine Verbindung der Formel I

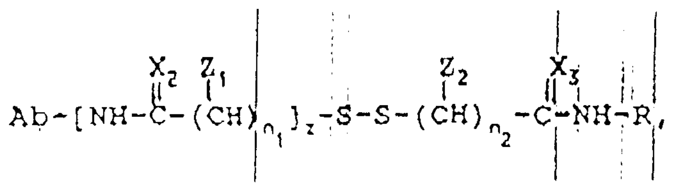

I

worin Ab und das Amino oder der Schwefel, woran Ab befestigt ist, ein Antikörperradikal $R_1$ darstellt, und die an Ab befestigte Aminogruppe ein Rizinradikal als ein ganzes Rizinradikal oder ein Radikal von einem Rizinderivat, welches Derivat per se Eiweisssynthese in zellenfreien Systemen behindert, darstellt; $Z_1$ und $Z_2$, die gleich oder verschieden sein können, sind H, Alkyl, Aryl, Karboxyl, Halogenid, Zyano, Ester, Karboxamid, Sulfonat, Hydroxy oder Amino; $n_1$ und $n_2$, die gleich oder verschieden sein können, sind 1-10; $x_2$ und $x_3$, die gleich oder verschieden sein können,sind NH, O oder S; und Z ist O oder 1 und worin die Galaktosebindungsstelle des Rizinradikals blockiert wird und pharmakologisch annehmbare Salze davon.

**5.** Eine Verbindung, wie in Anspruch 4 beansprucht und der Formel II

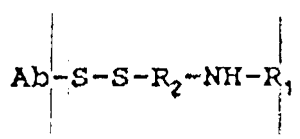

II

worin Ab-S- ein Antikörperradikal darstellt, $R_1$-NH- ein Rizinradikal als ein ganzes Rizinradikal oder ein Radikal eines Rizinderivats, welches Derivat per se Eiweisssynthese in zellenfreien Systemen behindert, darstellt und -$R_2$-NH und NH-$R_3$-, die gleich oder verschieden sein können, der Formel IV sind

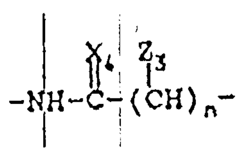

IV

worin $X_4$ O, S oder NH ist, n von 1 bis 10 ist, und $Z_3$ Wasserstoff, Alkyl, Aryl, Karboxyl, Halogenid, Zyano, Ester, Karboxamid, Sulfonat, Hydroxy oder Amino ist.

**6.** Eine Verbindung, wie in Anspruch 5 beansprucht, und der Formel III

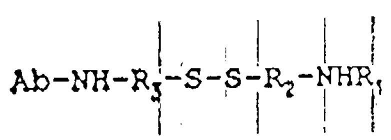

III

worin Ab-N ein Antikörperradikal als ein ganzes Rizinradikal oder ein Radikal eines Rizinderivats, welches Derivat per se Eiweisssynthese in zellenfreien Systemen behindert, darstellt und -$R_2$-NH und NH-$R_3$, die gleich oder verschieden sein können, der Formel IV sind

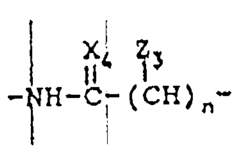

IV

worin $X_4$ O, S oder NH ist, n von 1 bis 10 ist und $Z_3$ Wasserstoff, Alkyl, Aryl, Karboxyl, Halogenid, Zyano, Ester, Karboxamid, Sulfonat, Hydroxy oder Amino ist.

**7.** Eine Verbindung, wie in Anspruch 4 beansprucht und der Formel V

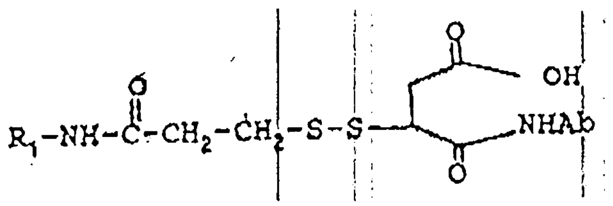

V

worin Ab-NH- ein Antikörperradikal darstellt und $R_1$-NH ein Rizinradikal als ein ganzes Rizinradikal oder ein Radikal eines Rizinderivats, welches Derivat per se Eiweisssynthese in zellenfreien Systemen behindert, darstellt.

**8.** Eine Verbindung, wie in jeder der Ansprüche von 3 bis 7 beansprucht, worin das genannte Rizinradikal ein ganzes Rizinradikal ist.

**9.** Eine Verbindung, wie in jeder der Ansprüche von 3 bis 7 beansprucht, worin das genannte Antikörperradikal ein Radikal eines Antikörpers ist, das ein spezifisches Bedürfnis für einen ausgewählten Tumor von Brust-, Gehirn-, Melanoma-, Lungen-, Pankreas- und Dickdarmtumoren zeigt.

**10.** Eine pharmazeutische Zusammensetzung, die aus einer Verbindung besteht, gemäss jeder der vorhergehenden Ansprüche zusammen mit einem pharmazeutisch akzeptablen Verdünnungsmittel.

**11.** Die Verwendung eines Verbindungsstoffes, wie in Anspruch 1 oder Anspruch 2 beansprucht, oder einer Verbindung, wie in jeder der Ansprüche von 3 bis 9 beansprucht, in der Herstellung eines einspritzbares Medikamentes für die Behandlung von Krebs.

**12.** Verbindungen der Formel VII

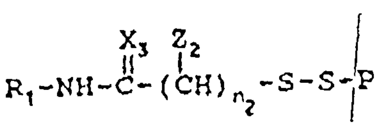

VII

worin P der Formel VIIa, VIIb oder VIIc ist.

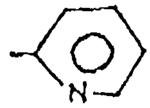

VIIa

$-SO_3^-$

VIIb

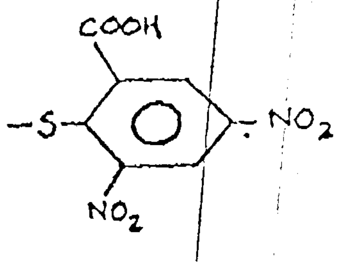

VIIc

und $R_1$, $Z_2$ und $n_2$ die Bedeutung haben, die in Anspruch 4 angegeben ist.

**13.** Verfahren, eine Verbindung gemäss jeder Ansprüche von 1 bis 9 herzustellen, bestehend aus ganzem Rizin oder einem Rizinderivat, welches Derivat per se Eiweisssynthese in zellenfreien Systemen behindert, zur Reaktion zu bringen, um ein -S-S Gruppe enthaltendes Rizin zu bekommen,einen Antikörper zur Reaktion zu bringen, um einen -S-S Gruppe enthaltenden Antikörper zu erhalten, einen des Verbindungstoffes zu reduzieren, um eine -SH Gruppe zu formen und den reduzierten Verbindungsstoff mit den anderen Verbindungsstoffen zur Reaktion zu bringen, um die gewünschte Verbindung zu erhalten.

**14.** Verfahren, eine Verbindung gemäss jeder der Ansprüche von 1 bis 9 herzustellen, bestehend aus der Verwendung einer Sulphydryl-einführenden Verbindung, um eine Seitenkette auf einem Antikörper oder ganzem Rizin oder einem Rizinderivat, welches Derivat per se Eiweisssynthese in zellenfreien Systemen, die eine -S- Bindung enthalten, behindert, herzustellen, um jene Bindung zu reduzieren und dann zur Reaktion zu bringen, um einen Antikörperrizinverbindungsstoff, der eine -S-S Bindung enthält, zu formen.

**Revendications**

**1.** Un conjugué de ricine qui est de la ricine entière ou bien un dérivé de ricine, dérivé qui est lui-même inhibiteur de la synthèse de protéine dans les systèmes sans cellules, avec un anticorps qui est absorbé de préférence par une cellule tumorale, plutôt que par une cellule non-tumorale et dans lequel l'agrégation de galactose de ladite ricine est bloquée et les sels associés qui sont acceptables du point de vue pharmacologique.

**2.** Un conjugué conforme à la revendication 1, dans lequel la liaison conjuguée se fait par une combinaison de bisulphides.

**3.** Un composé de la formule $Ab-Y-X_1-NHR_1$ dans lequel Ab et le groupe Y qui y est combiné représente un radical d'anticorps et dans lequel Y est S ou NH, $R_1$ et le groupe amino auquel il est combiné représente un radical de ricine qui est un radical de ricine entière ou bien un radical d'un dérivé de ricine qui est lui-même un dérivé inhibiteur de la synthèse de protéine dans les systèmes sans cellules et $X_1$, ou $Y-X_1$ ( quand Y est S ), représente une chaîne conjuguée qui inclut une combinaison de

bisulphides et dans lequel l'agrégation de galactose dudit radical de ricine est bloquée, et les sels associés qui sont acceptables du point de vue pharmacologique.

**4.** Un composé de la formule I

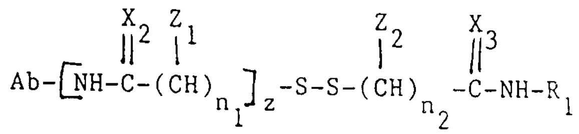

I

dans lequel Ab et l'amino ou sulfur auquel il est combiné représente un radical d'anticorps, $R_1$ et le groupe amino auquel il est combiné représente un radical de ricine qui est un radical de ricine entière ou bien un radical d'un dérivé de ricine qui est lui-même inhibiteur de la synthèse de protéine dans les systèmes sans cellules, $Z_1$ et $Z_2$ qui peuvent être les mêmes ou différents sont H, alcoyle, aryle, carboxyle, haloïde, cyano, ester, carboxamide, sulfonat, hydroxide ou amino, $n_1$ et $n_2$ qui peuvent être les mêmes ou différents sont 1-10, $X_2$ et, $X_3$ qui peuvent être les mêmes ou différents sont NH, O ou S et Z est O ou 1 et dans lequel l'agrégation de galactose du radical de ricine est bloquée et les sels associés qui sont acceptables du point de vue pharmacologique.

**5.** Un composé selon la revendication 4 et la formule II

$Ab\text{-}S\text{-}S\text{-}R_2\text{-}NH\text{-}R_1$ II

dans lequel Ab-S- représente un radical d'anticorps, $R_1$-NH- représente un radical de ricine qui est un radical de ricine entière ou un radical de dérivé de ricine, dérivé qui est lui-même inhibiteur de la synthèse de protéine dans les systèmes sans cellules, et -$R_2$-NH et NH-$R_3$-, qui peuvent être les mêmes ou différents sont de la formule IV

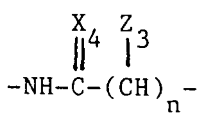

IV

dans laquelle $X_4$ est O, S ou NH, n est de 1 à 10 et $Z_3$ est hydrogène, alcoyle, aryle, carboxyle, haloïde, cyano, ester, carboxamide, sulfonat, hydroxyde ou amino.

**6.** Un composé selon la revendication 5 et la formule III

$Ab\text{-}NH\text{-}R_3\text{-}S\text{-}S\text{-}R_2\text{-}NHR_1$ III

dans laquelle Ab-N représente un radical d'anticorps, $R_1$-NH- représente un radical de ricine qui est un radical de ricine entière ou un radical de dérivé de ricine, dérivé qui est lui-même inhibiteur de la synthèse de protéine dans les systèmes sans cellules, et -$R_2$-NH et NH-$R_3$, qui peuvent être les mêmes ou différents, sont de la formule IV

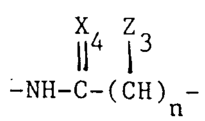

IV

dans laquelle $X_4$ est O, S ou NH, n est de 1 à 10 et $Z_3$ est hydrogène, alcoyle, aryle, carboxyle, haloïde, cyano, ester,carboxamide, sulfonat, hydroxyde ou amino.

**7.** Un composé selon la revendication 4 et la formule V

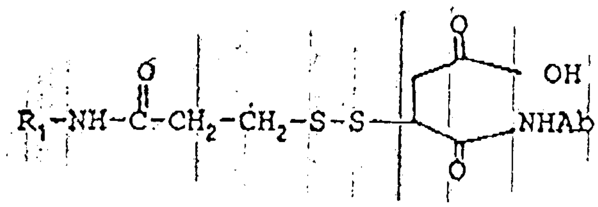

V

dans laquelle Ab-NH- représente un radical d'anticorps et $R_1$-NH représente un radical de ricine qui est un radical de ricine entière ou un radical de dérivé de ricine, dérivé qui est lui-même inhibiteur de la synthèse de protéine dans les systèmes sans cellules.

**8.** Un composé selon n'importe laquelle des revendications 3 - 7 où ledit radical de ricine est un radical de ricine entière

**9.** Un composé selon n'importe laquelle des revendications 3 - 7 où ledit radical d'anticorps est un radical d'un anticorps qui montre une spécificité pour une des tumeurs sélectionnés qui suit : au sein, au cerveau, au poumon, au pancréas, au côlon ou un mélanome.

**10.** Une composition pharmaceutique comprenant un composé conforme aux revendications précédentes avec un diluant acceptable du point de vue pharmaceutique.

**11.** L'utilisation d'un conjugué selon la revendication 1 ou la revendication 2 ou d'un composé selon n'importe laquelle des revendications 3 - 9, dans la fabrication d'un médicament injectable pour le traitement du cancer.

**12.** Composés de la formule VII

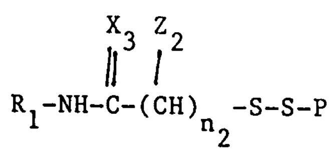

VII

dans laquelle P est de la formule VIIa, VIIb ou VIIc

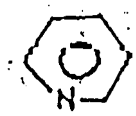

VIIa

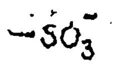

VIIb

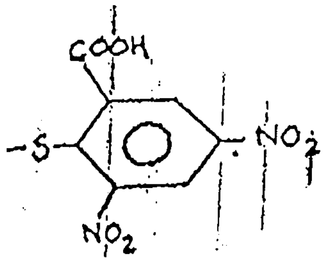

VIIc

et $R_1$, $X_3$, $Z_2$ et $n_2$ ont la signification donnée dans le paragraphe 4.

**13.** Une méthode de fabrication d'un composé selon n'importe laquelle des revendications 1 - 9,où on fait réagir la ricine entière ou un dérivé de ricine qui est lui-même un inhibiteur de la synthèse de protéine dans les systèmes sans cellules pour obtenir une ricine contenant un groupe -S-S, où on fait réagir un anticorps pour obtenir un anticorps contenant un groupe -S-S, où on fait réduire un des conjugués pour former un groupe -SH et où on fait réagir le conjugué qui a été réduit avec l'autre des conjugués pour obtenir le composé voulu.

**14.** Une méthode de fabrication d'un composé selon n'importe laquelle des revendications 1-9, où on utilise un composé qui permet l'introduction d'un sulphydryle pour produire une chaîne latérale sur un anticorps ou sur la ricine entière ou sur un dérivé de ricine qui est lui-même un inhibiteur de la synthèse de protéine dans les systèmes sans cellules qui contient une combination-S-, où on fait réduire cette combinaison et puis on fait réagir pour former un conjugué d'anticorps et de ricine qui contient une combinaison -S-S.

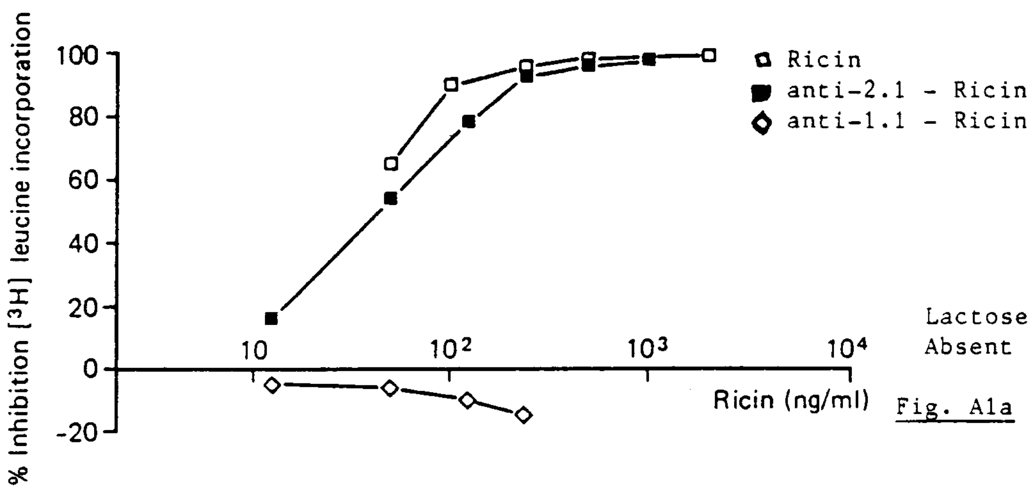


Lactose Absent

Fig. A1a

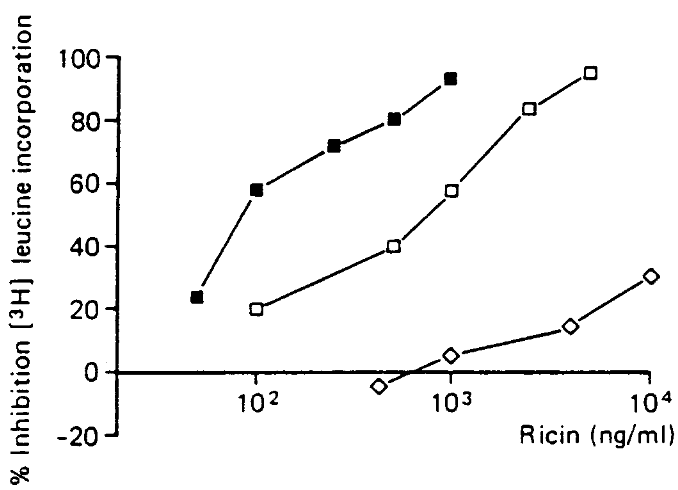


Lactose Present

Fig. A1b

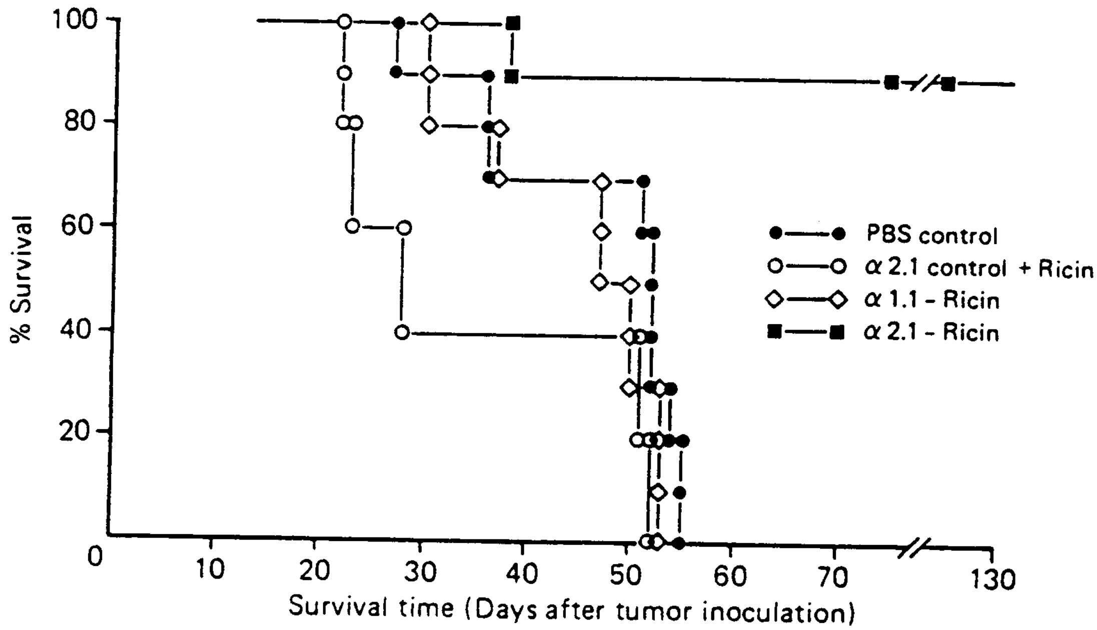
100
80
60
40
20
0
% Survival
10
20
30
40
50
60
70
130
Survival time (Days after tumor inoculation)
PBS control
α 2.1 control + Ricin
α 1.1 - Ricin
α 2.1 - Ricin

Fig. A2

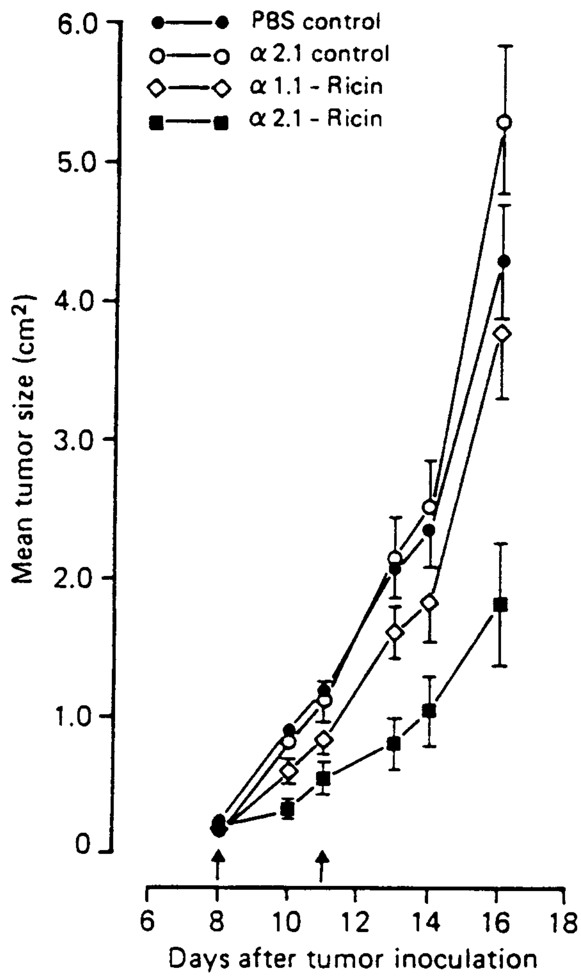
PBS control
α 2.1 control
α 1.1 - Ricin
α 2.1 - Ricin
Mean tumor size (cm²)
6.0
5.0
4.0
3.0
2.0
1.0
0
6
8
10
12
14
16
18
Days after tumor inoculation

Fig. A3a

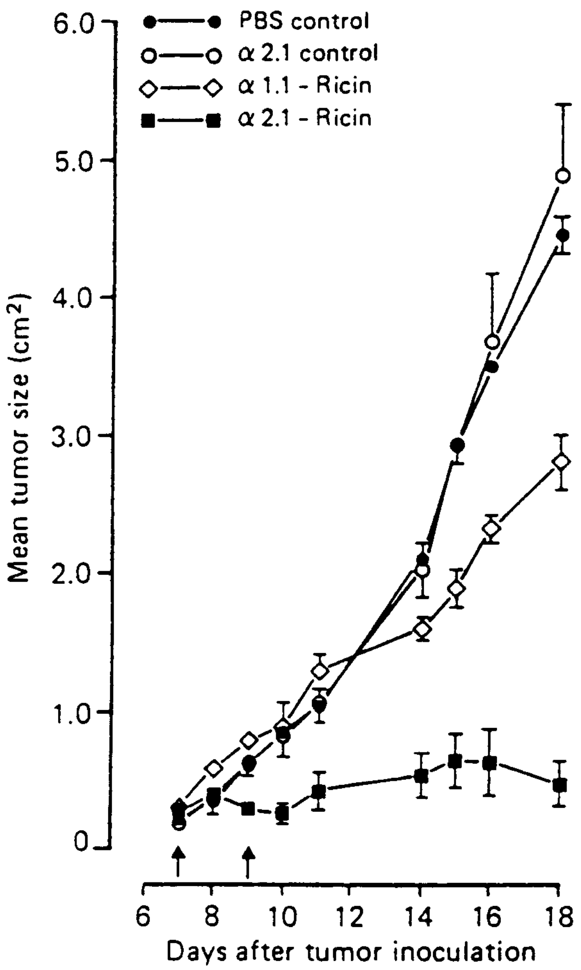
PBS control
α 2.1 control
α 1.1 - Ricin
α 2.1 - Ricin
Mean tumor size (cm²)
6.0
5.0
4.0
3.0
2.0
1.0
0
6
8
10
12
14
16
18
Days after tumor inoculation

Fig. A3b

Gel permeation chromatography on Sepharyl S-300

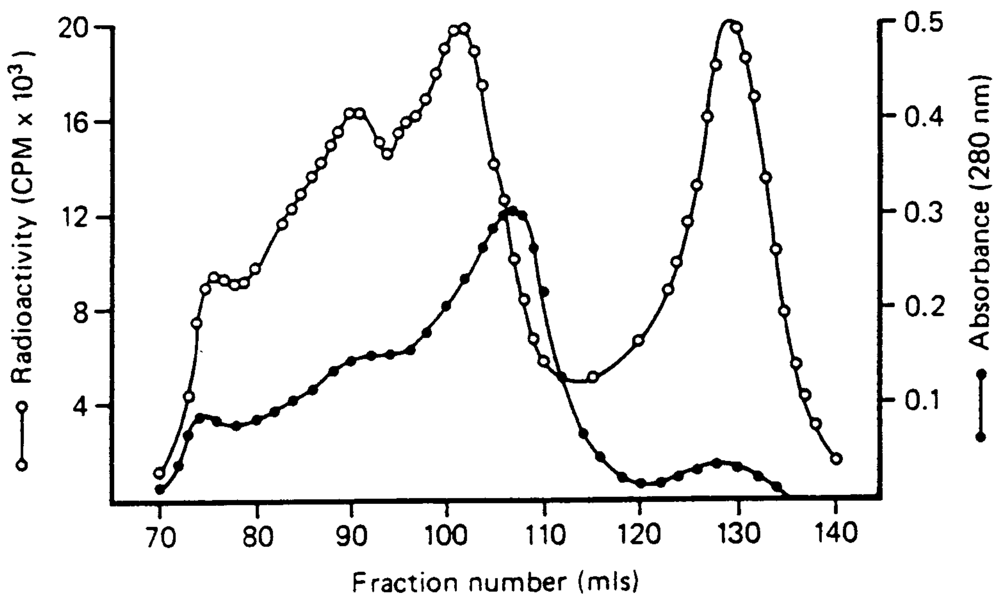


Fig. B1

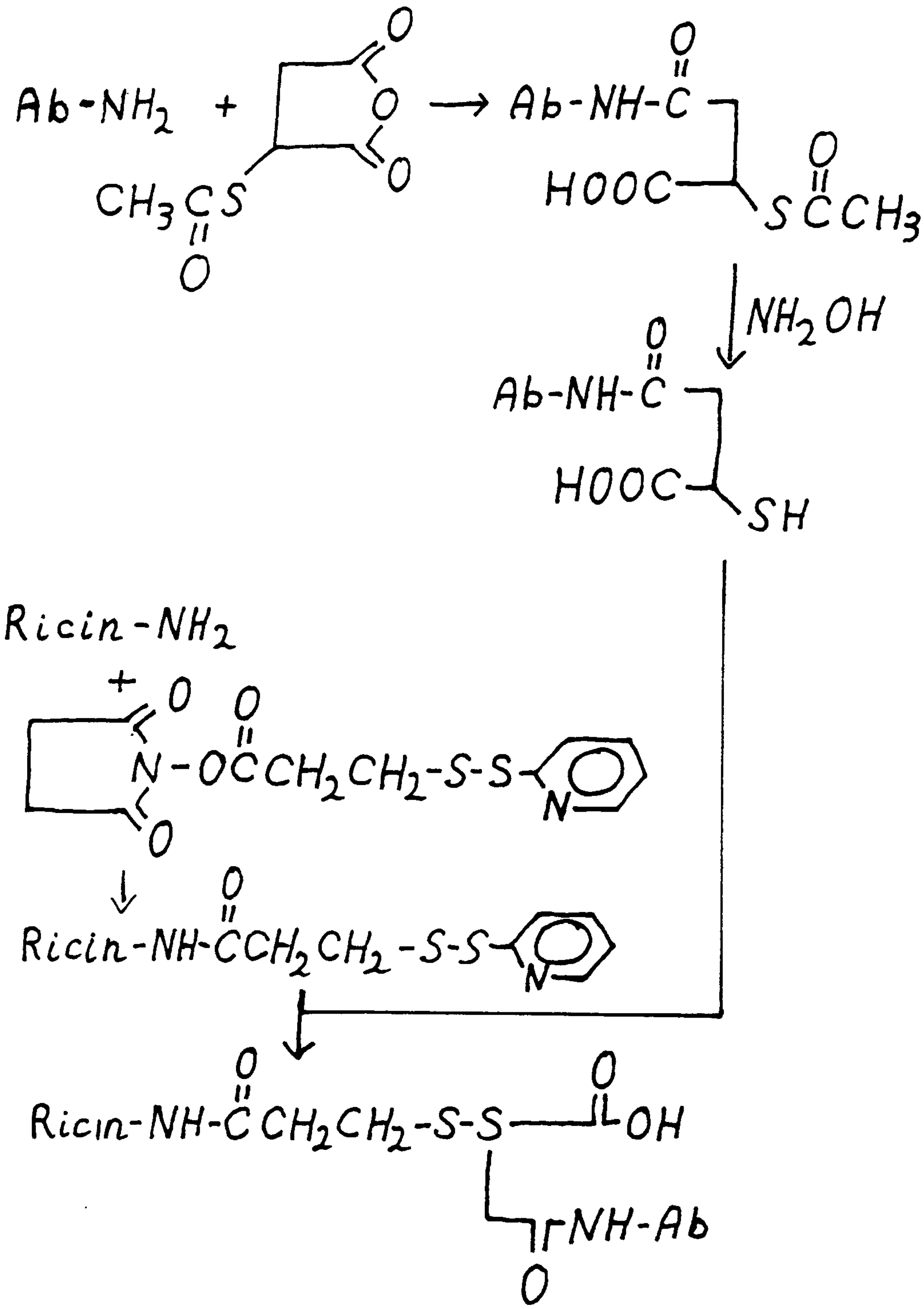

FIG. B2a

Conjugation of Ricin to Antibody via SAMSA/SPDP

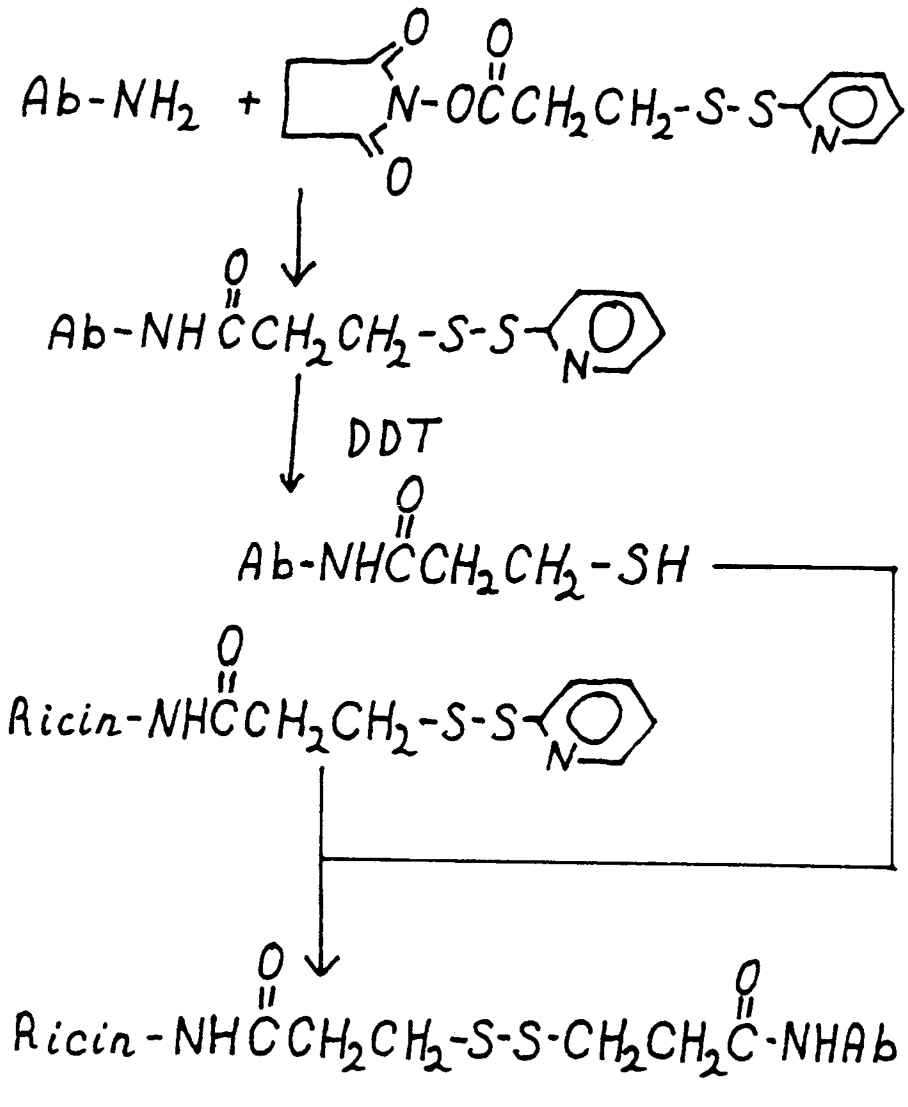


FIG. B2b

Conjugation of Ricin to Antibody using SPDP/SPDP

BINDING AFFINITY OF SIN β TREATED RICIN ON LACTOSE-SEPHAROSE

SIAB

Fig. B4

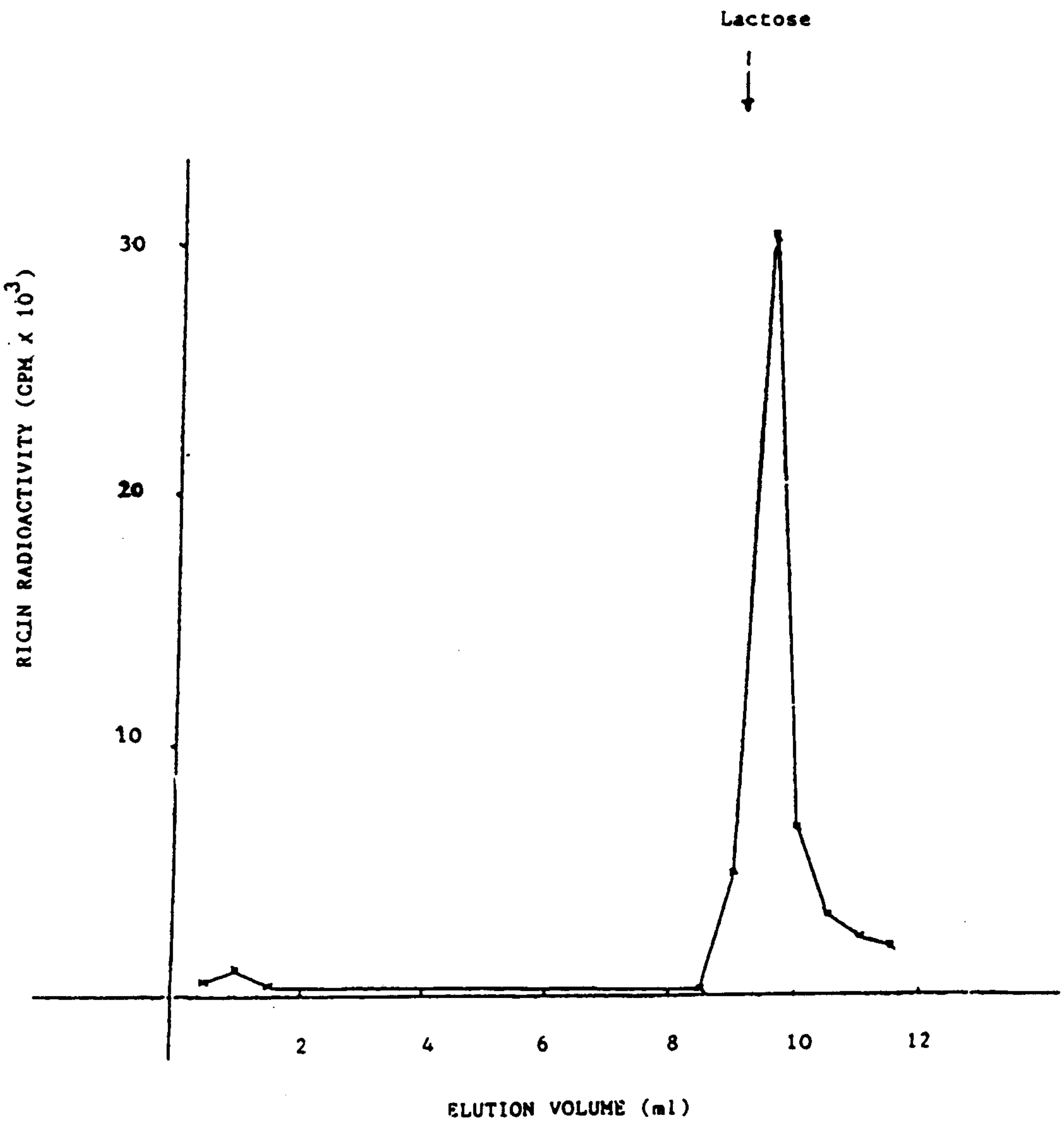

Competitive binding assay

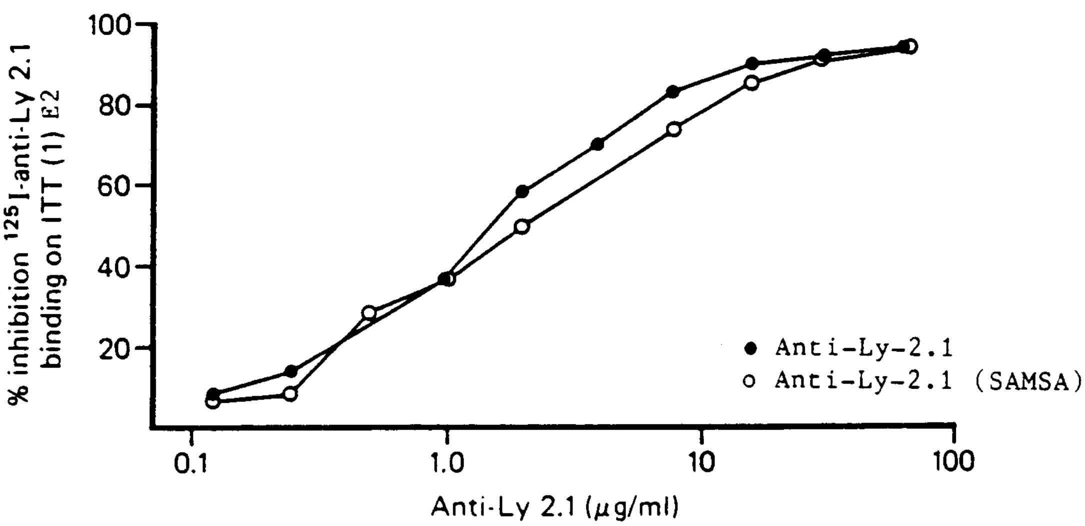


FIG. B5

Antibody activity of anti-Ly-2.1 Ricin

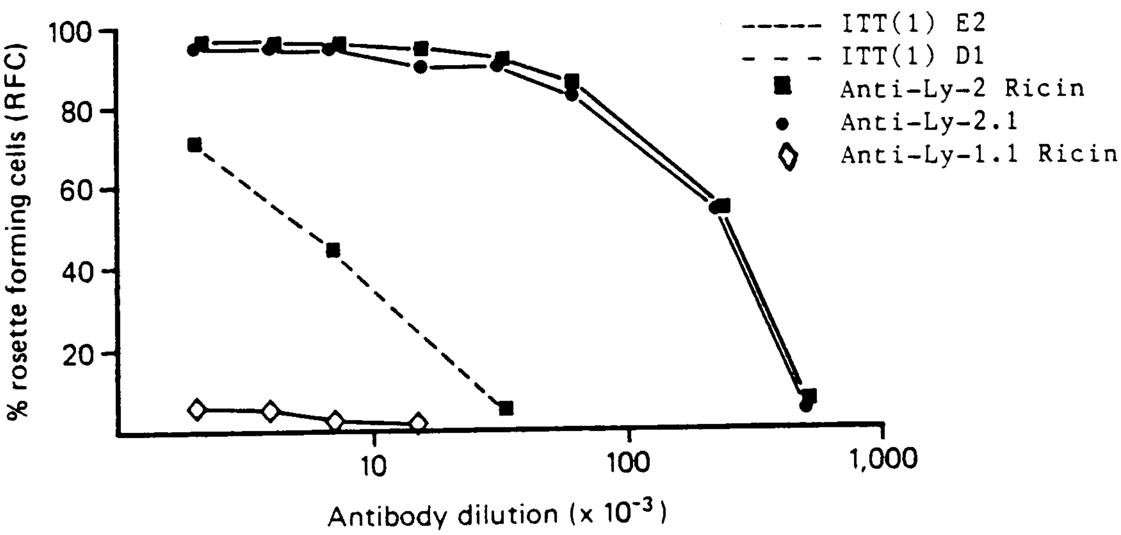


Fig. B6

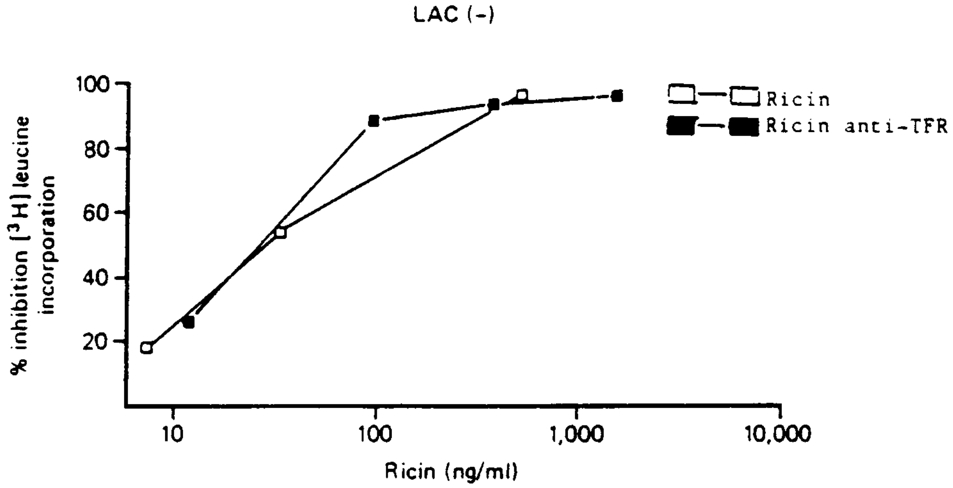


Fig. B7a

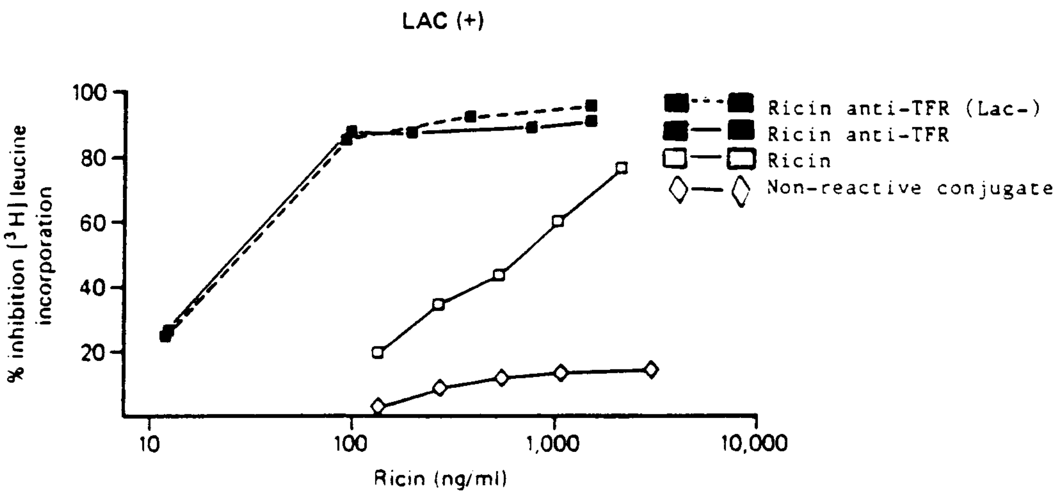


Fig. B7b

Cytotoxicity of anti-Ly-2.1R ($NAlO_4^-$)
on ITT(1)E3

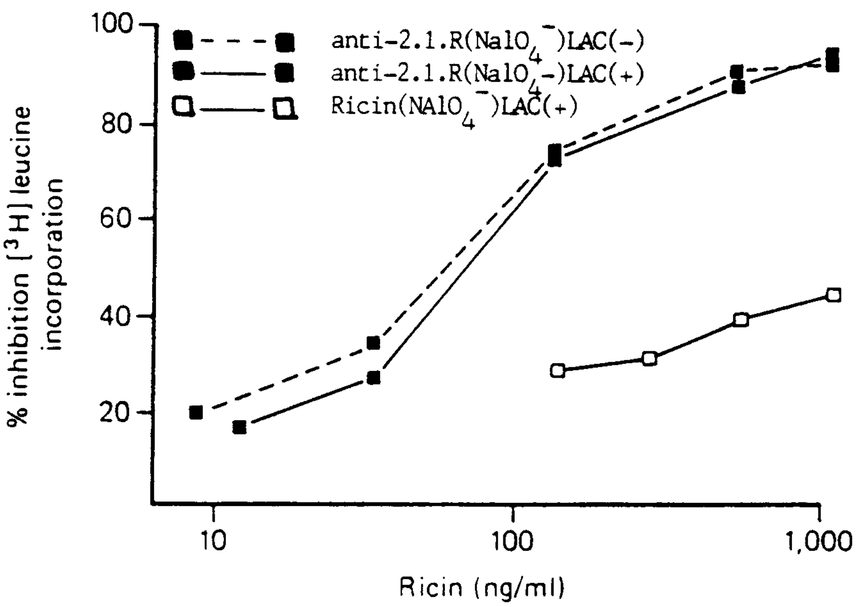


Fig.B8

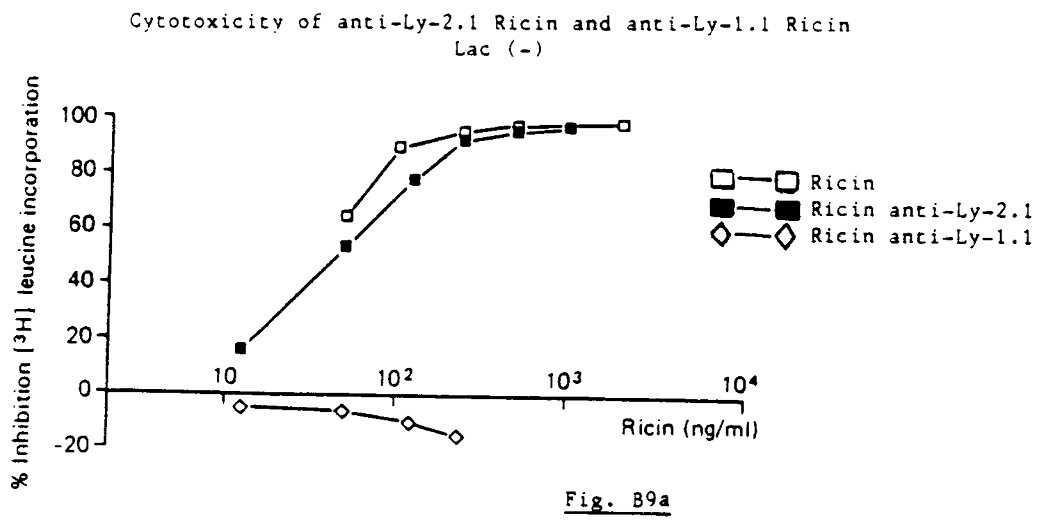


Fig. B9a

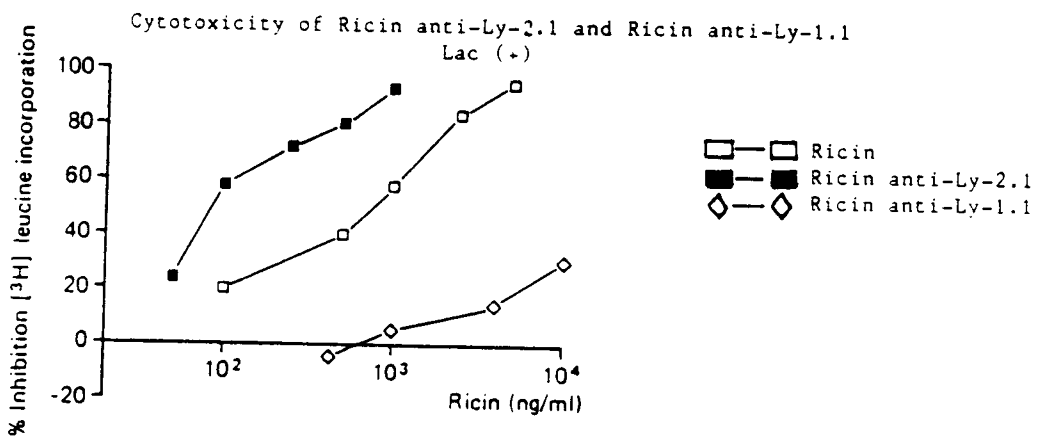


Fig. B9b

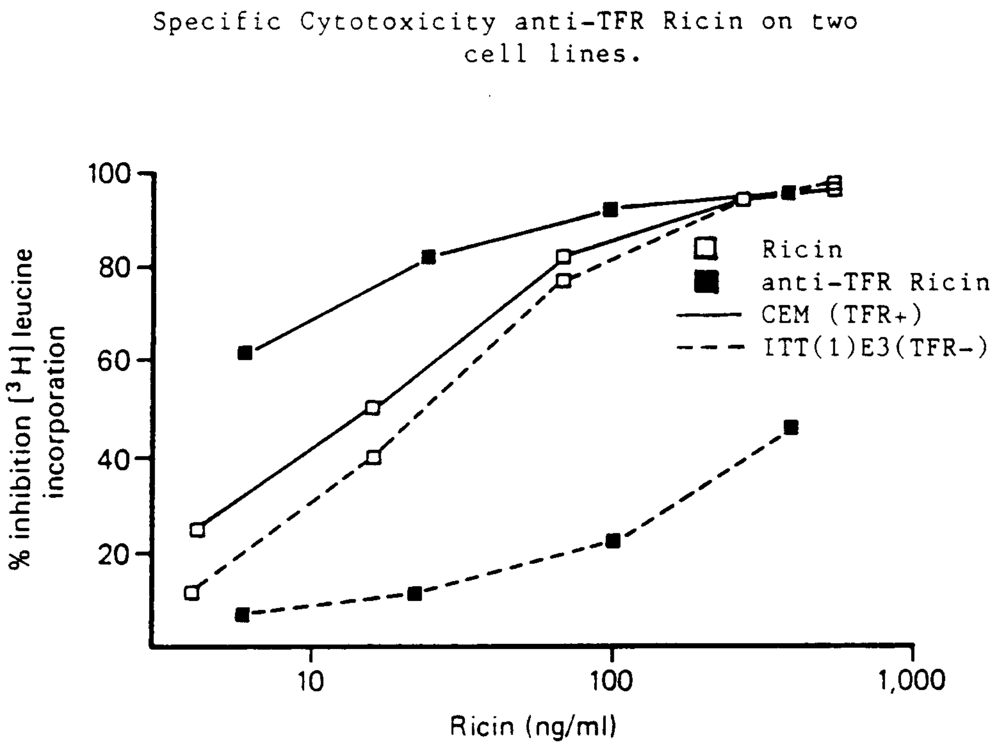
Specific Cytotoxicity anti-TFR Ricin on two cell lines.
100
80
60
40
20
% inhibition [$^3$H] leucine incorporation
10
100
1,000
Ricin (ng/ml)
Ricin
anti-TFR Ricin
CEM (TFR+)
ITT(1)E3(TFR-)

Fig. B10a

Specific Cytotoxicity of Ricin·anti-Ly-2.1

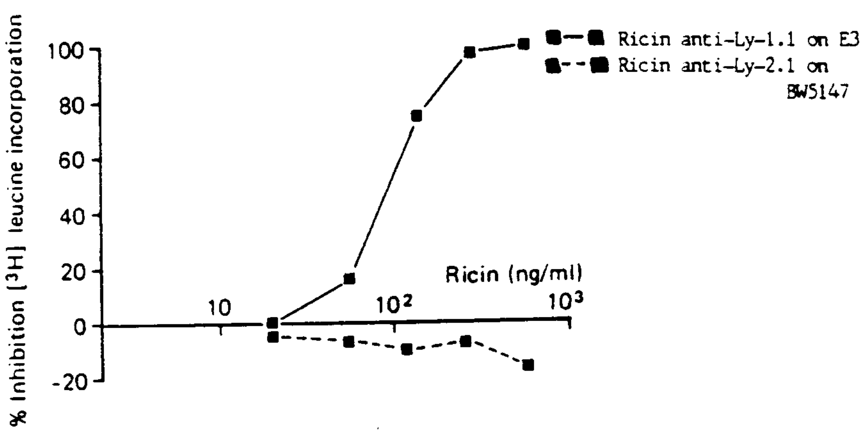


Fig. B10b

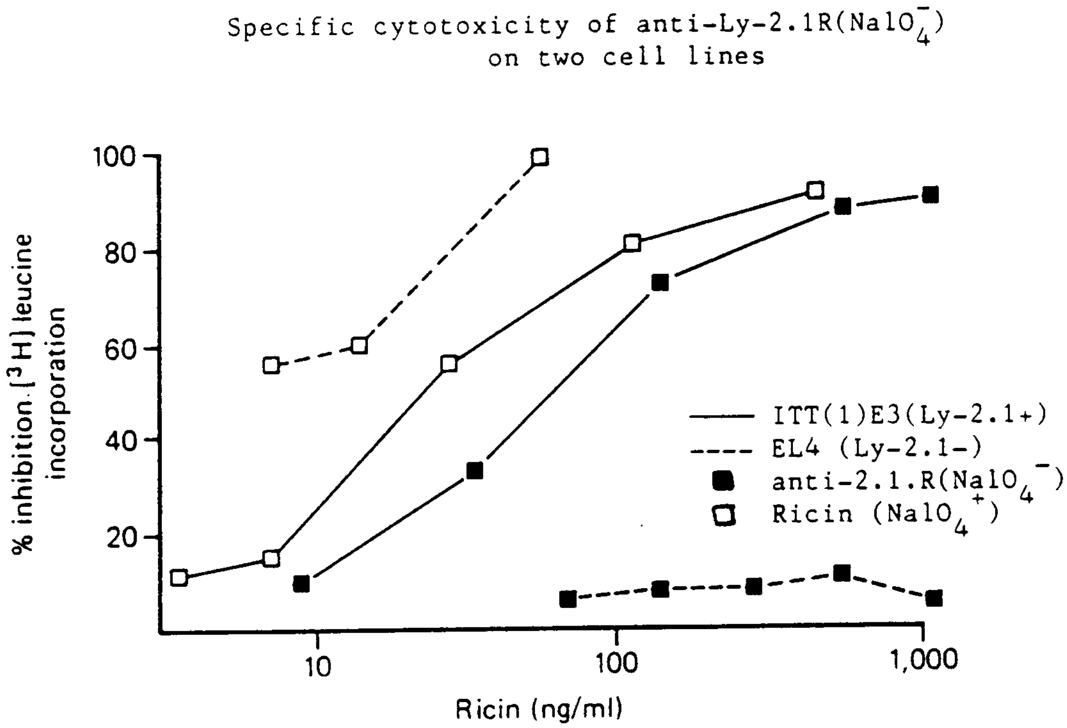
Specific cytotoxicity of anti-Ly-2.1R($NaIO_4^-$)
on two cell lines
100
80
60
40
20
% inhibition [$^3H$] leucine incorporation
10
100
1,000
Ricin (ng/ml)
ITT(1)E3(Ly-2.1+)
EL4 (Ly-2.1-)
anti-2.1.R($NaIO_4^-$)
Ricin ($NaIO_4^+$)

Fig. B10c

Inhibition of Cytotoxicity

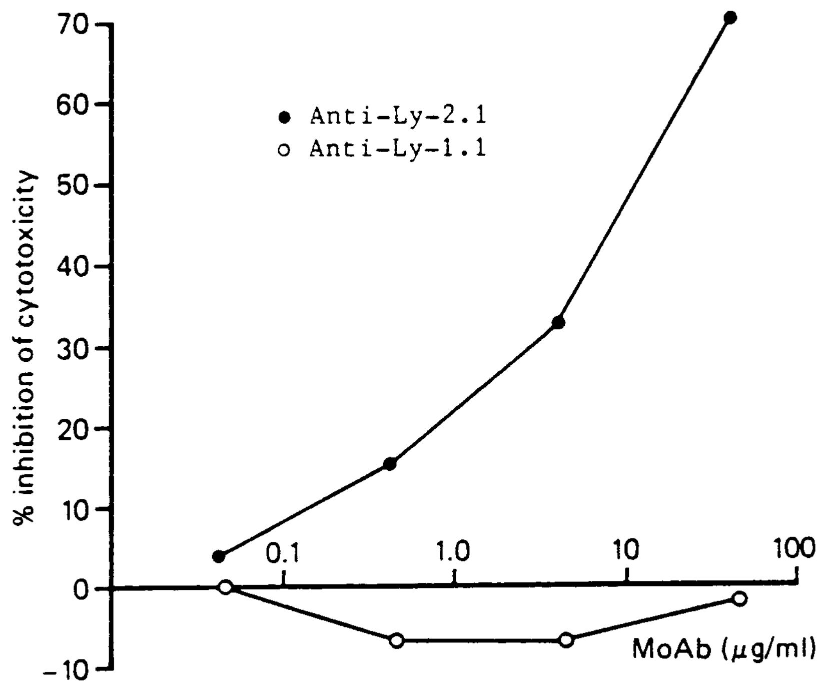


Fig. B11

Reactivity of Iodinated anti-2.1-S-S-Ricin on two cell lines (LAC⁻)

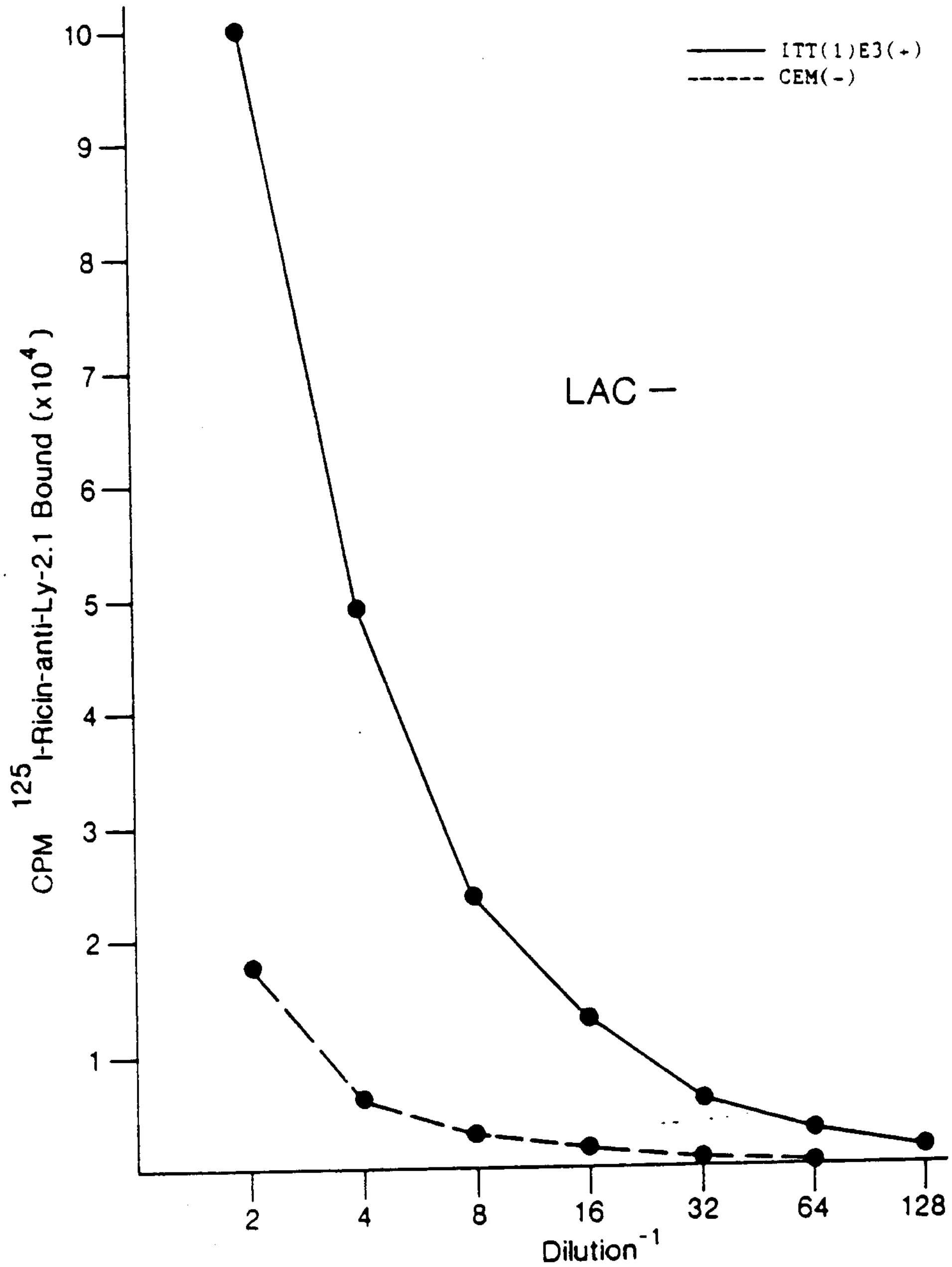


Fig. C1a

Reactivity of Iodinated anti-2.1-S-S-Ricin on Two Cell Lines ($LAC^+$)

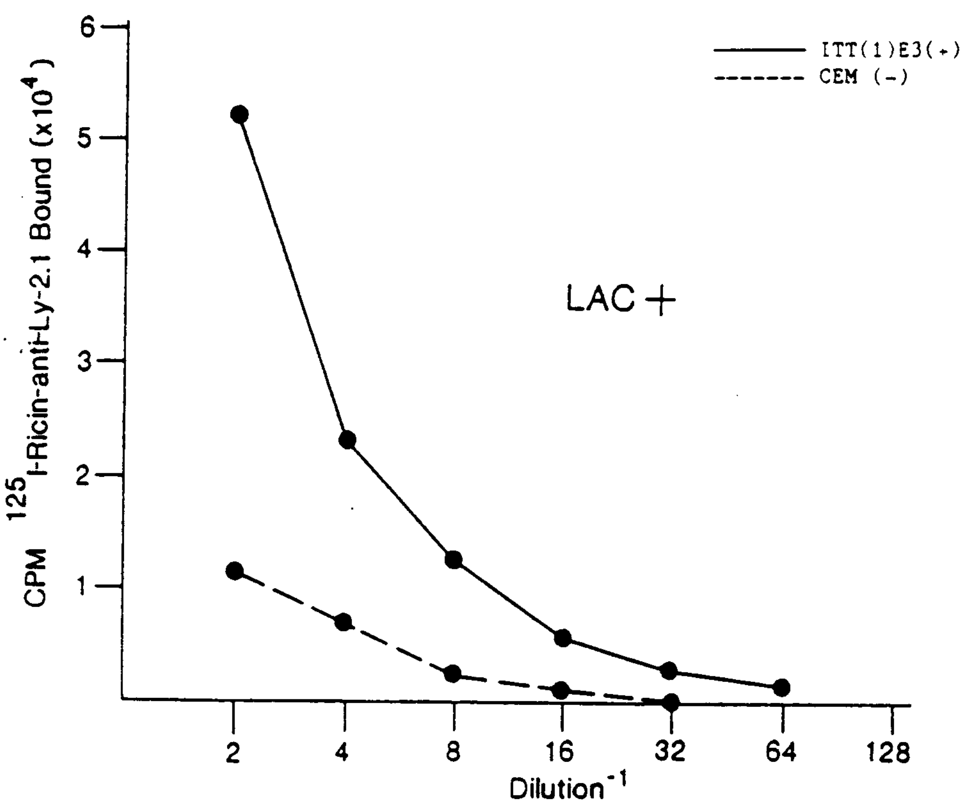


Fig. C1b

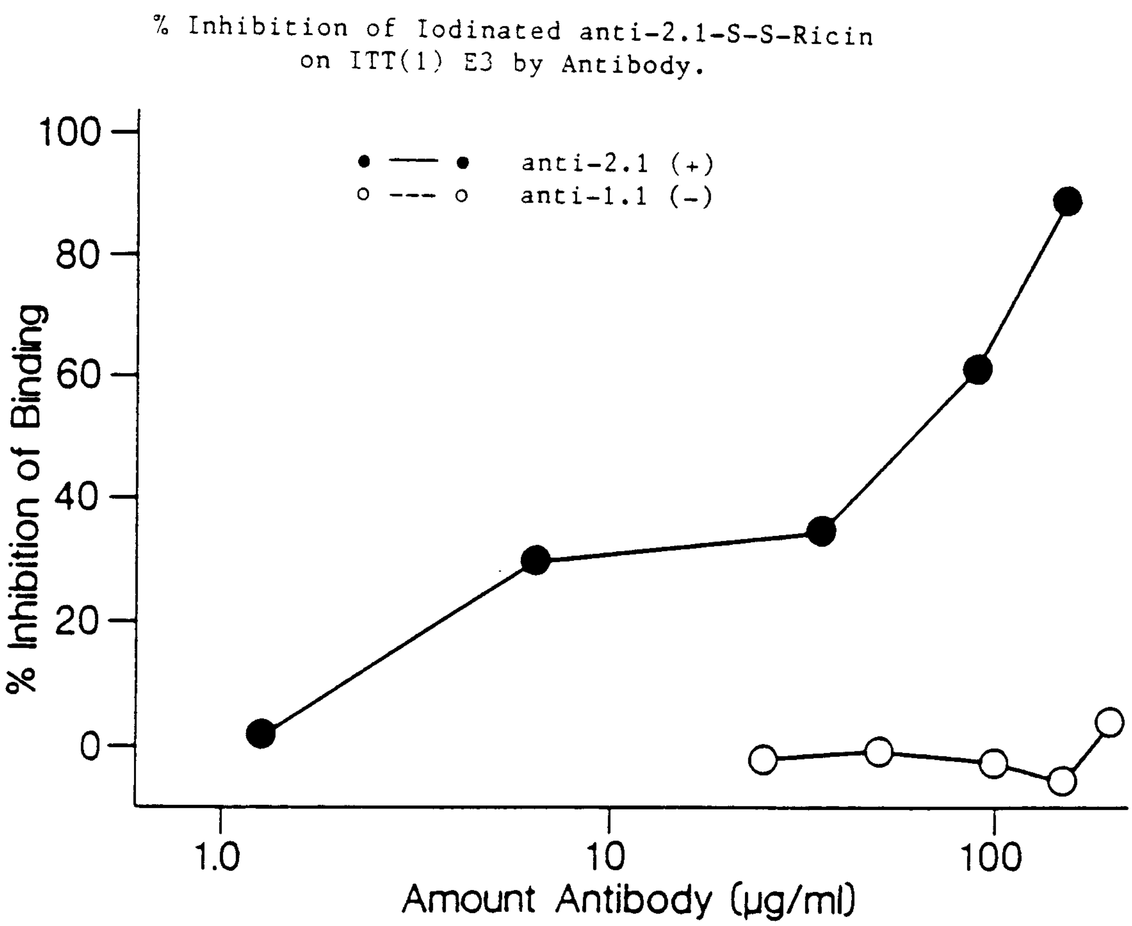
% Inhibition of Iodinated anti-2.1-S-S-Ricin
on ITT(1) E3 by Antibody.
100
80
60
40
20
0
% Inhibition of Binding
● — ● anti-2.1 (+)
o --- o anti-1.1 (-)
1.0
10
100
Amount Antibody (µg/ml)

Fig. C3

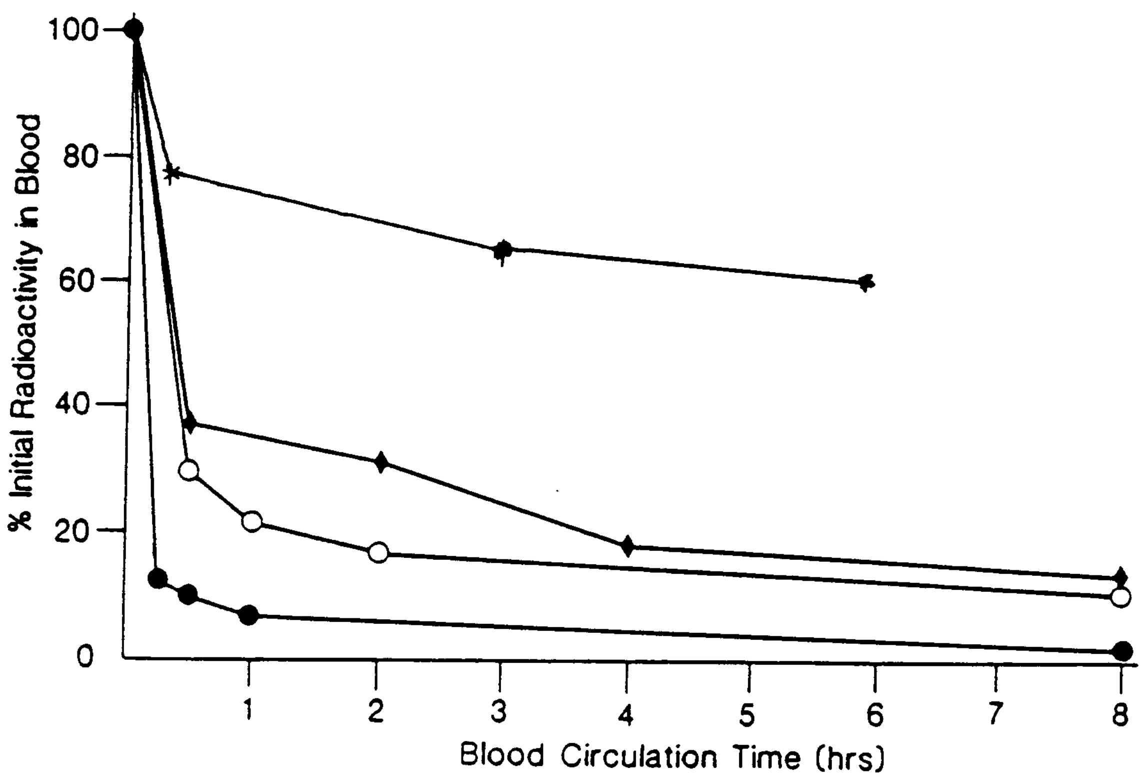


Fig. C4

● —— ● Native Ricin
○ ---- ○ $NaIO_4$ Ricin
♦ —— ♦ anti-2.1 Ricin
* —— * anti-Ly-2.1

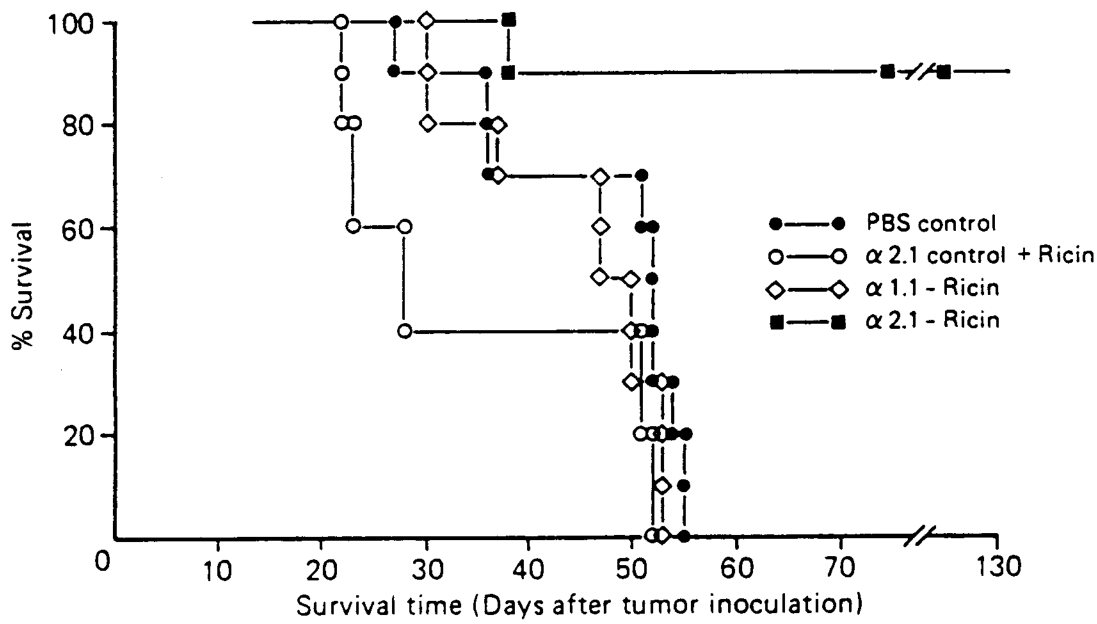
100
80
60
40
20
0
10
20
30
40
50
60
70
130
% Survival
Survival time (Days after tumor inoculation)
PBS control
α 2.1 control + Ricin
α 1.1 - Ricin
α 2.1 - Ricin

Fig. C6a

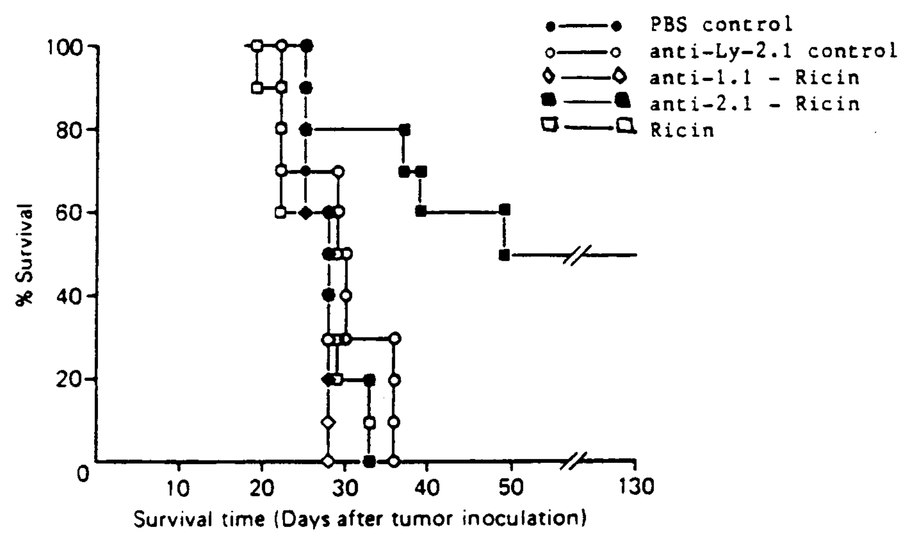
PBS control
anti-Ly-2.1 control
anti-1.1 - Ricin
anti-2.1 - Ricin
Ricin
100
80
60
40
20
0
% Survival
10
20
30
40
50
130
Survival time (Days after tumor inoculation)

Fig. 66b

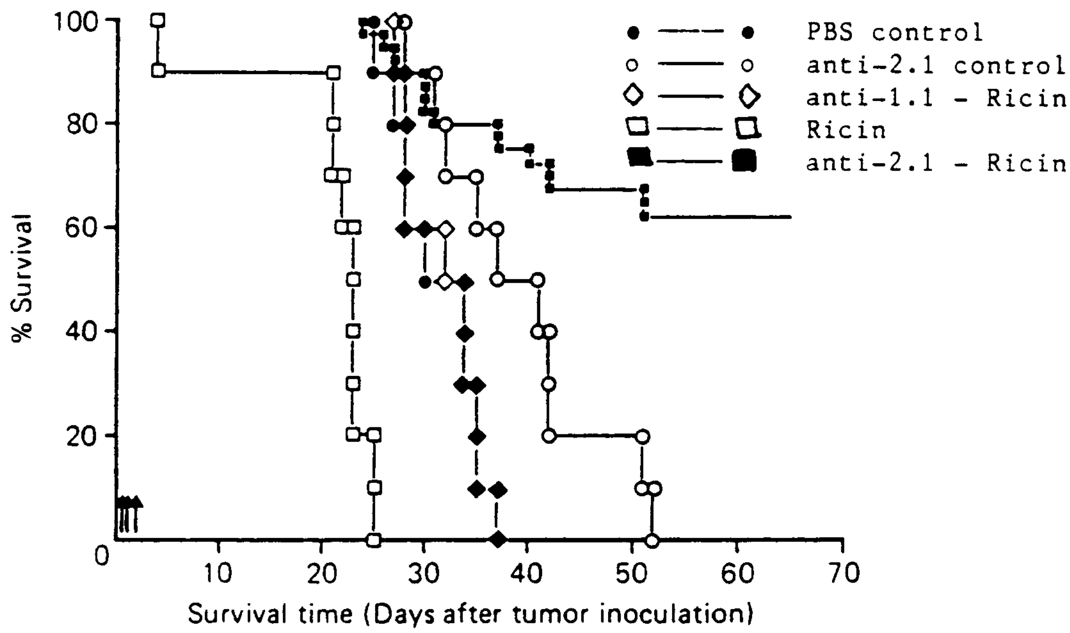
100
80
60
40
20
0
% Survival
10
20
30
40
50
60
70
Survival time (Days after tumor inoculation)
PBS control
anti-2.1 control
anti-1.1 - Ricin
Ricin
anti-2.1 - Ricin

Fig. C6c

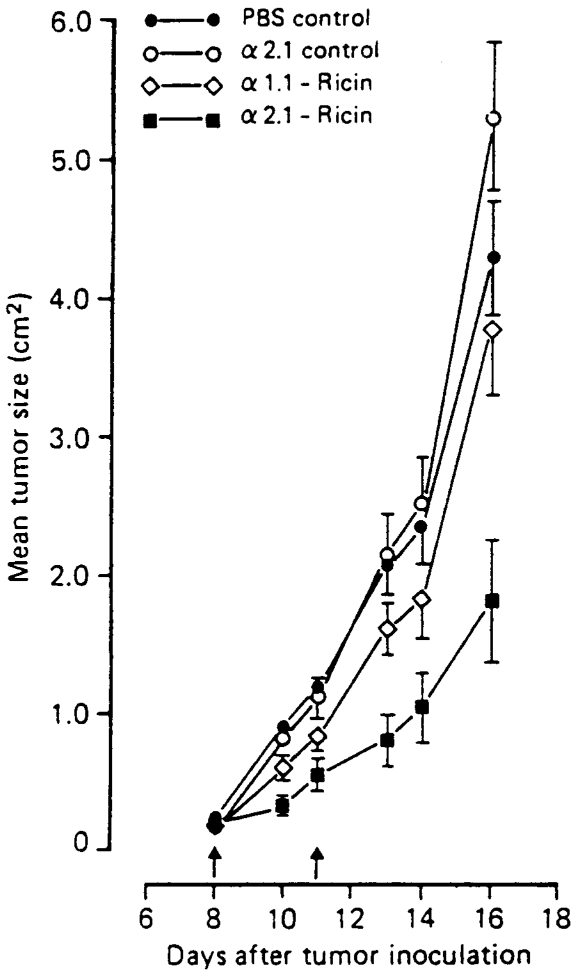
PBS control
α 2.1 control
α 1.1 - Ricin
α 2.1 - Ricin
6.0
5.0
4.0
3.0
2.0
1.0
0
Mean tumor size (cm²)
6
8
10
12
14
16
18
Days after tumor inoculation

Fig. C7

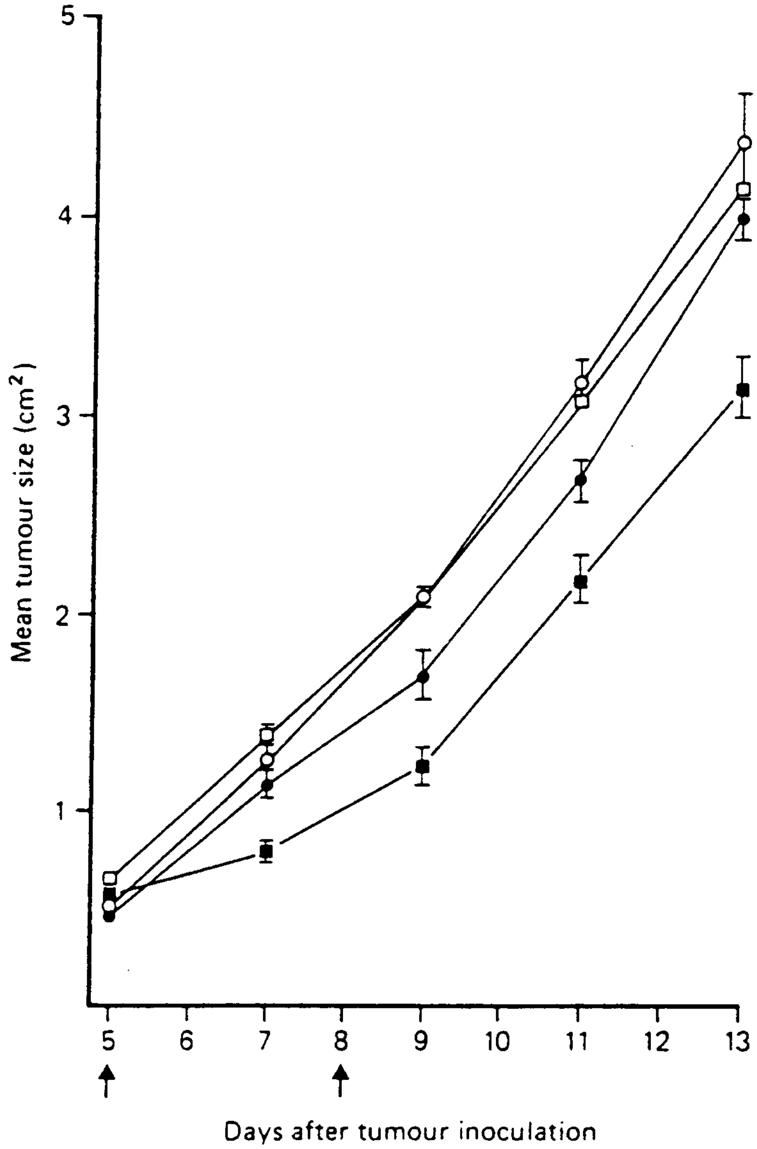

Mean tumour size (cm²)
1
2
3
4
5
5
6
7
8
9
10
11
12
13
Days after tumour inoculation


Fig. C8a

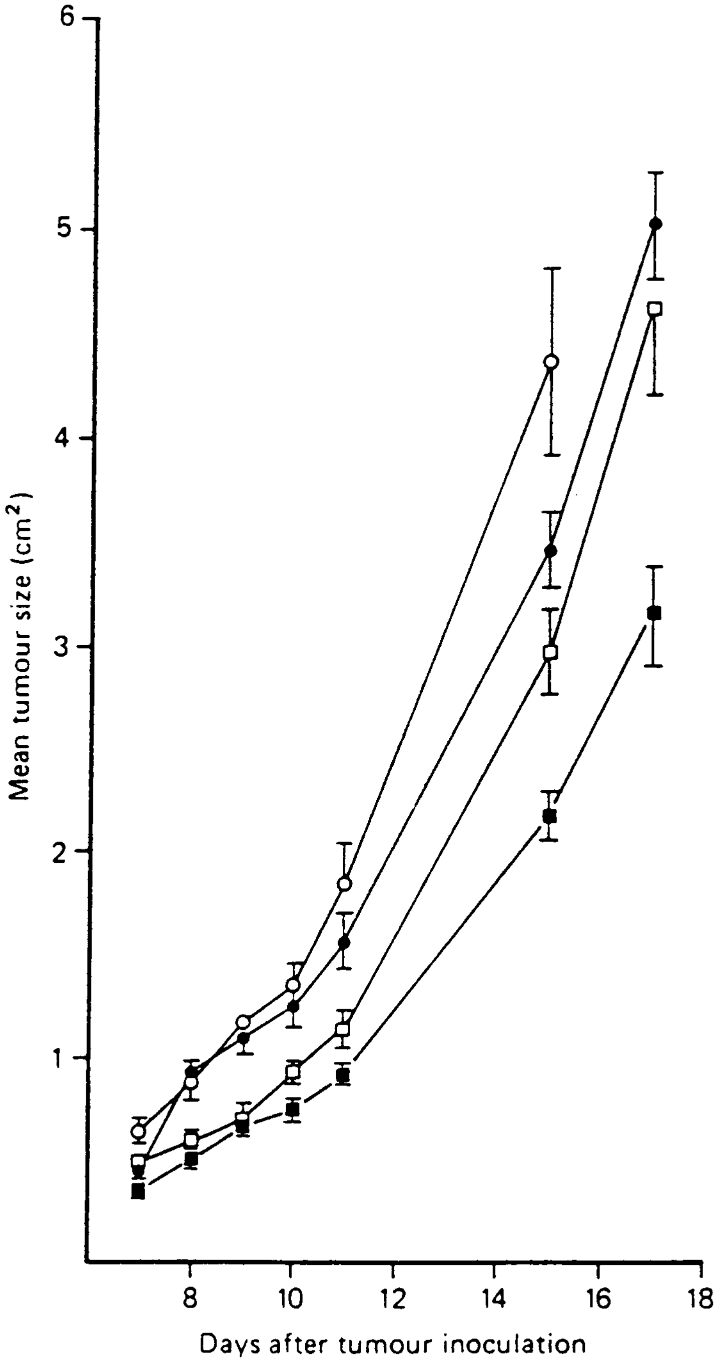
Mean tumour size (cm²)
6
5
4
3
2
1
8
10
12
14
16
18
Days after tumour inoculation

Fig. C8b

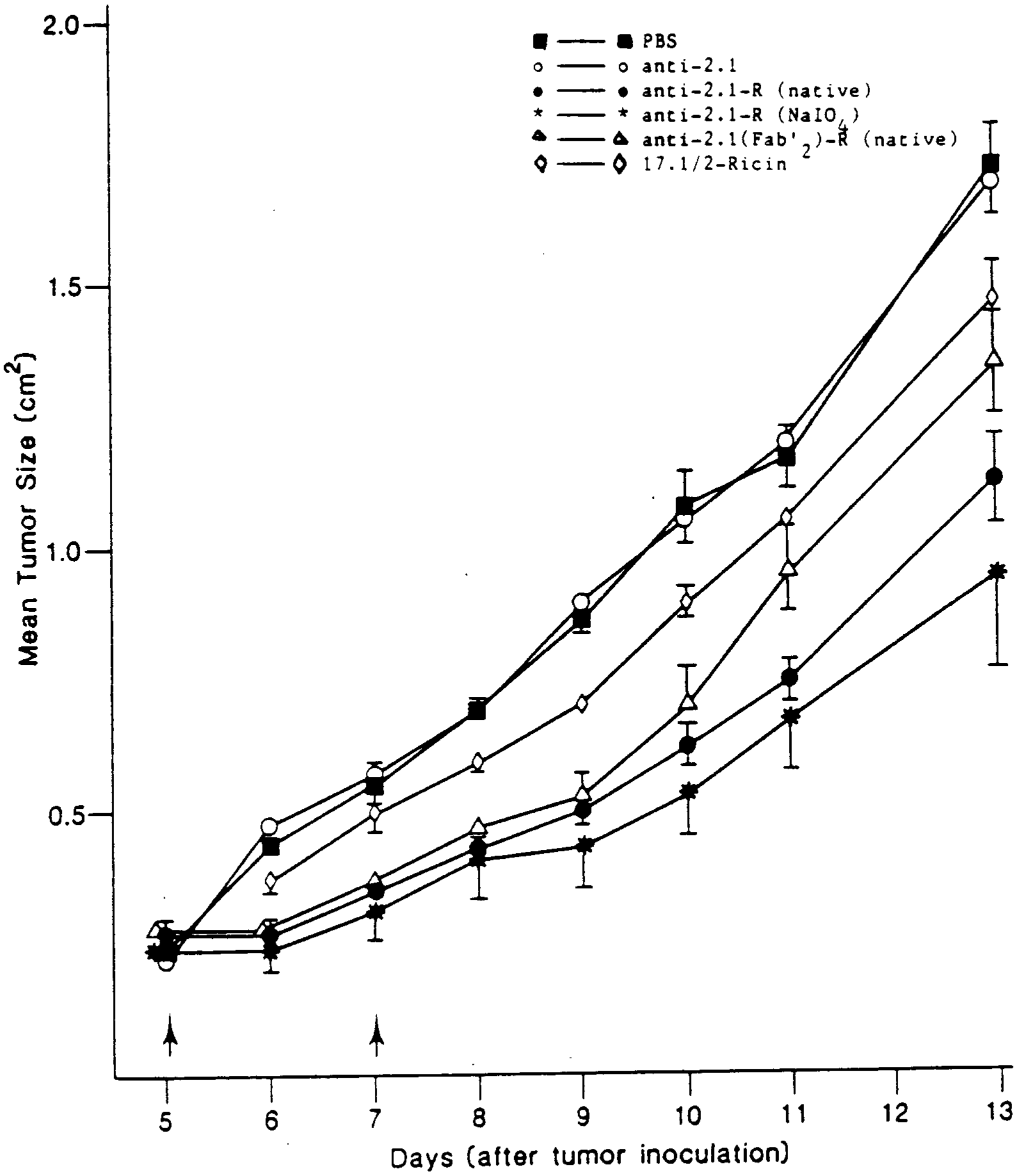
PBS
anti-2.1
anti-2.1-R (native)
anti-2.1-R (NaIO4)
anti-2.1(Fab'2)-R (native)
17.1/2-Ricin
Mean Tumor Size (cm2)
2.0
1.5
1.0
0.5
5
6
7
8
9
10
11
12
13
Days (after tumor inoculation)

Fig. C9

Clearance of antibody injected
intra-tumourly

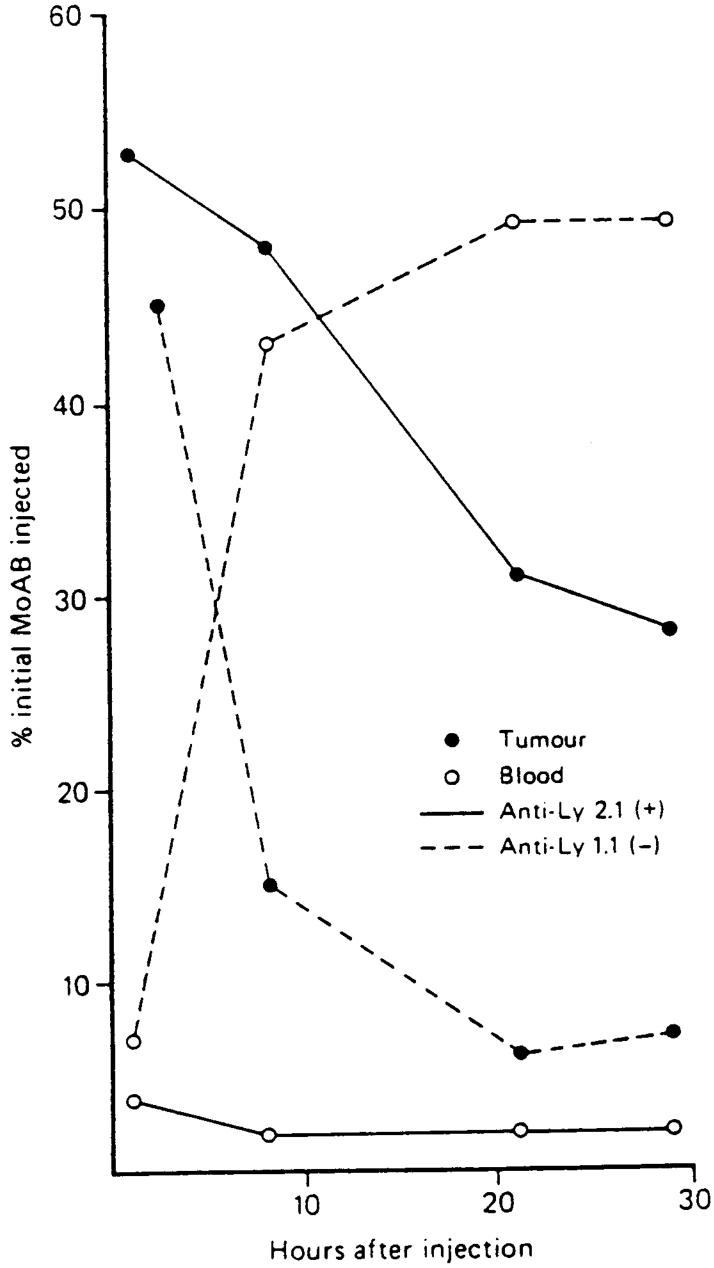


Fig. D1a

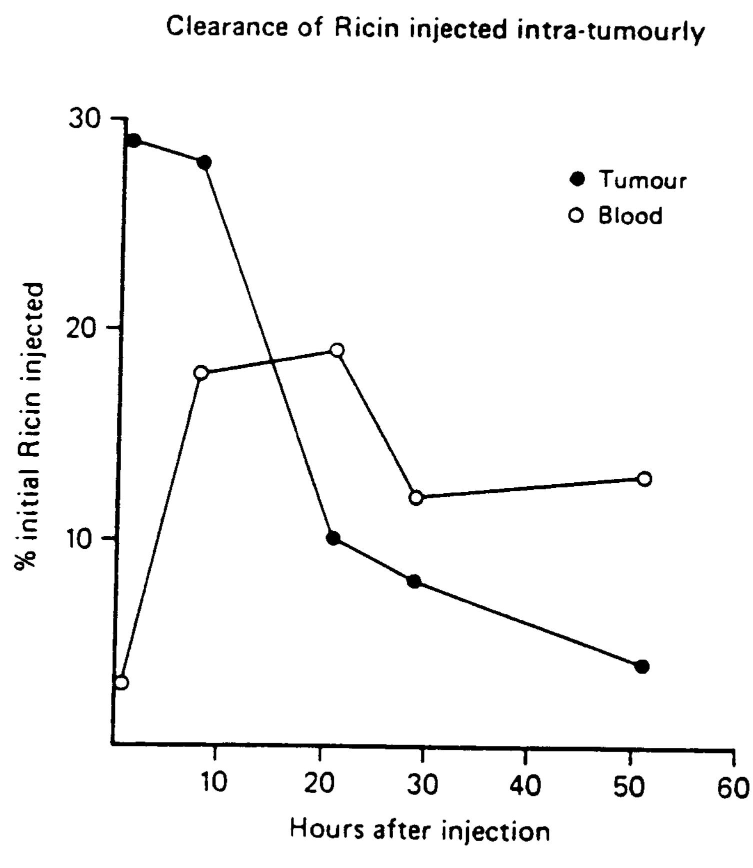
Clearance of Ricin injected intra-tumourly
30
20
10
% initial Ricin injected
Tumour
Blood
10
20
30
40
50
60
Hours after injection

Fig. D1b

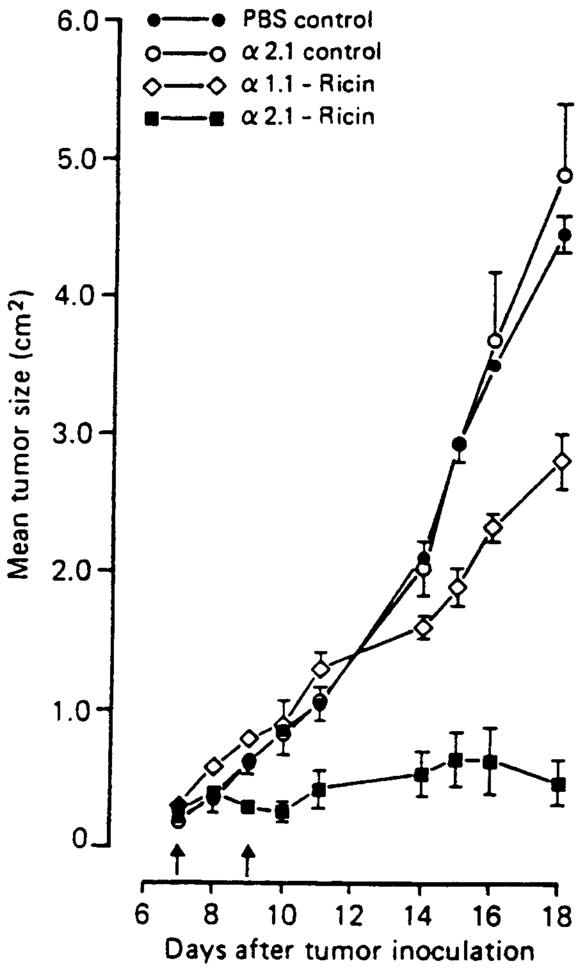
PBS control
α 2.1 control
α 1.1 - Ricin
α 2.1 - Ricin
Mean tumor size (cm²)
6.0
5.0
4.0
3.0
2.0
1.0
0
6
8
10
12
14
16
18
Days after tumor inoculation

Fig. D2a

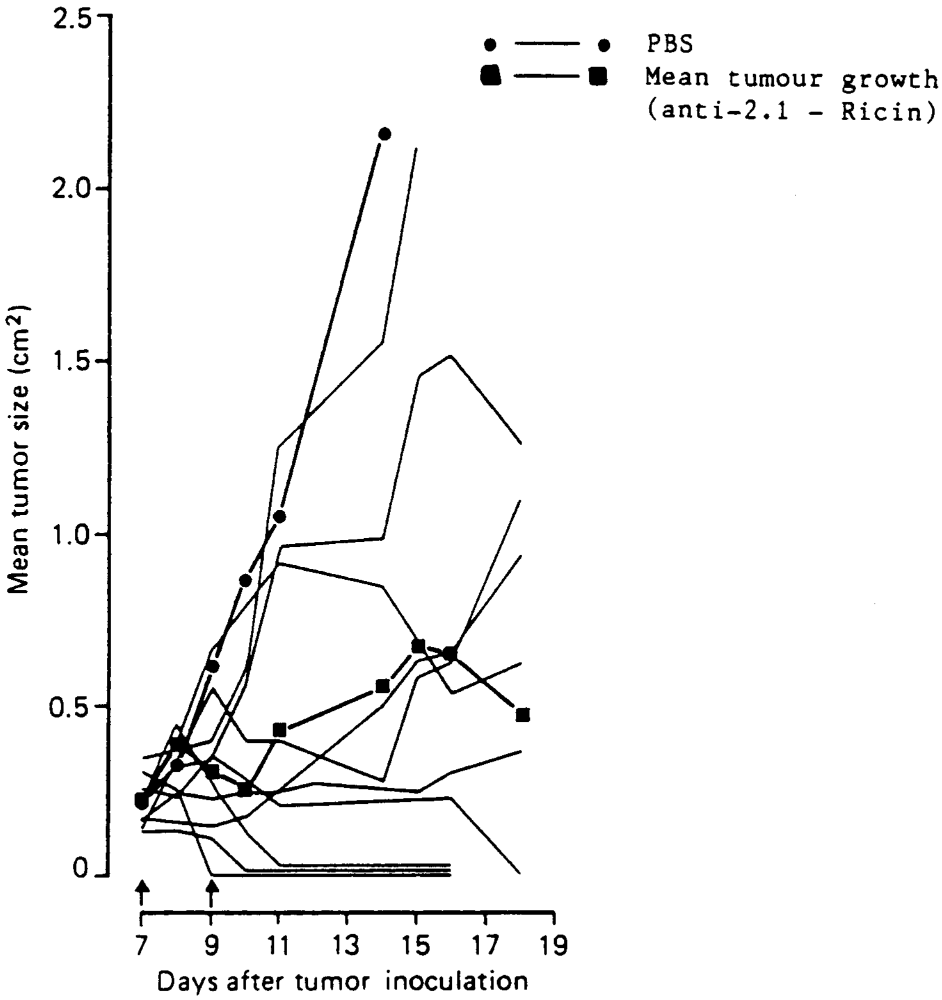
PBS
Mean tumour growth
(anti-2.1 - Ricin)
2.5
2.0
1.5
1.0
0.5
0
Mean tumor size (cm2)
7
9
11
13
15
17
19
Days after tumor inoculation

Fig. D2b

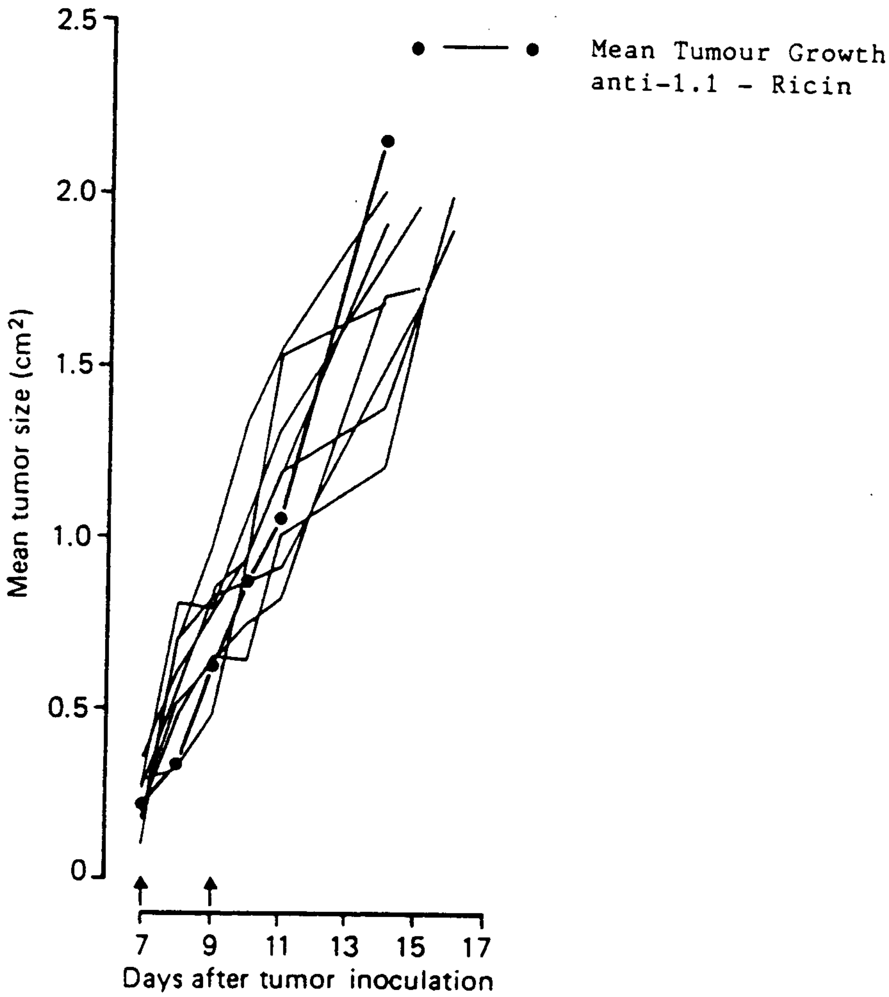
2.5
2.0
1.5
1.0
0.5
0
Mean tumor size (cm²)
7
9
11
13
15
17
Days after tumor inoculation
Mean Tumour Growth
anti-1.1 - Ricin

Fig. D2c

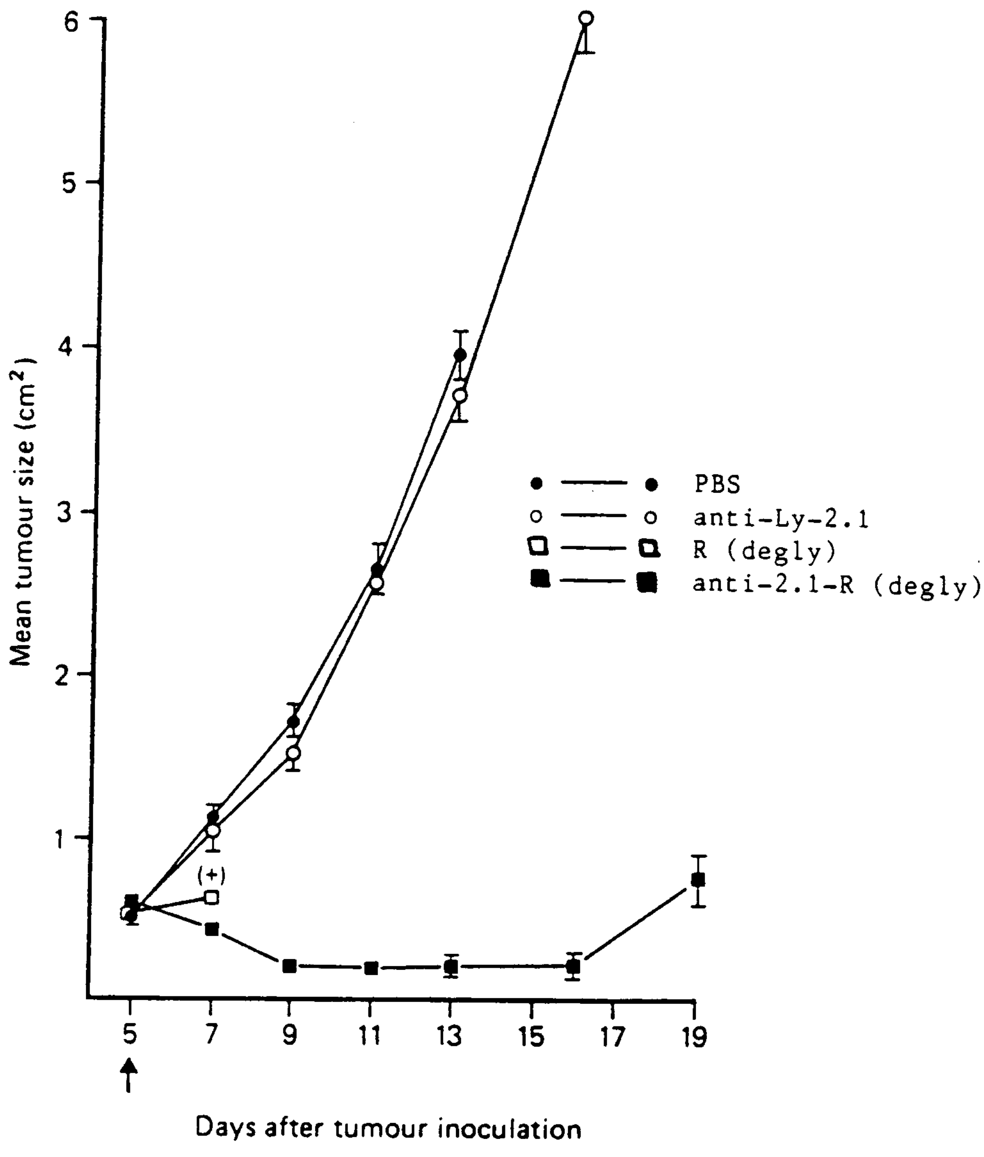
6
5
4
3
2
1
Mean tumour size (cm²)
PBS
anti-Ly-2.1
R (degly)
anti-2.1-R (degly)
(+)
5
7
9
11
13
15
17
19
Days after tumour inoculation

Fig. D5

Tumour growth nude mice bearing
CEM xenografts

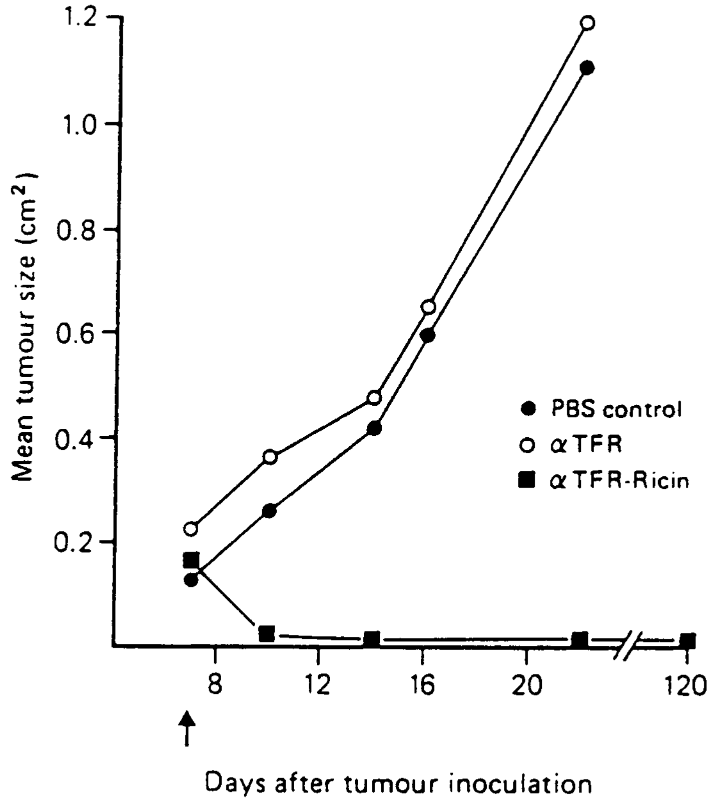


Fig. D7

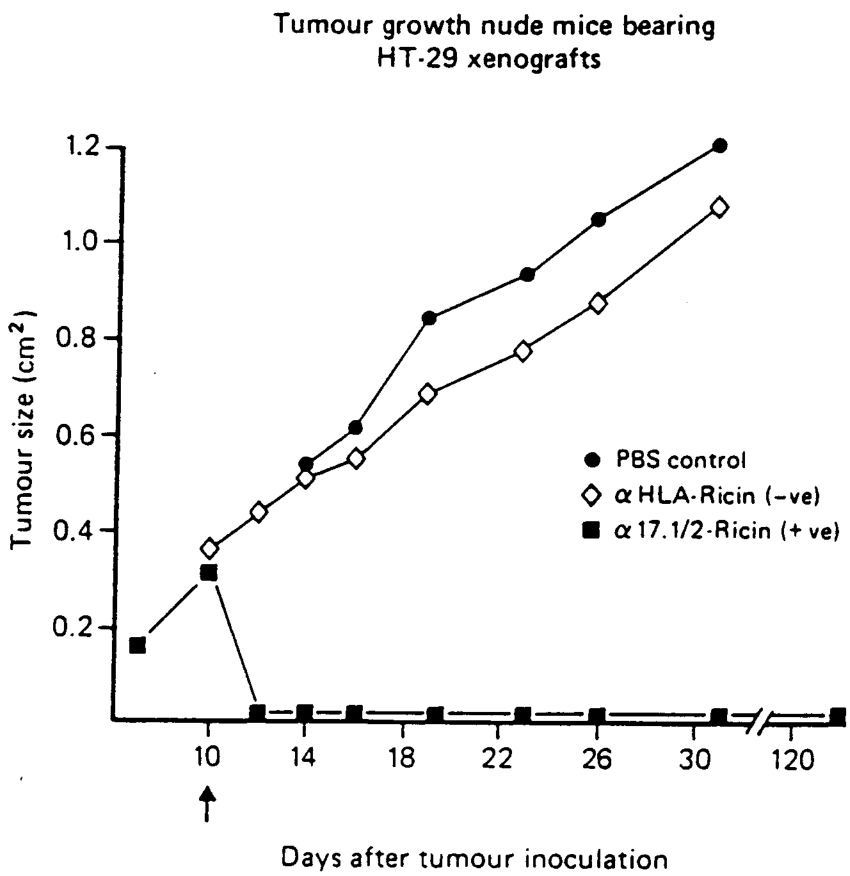
Tumour growth nude mice bearing
HT-29 xenografts
Tumour size (cm²)
1.2
1.0
0.8
0.6
0.4
0.2
PBS control
α HLA-Ricin (-ve)
α 17.1/2-Ricin (+ ve)
10
14
18
22
26
30
120
Days after tumour inoculation

Fig. D8

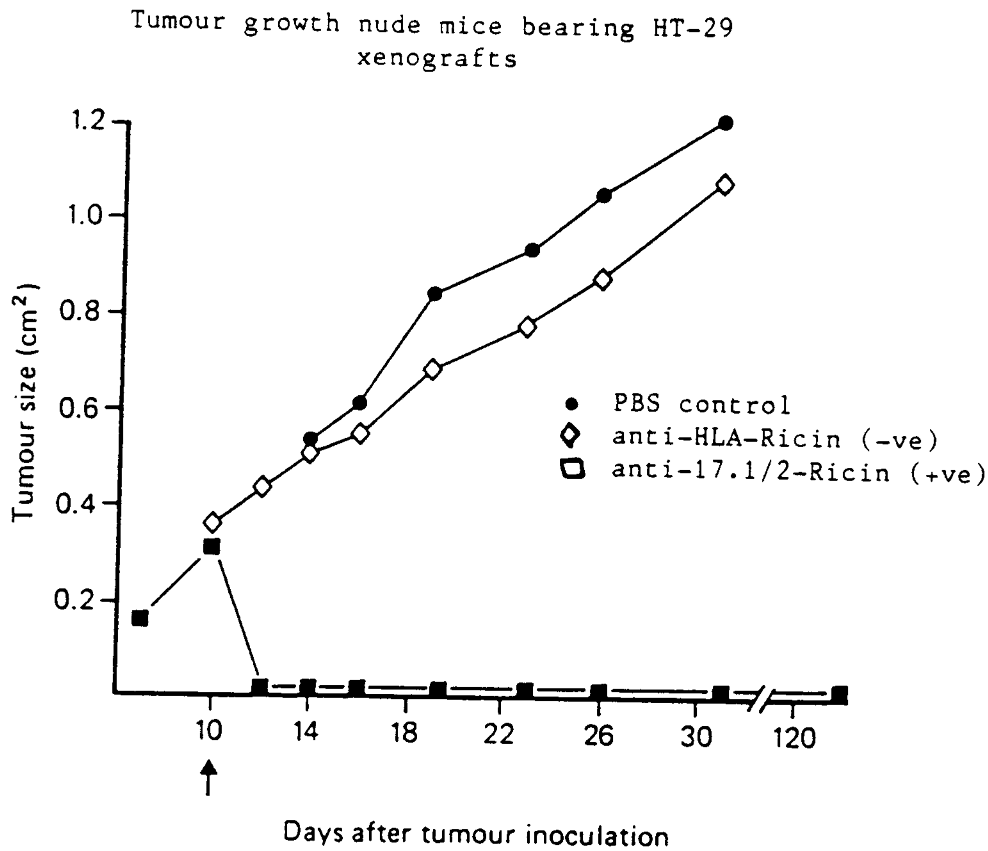
Tumour growth nude mice bearing HT-29 xenografts
Tumour size (cm²)
1.2
1.0
0.8
0.6
0.4
0.2
PBS control
anti-HLA-Ricin (-ve)
anti-17.1/2-Ricin (+ve)
10
14
18
22
26
30
120
Days after tumour inoculation

Fig. D9